# EUROPEAN PATENT APPLICATION

(11) **EP 2 465 926 A1**
(43) Date of publication of application: **20.06.2012**
(21) Application number: 10195995.5
(22) Date of filing: 20.12.2010
(51) Int. Cl.: C12N 7/00, A61K 35/76

(54) **Pseudomonas aeruginosa Bacteriophage and uses thereof**

(71) Applicant: University College Cork, Cork City (IE); Agriculture and Food Development Authority (TEAGASC), Co. Carlow (IE)
(72) Inventor: Alemayehu, Debebe, Mallow, Co. Cork (IE); Ross, Paul, Kilworth, Co. Cork (IE); Hill, Colin, Cork (IE); Martin, James, Montreal, Québec (CA)
(74) Representative: Purdy, Hugh Barry

(57) **Abstract**

An isolated bacteriophage MR299-2 or NH-4 deposited under NCIMB Deposit Accession Nos. 41729 and 41730, respectively, is described. The phages have lytic activity against *P. aeruginosa* strains, including mucoid and CF clinical isolate strains. Variant hages are also described, wherein said variants retain the phenotypic characteristics of said phage and wherein said phage and variants thereof have lytic activity against *P. aeruginosa* strains.

SEQUENCE LISTING

<110>  National University of Ireland, Cork

<120>  Pseudomonas aeruginosa Bacteriophage and uses thereof

<130>  P10551EP00/BP

<160>  6

<170>  PatentIn version 3.5

<210>  1
<211>  19
<212>  DNA
<213>  Artificial

<220>
<223>  Primer PA-GS-F

<400>  1
gacgggtgag taatgccta                                                    19


<210>  2
<211>  20
<212>  DNA
<213>  Artificial

<220>
<223>  Primer PA-GS-R

<400>  2
cactggtgtt ccttcctata                                                   20


<210>  3
<211>  19
<212>  DNA
<213>  Artificial

<220>
<223>  Primer PA-SS-F

<400>  3
gggggatctt cggacctca                                                    19


<210>  4
<211>  19
<212>  DNA
<213>  Artificial

<220>
<223>  Primer PA-SS-R

<400>  4
tccttagagt gcccacccg                                                    19


<210>  5
<211>  45441
<212>  DNA
<213>  Pseudomonas aeruginosa phage

<300>
<308>  NCIMB 41729
<309>  2010-06-24

<313>  (1)..(45441)

<400>  5
tcgccgttac gtaagcataa tgatacgaaa gagagcttat ttggcacatc ggcttgcatg      60

gctatacatg acttgcgagt ggccgcaact tgatatagac cacattgatg gagaccccct      120

gaataactcc tgggcaaatc tgcgcttggc aacgccaagg cagaacgcaa gaaacaggaa      180

gacaccctgc accaataaat ccggggtaaa gggcgtctat tgggttaagg ccaagaaccg      240

ttgggatgaa gtagttaaac aattaggacc tttagatagt gaagcagcac cagctccaga      300

agatgatggt aaaaatccag tttcaagagc ttttggtaat ttaattggtt tttattactg      360

gttcaatgag tccaattatt ggagtaattg ctgcagctgg tattattaag ggtatcttag      420

cccttctaac tcttcctcaa ttaggaagtt tgcttagtgt aaaccggggt agttaatttc      480

ggtggaaaaa tcatttcacc tgaacgtagg ccaacctcat ttgttcttgt tacacttgcg      540

gtaattattg gtggcttact ttatgccttt ttaacagtga aatttggttt agctcaaaag      600

atcattggac caaaagttga tagggtatta gaaaaattac atattagagt ttatttaccc      660

aagggtattc ataagaaaaa tcttagggta ttcagggggt ggatcggggg gaatatccct      720

tgatccccta ttgacttcca ttgatccccc catgttaatc aaagactgtc tatatccggg      780

ggtttgctgg gggactgata gtccatagac tgtatgaaaa accagtagac aaccccgtaa      840

agaatatggc gtaatggcgc cgccttgagg gaaaccgata ggcaccgggg gaaaccccag      900

gggcttgaca aaccccgcgg aaccgggtct aatggccccc acgttctacc taggccacca      960

tcccccttgc cgcagtgcta tccgggggct gccaaccagg gaagagcgcc caagtcaccc      1020

aagtataaga ccggcaggca tcgggggttg acaagggatc ggtgaagcgg tatagttcgc      1080

cccgtagtca acggcataac gtgcatgact ccctcgggat tagcgtagcc ttgacaagca      1140

agactgatgg ccgctcagca aaagagccta tgccagagaa ctggacgaac taacccccgt      1200

cagagggttg acaaggcaag cccaagcctc taacatgggc acccatcagg tgacggccac      1260

ctggggaaag tgtaccttcg acggtccttg gactgaacaa gggagaaacc cgaaagctga      1320

gggaataacg cacactccga aagagcgtag gtcctggcct tgtaccatag aaggcccgac      1380

tggtagcctg tcccagcgca atcaattgac agaccccgtg gttcaagcgc gacggggagt      1440

atgttgggtt agtatcaatg gcttagcgca gagggcgaag cttggtagat actggcgaag      1500

gtagggacga gtagacacga agactcccga ggatagccag aggacagacc ataacagcga      1560

tactaacgcg gaaaggggcc ggctacgcca atgccaaggt ttgccgataa acccctttgt      1620

cgtatcggga tgtgtgtccc ggctgatgat tcctaaagga tgaaacgacc atgactcagg      1680

ctcttgacaa aaagctgcgt cgcaaggcca accgcaaggc caaggctttg ggttacaacc      1740

ttgggaatct ggggaaggcc caacagcgta gtgagcagaa gtttgggatt attgcaagtt      1800

gtaacaagat cctcgacgac aagaccactt cgttgcaaga aaaggcaggt gcgcgtaaac      1860

gcaaggctct tatgagtacc gattggcgta accgtgaggt tactaacctt cggaactggt      1920

ataagcctgg caagtgtggt aactctgccg tcatcactgt cgaagtaaac aactgaggta      1980

aatacaatga gcgactacaa gcgtatcaat gggatcatca agaccattgc caaccggggt 2040
gctgccctgg acaagctggt tcaaaccacc ggcatggata tcctcaagca tatcgacgag 2100
catggcgagg tgtccttggc atgcaagctg ttcaacgcga tgccgcaagg ctcccgccgg 2160
ctggccctgg cccactggtt cgtagacaac ggcaagatcg aggccaatac cgacaaggaa 2220
aaggccaagg aattcccgtt cgtcttcgcc aaggacaagg ccactcgact ggagcgtgca 2280
gccgaaaaac cctggttcaa gtacaagaaa gagcgtgacg tggccgacga gttctccctg 2340
gaccaagcca tcgccgcatt caaggccaag gttcaacgcg ccatcgacaa gggccagctc 2400
caggcagcgg acgagcgtat cgccatcatc cagcgactgg aagtcaagga cgaagcgaag 2460
gctgcgtaac ggcgggtgcg actggggtgt gtactatgac gcgccccagg tctatcccta 2520
gagtgcatgg taggtccgtg cattgtagcg atagaccatc aggaggtttc cgtgggttac 2580
tgtatctttg ggcaggttca ttccatccaa ggccaggact atgtttctgg tttctgcgat 2640
gcaagggtag gtttcgacgc tggtatcgtg taccgctgga cattcaagga cggtaaattt 2700
gcctgcggtg tttacactct ggaaggtatc cgtctgaaca tctgcgggtg gatcaaatga 2760
agtcacctta cgaagcggcc catgaacgtg cccaaatgat taaccgtctc aagaaactca 2820
ctaggatgat ccgggtgcat cccgatcccc ggtggattgt tgagcgccag gaactcataa 2880
ggaaacttag caaatgacca tcgctatcat tgtatccagc atcggtatcg cctacttctt 2940
ctttcgtgat tggaaagagg aaatgggtat ctaatcccaa ctgatgaggc catggtgatt 3000
cctggccgaa acccccaccg gacctatggt cgcaggctgg ggagtcttgg gaaatcaaca 3060
aaggaaacta tcccgtgaaa cgcaacgact atcgtaaggt tagccgcaac atgcaagcca 3120
tcgaggctat cgaccgcaag attgccaagg ctgtccgtga gttgactaac tccggaggaa 3180
accacgttgg taaaaccctg gagttgaaca agctccgtgc caagcggcca tctttggtta 3240
agaacagggc gcggtgatgg catggaagta tattgcatgt gccgccatac gctcacgttc 3300
caatgggttg ttgctggtag tgtgggtggt tctcttcgtc ctgtcctcga ccttggacag 3360
catccctttt gcatcgtgag gtagccccat ggatatcaag gtgtggcccc gcaacggagt 3420
aaccctcagt tctctggtta agtcccaaac attcctcatc ggtggggatg tgtacatggt 3480
ctgcgaactc cgcagcatta agatgaaggg cgaatccgac gaggttcccg ttctcaatct 3540
caagactggc aacgtcattt acatgcctcc ggtgagtctg gtatacccag taactgccga 3600
gctgaattgc tacgaggtgt aacatgaaac tcaactacaa tatgctcacc agtaagatcc 3660
gggttgcctc ttgggatgat ctggagtctg gtcgtctgta catccgccag gacgaggacg 3720
ggactgcaaa cccgcgtgtc tttatgtaca tggaagttgc agatgaattc ttcatcgtgg 3780
atatcttcgg tggacccgtg ggtgccgtat acttcagcgg cgatgttgac tgcgggttct 3840
accccgtgac catcaaggct gcgtccatcg aagtccatcg gttcggacct ccggcctagg 3900
gtgtgtacta tgacgcccgc ccaatacatc taccactggt tggtatccaa ggttgtcatc 3960
cggcgatacc agcctaaccc caagctgtac cccaactggt ctgttactca catccgtgtg 4020

tcgatcttcg gcaaacgtgc aggtatcgtc tatgaaatcc actgaacccc aggtttactg 4080
gctggctagc cccgatggtg aattccacct gaaggtcatc aacttcggcc agttcatcac 4140
cgaaaccctt tacgagctcg gcatcccggt ggaccacaag gttcatcggg tccactgagg 4200
tgacccatga ctactttcac cgtagcagta tcccagccac gtagcgactt gcacatcagt 4260
aagcgtaaag atggtgtgta tgtaagcctc acgggcgatt cggtctacct caaacaaggc 4320
gggaccgtga cgattatcac caaggttggt ttctccatct tcgcagccaa tgtggtatac 4380
aaccatggtg aactctaccc agcgaaggaa gtccacgtgt cagccaggat gtgggggccg 4440
tatgaagagt aaaccaatga tcggccaaat catggcccag gagcggcgaa tgaagcgtcg 4500
agtagagaaa cgagggttca acatgacccg tcaggaaggc ccccaggagc cccgtggtga 4560
gattggcttc accttggcag ccgtaggtgt ggagtcgagc cgatcggctt acctgcggca 4620
cgccagggag gctatgatcc aatccgggga ggattgcccc cactgtatgc cagtcttcgg 4680
tcccaaagag ggtcgttgct gcaactgcgc gagggattgg tgatgaccat ccaagaaatc 4740
ctcatgatct ccgccactat cttcttcggg ttggcggtct acgggttcac atcctgcctg 4800
atctatgcgg tattggaaga gtgcgggatg aggtgggggt cagatcgtga ttgggcagtt 4860
ctcctgtcct tcttctggcc cttgtccctg ccggggatga tcctgtactt cgcctgctac 4920
aggcccctca aggaagtctt catatcagcc aagcggctct tcagcggcca ctacaggagg 4980
ttctaatgga aagcgtaatc gtctatgcag cggcggcctt cttcctcatg atgtgcctct 5040
tcatctactt cctcgtggcc gccgtaggag tgtgcgtgac cttggcccta ttcgaggata 5100
gcggaaggat caaggaagag cacgccatcg tcattggcgg gttgtggccc ctatcactcc 5160
cactgatcct cttcgggttg atgacctatt acctcatctt cccgatgaaa ctcttcatca 5220
tatccttcta caaaattatc aaaggagcca tcaaatgaga gagcataact tcccctcgtg 5280
ctgcaccgcc aaaatctaca ccggcatggg tcctacgagt accgctgact accaatcgat 5340
caaggactcc gaggggttca gccccaagaa gtttgcaatg gatgttatcc aggccctggc 5400
cgatcagtac cacgaaggtg aggccctcat ggtcttctcc ctgaacaatg agcagcgggt 5460
cgccgaccgt atcctcagtc gcctggggag caccagcaac ccttgggcca gcagcgacaa 5520
ccacgacacc ctcgtgcgag tccacatcat ccgggtccgt gacacctggg gcaagctgtg 5580
ccgggagaac ctggtccgct acgactggga ccacggatcc atcccgagcg aggacttcat 5640
cagcaagttg gttaaaggtt tgtaacattt gacctattga ctttctgctg gatgtgtggt 5700
ataataacct taaggtgccg ggggttgctc ttatacctat aggatacctt aaggtctacc 5760
tttatatctt acttattatg tgaggtatca aatgctcaag ctctataccc ggactatgtt 5820
ggtggctatc cctctgtgga ttctctacct tctgatcctc ccctacctga tgacctcgga 5880
ttcaacctcg gctagcgccc tgggtgccgg tctgatggtt gcatccatct tctatggcct 5940
cttcttcttg aagtacctgt acaactacag taagcgactg gagaaaaagg tcaagacttt 6000
gggggtggat gatgagtgag tcggagaaac ccatcgtcag tttcgagaag ttcatgcagg 6060

ttatgtatga ccgcgacgag tacgctaagc gtctggaagt agccacggcc aagatccagg 6120
aattggagaa gtcattggcg atggcgctcg acgcagcagc gaagggcgat gatgctcgcc 6180
atcagtgcgg cggaatggag atggagatcc aggaacttcg tgatgaattg gcaggactga 6240
aaaatgaagt aaaggttcgt acaacccaag aagtagtccc ccacttctgg cgtcaccgag 6300
ggggtggtcg aatcttcgtg tcaggcagac tgaaccttga caccgagacg cttcccttcg 6360
tatgcctgga gactggcaac agttgggaca ttcaaccagg gcgggtagag tatatcttcg 6420
aattacttcc caagggccat accttcacgg tggaggtctg atgtactatg acgaacagca 6480
atggggaccg cacccaaggg caccgccggt gttaccgtac cagtgaggta acttcggaca 6540
tatatgacgc tatctacaat atcccagcaa tctctggatc ccccaagagg ccaccggatg 6600
atgattgggt ttactacctc ctgatggtag tcctcttcat cagcctgtgc atggccttcc 6660
ttactcccct catcaccagc aaggatacag cctatgaaag ccaaagtctt ctacgagcga 6720
ctgaaaacct ccggcgagct gaccctgaat ggcggcatct acaaggccac caaggttcct 6780
gcgccccaca agggtaagca gcagcgtcgc ggccccttct aagagggcgt tagtaatatc 6840
cctaggaact ccctggggat attgctggct aactcaacca aggagaaaca agatggctcg 6900
actcaagtac gcaatgaacc acgggaactc cctcgtcatg atgaccagta gctcctgctt 6960
tggcgctctg gaggctaagt acttctcgaa ctggaacggt tggttctaca tccaagctcg 7020
ccgtaccccc aagtacatcc gccagatgaa caagatgttc gctgacatta ccctgacggt 7080
ggccaacacc aaggtaaaag tccccaagat ccaccactgg cgcatggggt ccaaggccaa 7140
gagcgcaccc tggaatcgat tccccaacca gaaggtattc accgatgacc gcatgtgggg 7200
taacgattac gtgtgcggta tctacatgaa cccccggacc actaccccgg aggtctggta 7260
tcccgtggtc aaactcctct tcaagtgggt atcctctggt gcgttcgatt gggaggaaga 7320
ggcccatctg gagttggccg ataagcacgg attcctccta tcctacgtgg caatgtcctg 7380
ctacaacctg atggccaatg ggtacaccga gcaccccctg agaaacgtcc acggcaaggt 7440
gtggaagccc gaggccgtcg aaagactcct caagggcgac tgggaaatgc ccaaggtcga 7500
agaccaagga acctatggcc ggtggttgaa gcaggaggat ggcacctggg acttcatcct 7560
gggggttact cctacttact atggtttcaa taaggagaag atcgagggta tgcggaagac 7620
gatggacaag gagttccaga tcaccatcta ccctcagccc tgctacctgg ggaaatggga 7680
ggatagccac tggcccttct tccagagcaa caagctgctt cctcagcacg taccctccgt 7740
gaagaacgcg ggggagcgta tcgagttgat accccctcac ctgtctcgtc cgatgaatgg 7800
ggcacgaaaa gagtccttcc tccccagtct ggatgcaatc tgtcaacacc tcaagaaact 7860
catgggagat taatcatggg aatgtatgcg gcagaagtaa tcgaccggga gggaagaccc 7920
ttctcccaag ttaagaacct caatggtcat acccgttgct acggggactt cgaggacagg 7980
aatggtgaag cgggctgcgt tgtccacttc atttcccata ctgccaacga agtgacccga 8040
aggatcgctg tggacttcct caacagtgat ttcgtcaacc ggatgaccga tggtgctatc 8100

ctggcggcgt ccgaggatct ccccaaggag tggtgggatg gcaatggtga tctcaagccc 8160
atcggggact tcgaactccc acgggcgatg agccgtttct atgtccgggt agatatggac 8220
aaaaccggag gccccatcat caatgccctg cggatggccc acatgctggt ccacagcagc 8280
catcagccga acctgctaca gaaactggac cccgtggttg ctgtcctcgt ggcctgcgca 8340
aggattggcc acaacttcta ttgcgacagc tacgtcagca tgatccccgt gactgctggc 8400
cagtatcgtg agcaatgcga gaaggagggc atagaggtag atgactccta cctgacggag 8460
acgatggcca aggctttggg ccttgggcgg attgatagct ggggtgatta tgtacaagcc 8520
ccagagcttc tccacgggcc caccctgtac gatatccaag atatcatggg tcgtactcgg 8580
gaaaccttca agcgtctcta cagcggccac aaggaccctc tctggcaggg gtataccaaa 8640
gactgccgag ctatcgagga actcggtctg gatgggtacg aggatgatga tatcatcagc 8700
gtgtccgcag gtatcactta cgacctcaac gacaagaacc gtgagcaata tgaggtaatg 8760
cctctcgata gttacgagga caccctcatt aaattcttca ccgaaaacca actgattaaa 8820
taggagaact gccatgaaaa atatccctct gattggtgct gacccggaag tcttcgtagg 8880
cttcggtcgt gaacccgcat ctgccatcgg cttcgtcggt ggtactaaag acgagcccat 8940
cccggtggaa ggtggggcgt tgcaggagga taacgttctg ctggagtaca acattcatcc 9000
ggccagctcc ttcgagcaat tccaccacgg catcaccagc gtcctgaaca tcggacgcga 9060
cacaattcgc cccttcggcc tggaagtcat cgacaacctg tcctcccacc tctacgagga 9120
agatgttctc cgtggcttcg gtgatgcagc attcgtcttc ggttgtgagc cggactacaa 9180
cgcctggacc cgcagccgta accgtatgcc gaccgatgca cccccgaccc ttcgtaccgc 9240
tggcggtcac atccacatcg gcttcgctga cgacctgcct gttaccaagg ccaactgccg 9300
tgcggtgatc caagcctgcg atatctacct gggcctggcc tcggtgctga tcgacggcga 9360
caccgaccgt aagctgctct acggtaaggc cggtgcctgc cgctacaagc cctatggtgt 9420
cgagtatcgt accttgtcca acttctggat cttctccgat gagttgatta agtgggcata 9480
cgactcggcc ctcaaggctt actccgtggt agtcgaaggc ggcctggatg acgccatcgc 9540
agccgtaggc ggtgcagccg aggtacaacg tatcatcaac gagaacgacg cagtggcagc 9600
tctggcggcc ctggagaagc tgggggtggc tcatgaatga cctgaacaac gatcatccgc 9660
tggccggcga cttcaccatg tactacggaa acacctatgt cttcttcgag aaggatggtg 9720
ttccccatgt gatgtacgtc gagggcaccg aggaagatgg cgacgatacc cggttcgatg 9780
ggttcgttct cctgggacgt acctacaccc tggatgacaa cgagggccga tattctcggg 9840
ttcgcttcag tgatgtagtg accgacagac cggccagcgg gtactatgac attcacggga 9900
cgggcttccg taatgtttac gtcaccttcg ctgtcaacaa ccgctctcag aagaaaggtc 9960
tggacccccg gaacttcatc ctgaacggga ttggcagcaa tggcagcctg tatccggtgc 10020
atatcctccg gatcttcctg caatcccgag acatgatctc cagcccatcc catcgggact 10080
tctacaccga ggaaggcaag cgagtcttct ggaagggtat gttggtaggt aatctggtgg 10140

aaggccaact ggttgtcaat gatgaattca agaaacagga gggactgcta tgtcggctct 10200
atactctctg attggccacc gcaagaggga tcttcctgag atccaagccc atgaggaact 10260
cccagcaatc gcgtccttcg ggttcgaggt tgaactggag ggttccgatg aatggccccg 10320
tatcgatggg tggaaccgca aggaagatgg ttccctccgc agtggtatgg agtacatctt 10380
cgatggccct tcgagcggtg cagaggcaat ccaacgggta gaccgtttcg ctgccaccat 10440
ggaagctgtc aagccggcac ccaccttccg ctgctccacc caccttcaca tggacatgcg 10500
ggactcatcg atggatgttc tcatcaagac cgtcctgacc tacatggtgt tcgaggatgt 10560
gttcttcgac cactgtgaac cctaccgccg ggatagcaac ttctgtatcc ccttcttctc 10620
caacgattgg ctggctcaaa ccttcggtcg tcgtctcctg gctgtcaatg aagacggtct 10680
tcggattcga ggcctgatga actggcccaa gtactccgcc ctgaacctcc agaccgttac 10740
gaccttcggg tccatcgagt tccggggctc ccatgcgatg acctctcggg atgaaatggt 10800
cgggctgatg cagcgtatgc tctgcctcaa ggccttcgcc cagggtcacg aaggggttga 10860
cacctctgag tttctggagg tactgaccaa tgtggatctg cgggatgtgt tccacctggg 10920
gctctccccg gactacaaca tgtctccggg tggccgtgag atgggtctgg cggctgctgc 10980
cttggctatc agcacgggta ttcagatcgc caatggtgtt gacccgatgg aggaagagca 11040
ggatcgtcag cggcgcatcc gtgaacaaga gcgtgctgaa cagaaccgcc gtcgtgcaga 11100
actcactgcg gcagcgacaa ctcctttgag cttctctgag gagggtgtca atcggttcaa 11160
catcgcaagg actccagggg aggtatctaa cctgggtaat gccttgatga ccgtgaccgc 11220
cctgcgtggt atgggttatc aggatgcaac cgtccgtaac gtcttcgatc tcactgacga 11280
gggggtagcg gctcaggttg tacaagcttt cgttatgctg atgaccgacc ataccaactt 11340
cgtcacccgt acctacggcg taaacattca ccaagaactt tactaaggag taacacctat 11400
gtgcggatta gtaggcttct gttctacaac taacgcgagt gataatgaaa tcgctcttct 11460
caagaacctc ctggcggctg atatcgtccg tggtgcccat gccacgggtt tggccaaggt 11520
cgatccgatc aagaacgagg tcagtatcca caagatggct gtagatgcct acgacttcct 11580
ggcccacgag gataccaaga agtttctcac ggaaaaccgt gcccgcatct acatcggaca 11640
caaccgttac gcaaccatgg gtgacaaagg ggaccatgag aatgctcacc ccttccaagt 11700
ggaccacatc acgatggttc acaatggcac cgtggatgcc tggggtatcg atctgctgga 11760
gggtaacgat accctcaagg ttgattccca catggttgca gccaccatcg ccaagcatgg 11820
gattcgtagc accctggagg aacgcttctc gggtgctgct gccctggtct ggtgggatgc 11880
caaggagcgc agcctgaact tcgttcgtaa ctctgaacga cctctgttct tcgctgttac 11940
taccacgggg accatcgtat gggcatccga ggattggatg ctcaagtcct tcctggagcg 12000
tgcatccagc aaaatcaagc ttcgcggtaa catcgtggag ctggctgaag aaactctggt 12060
atccatcccg ttcacggagg ccggcgtgcg gaagggtgta gagcccatca gctctccggt 12120
cacctttctc gacctaccga ttccagaaag ttatcgtcag gcagaaaagt tctgggggaa 12180

ctatgtcggg gcagacgcct ggaagcaata cctcggggag tactatgacg atcaaggaag 12240
caaaggcagc caagcgggaa cgcaagctgg ccaaaaagct tcgtcgtcaa gaacggcttc 12300
ggatgaggcg tatgcaagga acaccctgcg aatcaacaac aatctccgat ccgcaggaag 12360
ctaccgtaag catcacagcc tcctcacctt ccgaatcgtc gaagtccaac cctacaaggc 12420
cgaccctggc tttggtacag tcgtcgggat cgacctcgat gaaggaatgg tcgtcgaagc 12480
ctacggactc aacgtcgaag ccctcgaagg atacaccacc ctccgtggaa gtatctccaa 12540
cgcctacttc atcggtgaag gacgcgattt taaagttgtg gtcgaaggtg ttgccgtaag 12600
ttgccttgac ccaaagcaca aggccagtcc ggtggatggg accccgttgc agatcattgg 12660
aacgcggaca tccccttcta aagttcttca gatgaagtcc gaggctggca atactccacc 12720
ggcgaatatc tcgtatccac tgaagtgcca agggcatacc ttcgtgaaca gctacgtctt 12780
ccaagacttt gtatgtcgtg gttgcgctgt ctgcggggat atccctacag cctatgacca 12840
acggaaccgt gatcttacgg tgtacgaagg tagcaagttc accggcgagc ttaacgagtg 12900
tgagttcgtc tgtggcaaat gtgtccgtga aggactttcc gaagcagtat aaggaggtca 12960
catgacccag caattcaaac gtcaagtagt aatccacatg gaaaccgacg caacccgcaa 13020
gtatccgttc tccaaagaga ctctggataa gatggcggat atccgtcggc tgaaggagca 13080
ggagctgaat gaggctaatc cgggggaaga attcctggtg cctgccccga tcgtcctggc 13140
cgaagcggtg gatatgctcc acgagtacta cttcgagtaa ggtagtgcgt tagtaatagc 13200
ccctggccga cccatgccgg ttcctaagat gtgtacatgg ggttcaactt aggacctcag 13260
gggctattgc tggcttcact aacctcaacg aaacaggaga tacactatga tctatatcta 13320
caaaggcaac cgtgcatcca atggtgctgt agcccttcgc aatgccctgg gtggacgaat 13380
tcttcgttcc gagggttcta cctaccgtgg ccgtcagggc tccatggtta tcaactgggg 13440
tactcagaac caagaggctg aacgtctccg tcgcctggcc cccatcttcc ttaacaaccc 13500
ccaggttgta tcgatggtga ccaagaagga tgagttcttc cgccatatgc aaactgtagg 13560
cccggacctg atccctcgct ggactaacaa cttcgatgac gccgttgcct tggttaataa 13620
tggtggtggc cgtatcttcg ttcgtaccca actcaatggc catagtggcc aggggatctc 13680
cctgctggtg tctactcggg atgcagaaat ccaggctatc cagcagttga tccgcagcag 13740
gcagtacccg gtatatgtca tgggtgttac gaatatcccc gatgaagtaa tcaatgctcg 13800
tctgttcacc cagggcatcg taggccgtcg taccgagttc cgaatccacg tggtgcgtgg 13860
ccgggctatc ctgtcccagg ttaagctccg ccgtaacgtc gaggagaatg tggcagatga 13920
ttcccagagc atcgtccgca acgtagccgg cggctgggtc tacggtgtca atgatgccga 13980
agaacgccag ggccatgctc aggcagttgg cgcagcaatc caagcggtca acttctgtgg 14040
cctggacttc ggtgctgtag atatcgtcta caagcacgag actgaccgcg cctacgttct 14100
ggagatcaac actgctccgg gtctggatga agggggtagc gccctggagg catacaccca 14160
ggcattcacc aaaatctacg aagagggcgt aatctgatgt ttgtttttgt ttatggaaca 14220

ctgatgcacg gcttcggcaa ccactgtctt ctagagggcg cccgattcgt cgggcatgcc 14280
acctcccaag agcgaggccg tatgtactcc tgcgggggat tccccatcct gtccttcaaa 14340
caccgtgagg atcttgtcac gggtgaagtg tgggaactcc ccggtgggga cgaaggtaag 14400
gaaatgttaa acaacctgga tcgccttgag ggttatccgg gttggtacga tcgtagcccc 14460
aagaactttc gtatcaacgg ggaaatgatt accgcccttg tctaccacca agacgaagat 14520
caagacctgc atatcgtcga gggtgctgac tggcgtaaat ttgttaataa aaggaatgct 14580
gcatgaccca tactgatgta gttaaggccc acaagattta ccgtgatatc cccaacctga 14640
tgaaaaccga tgatgagggg aaacgtcagc tcctccgtcg gatcatcggc ctgatggcat 14700
cccagagaca actacaggac tccatcagtg cgtctgtaga gcctgctacg gatgactcgg 14760
attacagcgg gtactatgac gagtctgctg gtattcgctt catgtgggag cgtctcaagg 14820
ccgtagaaga tgagttgcgc ctgctggaga aacaattggg tgacttctcc tacccctatc 14880
tgacctatcc gggtaatgtt aacccggttc cttccctggt cctcaagagt caactggagg 14940
gttaaatgaa gaaaatcatc ggtgatacgg cttgtccggg ttgccgagct aaaggtgggg 15000
atcacacagg taatcacctc atcttgttca tcgacgaaga aaaaggtagc cgcttcggta 15060
agtgcaaccg ttgtggccac tatgaggttc tggacgaggg ctttgtagta cctaagcgaa 15120
cggagaaatc cgaagaggat atcctccggg aagtcgctga ggtccaagaa taccccttca 15180
aggccctcga ctcccgcaag atcagtaaaa ccatcgcaga acgctatggg gttcgtgtgg 15240
gattgtccat ggagaatggt gtagatgtga tcgaacacta ctacccacgg acaaaggatg 15300
gcaaataccg tgcgtataac gtccggatct tggatcctaa ggcgttttac tatcggggta 15360
gccccagggg tggggtcgat ccgttcggcc ttgagacgct ctttcacaag gacgtaggac 15420
accttaagct tattatctct gaggacgagc tttcggctat gtcgattgct cagatcatgg 15480
aaagtaagtt accagagaag tggaaacacc tacggcaggc atccattagc tggtcttctg 15540
gagttgggtc tgctggtcga gacattggtt tcctcaagga atctgggatt ctcgataggt 15600
tccaagaggt tgtctactgt cacgatgctg acgaggaagg tcgcaagtct gtcgagaggg 15660
tccgtgccct cctccctgac tgtaagtttg tcgaaatgcc cctcaaggat gctaacgaca 15720
tgcttatgcg caaccgggca gatgaagtct atcagctcat tcgattcggc agtaaggtga 15780
aatccccaga ctgtgctgtt accgtagatg aggtctttgc agaagccctt gagccaccta 15840
agtggggcaa gagttacccc tgggaaggcc tgacgaatct aactatgggt cagcgcgacg 15900
gtgaaatcgt tggtgtaggt ggtggtactg gcattggtaa gaccttgctg gctcaccaca 15960
ttgcagcatg gaatatcatc gaacatgggg aaaacgtagg aaccttcctt ctggaagagc 16020
aagttgctat gacactcaag aacatcgcag gtaaggtagc tcaaagggca ttccaccggc 16080
ccgatcttga gtgggacgta gaggcgttca aggatgctgc tggcaagctt cggggcaagc 16140
tcttcatgtg gaagaacaag ggtcagaatg attgggatca tatcaaggag tgtattcgct 16200
attgggcggt cgctatggat gttaagaccg tcatccttga caacatgact gccatgacta 16260

accacttgtc tccatccgaa atgaacactg agattgctcg tatctgcaca gagttggccg 16320
ggatggctga cgagttgggt atccgagtat tcatcttctc ccaccttaac ccgccaaagg 16380
gtaaccgtac ccacgaggaa ggggctgagg tgaaggaaag tcaattcact ggtagccgtg 16440
cgatgcagcg ttggtgccaa ctgatgattg gcttcgagag gaacaagcaa gcctctggag 16500
aagagaagca cgagagccgt atccgggtca tcaaggatcg gaactacggt aacaccgggc 16560
tagtgttcac caaatataac cctgacacgg gttgcctaac ggagcgtgaa ggtagctatg 16620
atgaagtacc cgaggacgac gagtccccaa tttgattacg ttatctacga cctagagggt 16680
gatgcccttt acgataacgt cacaaggctt tggtgcgctg ttcttgtaga tataccaact 16740
caggtagtcc ggggattccg gcctgaggaa atggagacat tctacaggat catcgcccat 16800
gcaaagttcg tagtcgggca caacatcctg gactacgaca acagggtcct tgagaagctt 16860
catggtatcg tgattccaga agaacggtct tacgatacct tggtcgcctc ccgactcact 16920
tggcctgacc gtcctatggg acactcgctg ggagcttggg gtcgattcct gaagtgccac 16980
aagggtgatt tcaacgactt ctccaagttc tccgaggaaa tgtttgagta ttgcctacag 17040
gatggagttg ttagtatggc actcttcaac tacctgctta aggttctcgg gatgacctgg 17100
gaggagctag tagagtggag gacaggttct tggctaaacg tgtgaggaac tacaagcgtg 17160
aaagagaact ggctatacga cgcggcgaaa cgggcgttgg atctaagtca ggagatgctc 17220
aacggcaccg cgcccgccga aaggtggaaa agcgtcttgg acggaagctc aggtctgacg 17280
aggtggtcga tcacatcaaa cgtgttaaag atggtggtgg taacggggat tctaacctcc 17340
gtgtccgtag ccactcttct aatgctgctg atggtggtcg tgttgggaat cgtaaggcca 17400
aaggcattcg aaagaaaaag taaatgaggt ggggccttcg ggcccctgag gatctaggat 17460
gaagaagtac gtggtcaatg ttcagtttca ggacaccaag gtgttctatg ctgacttcta 17520
tcggatcgaa ggagagatca ttcgattctt ccaaggcgag gctgattcta accccttgct 17580
caaccgcagc ctagaagtag gcgcagtccg tggatggact tccgtagagg agaaagaaga 17640
tggacaataa gtacaagcct acgccccagg atgaagcccg caaggaagca gccctagagg 17700
cagccttcag tggtggctgg ggtggtcccg aggtcgatgc tgatgacttc gagctgggta 17760
atgcctgcgg gttagatccc gaggtatgtg agtcgtgtca gtaaccctac aagtccttgt 17820
catcttctgg agtgcattct ttcaggtctt cctcttgggg ttgaactcta agcttctcag 17880
ggatgacaaa atcaaggctg gttttgtagt ctcctggtgc atcaccctcg ctcagttcgc 17940
ctatatcaag gcagtaggat cgagccatct ggatgtagga tggttcatct ttgtgagcgg 18000
ttggggtggt gcccttggga ttaccttcgc ccaatacttc tatcgctggt acgacaaggt 18060
tttccacaaa aaggcttgac aaaagttact ttatgtggta taataacctt gtggtgccgg 18120
gggttgctct tatatctaaa ggagagacac atggaatctc actacgcatg taacctgatg 18180
gtaactgatg ccatagccca agctaaatca gaggagaatc gtaagaaatt catccgagcc 18240
tatctgatga atcccaaagt acccctccag gctgcacgta gcatcttctt caagctgccc 18300

aaaggctacc ttggctctac ttattttagt ggccatggta aggtagagcg gtattccgac 18360
aagaagcaac aacgggttgt cctcaaggat ctcagcctct atgacgactt ctacattgac 18420
cgtcacgaaa cgttcgatcc tcggaacctc ctacgggact cccttttcaa cctccactac 18480
tacctgaaga acaacggtag tgaggagcgt atccgtaaag agcaacgtag ggttgacctg 18540
gccaacttcc ttgtggacct tgctatttgc aggggcgtat ccaccttcgt ccatgattgg 18600
tgatgacatg agtaaagtga aaagtgtgct gatggagcgt atcgacgact accttctaaa 18660
gcaggttgcc gtagccttcc tggagcagca atggaagctg gaccgtagtg gtaccgtgga 18720
ctacctctcc tacctggagg gtgtatccca cgaagctgtg gagacagtag tagagaatct 18780
ggcggagcgt cttaaggggg attaagtgga ttggagaaag tcactgttcg ttgagcacaa 18840
ggtagctgat atcattagcc gccagagtaa acggggggtc tacttccaga ctcaacgggc 18900
taagtggctt atccacgtac tcaacgaacg aatcctcaag attgacctag aggcagtccc 18960
ccagatgcct cccatgatcg tccaagcggg agcattcagt aagccattcc taaagagtgg 19020
actgcctaac aaacgcttgg tcgctctatg gcaacgattg ggtcacttcg aggtatctgg 19080
accctttaca gcaatcgagt acaaggactt tgacctgggt aaaaccgcaa ggttcaaaga 19140
ctggatgctg tctcaaggct ggatacctga ccagtggaat atcaaggaca ttacagtcgg 19200
cactgatggc aagaagctac gtggatccga tctgaatggt gccttgaacc gctacatcga 19260
agacctccga agctccccct ctggactact ccgaatgaag ctccagggaa tcatccctgg 19320
gaagactaca gtcggtgagg tcaagaggaa gcttgagaaa caacgtaagg ttctcactac 19380
gccgaagatg actgagacct ccatggatac cgtccagggg gatcttggta agcttgtgat 19440
gcagcgaatg gtttgggctc accgacgctc cctccttcag ggattagtcg agcaggtgaa 19500
gcctaatgga cgcctagagg ggagtgctaa cccctgtgca acacccacgg gccgtatgag 19560
acaccgtgta gtagttaata tccccgctgc tcgttctccc ttcgggcctg aaatccgagg 19620
gttgttccag gggactccta atgctggtga atggaaatgg actgtcctcc gccgcgacat 19680
aggtgagaac gaaagggtaa gaccccacac taacatcgtg gaggtcctca aaggtggtaa 19740
gtggaagacg gtaggcaagt accgagtata cgtcccagcg aatcaactga tcttcgtggg 19800
ctatgacggt gctggtctag agcttaggat gcttgcctcc tacattggcg acccagacta 19860
tacccgagag gttgtagatg gtgacgtcca tactgccaac caagtagccg cagggcttcc 19920
tacccgagac gatgctaaga cgttcatcta cgccttcatc tacggtgctg gggatgcaaa 19980
aattgggcag atcattggtg gcacgagggc cgatggggct aggctgaggg agcaattcct 20040
taaggccaac ccagagctgg ctaagctgat tgagagggtt aagcaggaag ccgaaagggg 20100
ctaccttgta ggactcgacg gacgtaagct aacgatgcgc cgtagtgagt ctggagaggt 20160
gatggtccac aaggcattga acaccctcct ccaagctgct ggtgctattg tcatgaagtg 20220
ggcaatggtg atcctagatg agaaggtacg acgggctgga ctcaaggcgt ggaaggtttt 20280
ggatatccac gatgaaggcc agtgggagtg ccaccctgag gaccttgcga ctctccgtgg 20340

attcatggag acttgtgtca aggaagctgg ggaaatcttg ggttgtaact gtcctcttgc 20400
cagtgattct atcgcaggta ggtcctggta tgacacacac tgatacatct aggggttgac 20460
ttaagacccc ttttgtgcta taatatcttc ttccctacaa gaggtatatt taatgtctaa 20520
gaaagtatcc caacgattca ccttcccggt agcgaagctg atcttcccct acatcgtaac 20580
tccggatacc gagtacggtg aggtttacca agttactatc tgcattccga ccaaggagca 20640
agccgatgag ctggtagcca agatggagtc caaggattca cgcctcaaag gcaccatcaa 20700
gtacaccgag cgtgacggtg aattcctgtt caaggtcaag cagaagaagc acgttgactg 20760
gatgcaggat ggtgagcgta agtccgctgt tatgaaaccg attgtcctga ccagcgacaa 20820
caagccgtat gatggcccca atccgtgggg tggctctact ggcgaagtcg gtatcctgat 20880
cgaaacccag aagggtgctc ggggtaaagg cactatcact gcccttcgtc tccgtggtgt 20940
acgtctccac gagattgtat ctggtggtga tggtgaggat gatccactgt tcggtggtgg 21000
tttcaccgag gaagaggaca agcctgagga cgtatttggc gaggacttcg atgacgaaga 21060
tgctcctatc taagagccta gaggatcatg aggcgggggt atgccctttg ggctgcccct 21120
actgcttagc tgactatgag agaatttggg gtgtacgagt ggtaagttct atagctgcat 21180
ctaatgataa ggtagaggtg gatcccaatg gaatcaaggc aggagatccg ggggctaagc 21240
tcgataaggg caaggtggat gttggaatca tcttcgaagc gttcccgaga gctctttatg 21300
cggtggcaca agttgctaac ttcggagcaa gcaaatatag tcgcgggggt tggaggttcg 21360
tcgagaacgg aatccagcga tatgatgctg ccttcggacg acacctcctt gagcgacaca 21420
agggtaagtt tttggacccc caaagtaatc taccccaccg gtatcacgaa gtttggaacg 21480
ccctagcagc cctagagctg gaaatccaac aagaggaggg ttctaatgga acttctactg 21540
gatccgaggg ctaagaccat tcccagcaat tactctgtca aaggccttga ggtagacctt 21600
ggggaaaccc caggctacag cctgacggat gctgctatgg agaaagccaa gcgtcaagag 21660
agtgagtatt atgactggaa gggctacgaa gctctagcca accccgtggt agaacatccc 21720
gagtatcggt ccaagggtga agcatttgcc ctcaccaagc attgggaaga gattcttaag 21780
aaaggtccgg agggctctaa ggatgatcgc ggggattgat ggtgacgtac ttcgatacga 21840
gctaggccac atcgctatga agaaggaaca catcttcgac attgaggtag ttaggccctg 21900
gcccgaggaa gaagttaacg agcttgtaga taaaaagata gaacaaatca tagagagagt 21960
aggcgctgat gactatgaag tgtacctcac tggaaaagat aatttccgat tggagcttgc 22020
gacgatccgc caatataagg ggactcgcgt cggaaataca aagccacatc actggcaaac 22080
agtgtcagac cgacttaagg acaagtgggg agcagtcact gtcaacggta tcgaggctga 22140
tgattggctc ggggttcggg gaactgagga gggggataac tttacggcat gttctcgcga 22200
caaggatatc cgccaaatca ccgagtgcca ccattacagt tgggcctgtg gcgataagca 22260
gccagaattg ggaccctttc gtaacgaggg ccttggacga gtgtccgctt cttggagact 22320
ctatgggaag aagaagcccc aaaagtcctg gaagcttgag ggctacggta cggcattcct 22380

ctatggacaa ctccttgttg gtgactctgt ggataacata ccaggttgtc ctgaaatcgg 22440
gcctaagtca gcggcagatt tgcttgggca ccttactact gagaaggatc tcttccaggc 22500
ttgcgcctac gagtaccaaa aggtctatgg agataattgg aaggagtacc ttactgagaa 22560
ctttcgtctc ctctacctca tcagagaccg taattggttt gatattgaac agtccggtaa 22620
cgagcttaat tgccgagtaa taagacattg ggagatacct tatgaagatg gagaactatt 22680
ctattgaaga ggcccttaga gtatgtcggg aactcttcaa gaccctagag gaaaccacaa 22740
aattcactgg ccttcgggtt ggaggcgggt tcctccggga ttgtgacaac ggggttgtgc 22800
cgaaggatat tgatatctac gttgtagagc cccgtaccct ccaagaggtc accatccgta 22860
atcgggtccg tgtagttcct caggaagtgg atgaggatac ccttgaggtt aacgtcatcc 22920
gattcttcaa cgccattggc cataagaaag tacggctagg ggataaagag gttaataacg 22980
actatcccga ttcgtttaag gtttgggaat cttgtgagtt ccccgaggga cacctcccgg 23040
ttaacctgat tgtgtgtaag caggagcacc cctgcgtatt tgacattggc ctgtgcagta 23100
tcagcttctg gaatggggat cgtcagttgc tctactctcc tgactacacc agggataagg 23160
gcaataagtg gattaccctc attaacattg ctgacccgtt gattggccac actgttcatg 23220
agaacattat ctcagatgga gcagtagatc gtcttgtgaa tcacatccgt agggtcatgg 23280
agaaataccc tgaccataaa gtcaaactct cggataacct tatttcctgg gtcaatttgg 23340
ttggtagcaa taccagccat agtgtcctga aggttatcgg gagacttcaa gaggagaata 23400
tcattgagcg tccaggggag attcttccgg cccaagctga agtcattgat tgggacgccc 23460
tacgactcca acaccgagaa gatcgtccac gagacgatgc cgtcgatgga gttcaagccg 23520
caaccactca cttacgtggt gaagcacaag tattaccccg acttcaaggc ctctttagag 23580
actggctcat caacgaagac tttgctggtc gaggtcaagg ggtacttcca agaggcgtct 23640
gaggcggcca agtatgtctg ggtgagggag agtctccccc cagatacaga acttgtgttc 23700
atcttcgaga agcccgatac agcgtgccat tggcttaaga agcgcaaaga tggtacaaag 23760
caaactatgg cggagtgggc cgaacgaaat ggcttccgct ggttcactct agagactttc 23820
aaggagtcct tccataatga gtaagcgcta taatgaagat accctcgtca ttgcggatac 23880
ccaagtccgc ccaggggtca atgttgacca cctcgggaat ctcggggaat ggatcgcacg 23940
taatcgcccc aagcgaattg tgcatattgg ggaccattgg gacatgccca gtctgtcaag 24000
ctacgaccgt ggtaccgcca agatcgaagg acgtcgagtc ctcagcgata tccaagcggg 24060
taatgatgcg atgcggctgc tgctcaatcc tcttcgccgc ctacagcgtc accaagcgag 24120
ttgtaagaag agagtctacc gaccagaaat gcacttcttc atcggtaacc acgaggagcg 24180
tatcaagcgg tatgaaaatt ctaaccctgc tctccaaggt tttattgggt acgatcattt 24240
tgatctgtcc gattggattg tccatgattt cctcgacgtg ggtgttatcg aaggtgtcgc 24300
cttcgcccac tacttctaca atcccaacag tggtcggcca tacggcggga gtgccgagca 24360
tcgcctcaat aagatcaagc gcagcttcgt ccaaggccac gaacagggat tcaagtacca 24420

catcgaggca gtaggcaaga agcgaatcca cgggcttgta gtcggtagct tctacaccca 24480
tgatgagtcc tacaaagggc cccagggtaa cgaccactgg cgaggtgtag ccctcctccg 24540
gaaccacaag gacggagagt atgacctcaa gctgatgagt gtggaggagt tcctgtgagt 24600
aagttcttgc cagacctgta ctacattaag tctgagcatg acttcggtca acgggggttg 24660
gcgtttaaga cgccgatctc cgcagaactc tggctggata tgaagtttgg gaaaggtggt 24720
gctgaggagg ggcttagacg agggatgtat tccatcgaag tcctggagat cctctacatc 24780
cccagcgttc accttccgga tatcttgggg taatctatga aagatcgagt gggacgtaag 24840
ctagaggtag gggacagtgt agtcttcctg gtccacagga acacctcctc ccatctagcc 24900
attggcaccg tcgatgggtt tacccccaag atgattcgga tcaaatgccc gaccatgagt 24960
tggactattg acgctgagta tgttctaaga agcagtgaca aggtggtgta ctatgacgaa 25020
agctgaacta gaggatcttc tgacagaaat ggagggtcaa ctggctaagt ctgaggcaaa 25080
gctagcgacc ttcaagaagg cagctctgga ggctaaagag cggttggaaa tgctggaggc 25140
tatgctggat ctggtggacc aacgatcctc tgtattctat ggagattgga ggggctatga 25200
agaacgatca aaaggttaaa ggcggtttcc cgtggacata catattcgta tcgatcctgt 25260
ttgctattct ggtctatgtg ggatacagtt gactgatgtt actcctgacg atgggagaac 25320
tcttaagtct attcttcgag atattgtcat gggtgggaag tttatgaaat atcgtgtaaa 25380
gcaagttgga aagtacttct tccctcagta taaaaaggcg ttcctttgga gaaactttga 25440
gagtgaacat aaggacccct tccgcccctc cctcagtgac ataaccctcc cggtaatctt 25500
cgaaacccta caggacgcca tgtgcttcat tcgggacttc gctgatacca agaatgagag 25560
tgtagtcaag tatcaccccg tagaagtctg aacctaacca aaaaaaggcc ccaagggtaa 25620
atcccaaggg gcctttaggt tagccccgaa gggcgttcag aagggtgtta aacttatcca 25680
caagggcctc gtggctatcc tgataggatg ctacggggat atccttgata cggtcttcga 25740
tagcagcttt aactactgca tccagaccta caacttcgga agccttaggt ttccaattcc 25800
caggcttagc ctcgttagcc ttggatccca cctccagaag ctcaggcttc ccctccacct 25860
cgtcccattg aacaggttta aggtctttgg gaccgatcac atgacgaaga gtaccgtctt 25920
cattccgaac ggcaatagcc ttggactgaa tcacgaatac gctttcacca tttgcatctt 25980
caatataagt tgccattaga ttacctcact tcccgaataa gaatagacat tgggtccatt 26040
agagaccact cttgaaagaa ccaacagtcc caccgaaggg gtatacccgg acagtgcatt 26100
caccgaactg agcatcggtt tcagtctggt tgcccttggc ataccaagcc ttgtctgggg 26160
agacagcgaa cttgatctta ccagcagccc cttgcacaaa gtggaataca caacctgggg 26220
ttacatcggg gcccaaagtc acggtgatat ccgtagctga catactcatg aagtgctgac 26280
cagagttctg tcgggtaagt gtgatgttac cgttcagttg tgcagtgttg accacattac 26340
cacccaggtg agtagtcaca aggcggggat ccaggtagtt ctgttggaga gaaccaatgt 26400
tcttcacagg accacgcatg tcatcaaact gctcgataat gtaatccccg atggtaccac 26460

cattttggtc atccgggcca atcacgtgct taatagccac gtaaacaccc aggtgttccc 26520
gtcgagggtc tactcggaat tcactaccga aggcagtacc acaaggggcc tcaaccatag 26580
tctcagagaa caaaccgatg acgtgacctg ggtctacgtt accaccagaa gtctcaccag 26640
gggcaccgat gagggtgttc acgaggacgc tggggactac cgcttccgag ggatggtcgg 26700
ggtaagaggc agttacgttc tctacaacca gggctgcgtt ctctttaacc gcgtaatagt 26760
ccgggaggaa ctctgtagca gttacaatgt tggtattcac tcgtccagcg tcagcagtaa 26820
tctcaatgag atccccatcc ttgttacgac ggaatacctg gggtacgtcc acgacgtagg 26880
ctcggccacc tgggccggct gcttcagttt caacgtaagt tgccatatac ttctccttat 26940
agatccagag cgtcttccat tgcttccata cctgcaacga aaggacgggc cggagtttgc 27000
ttatacaaga acttccaaat ctcaccagcg gaaggatcat cattgaagga tgcaatagtc 27060
ttaccagcag ccagagctcc ttcggtagct gcacctgcga cagggcccag gagtacctct 27120
gccggggtgg tacctctccg gtaagctgtt agcatatcgt agatcataga ggcctggagt 27180
ggcatctgtt gcatcaatac atccataatc ctttgctctt ctgatcgggt atcctcacga 27240
ctagagccac caaacttagc catctgacgg agttcatcct ggatgtaacc caggctcatc 27300
atcagaccaa gcgtgaaggc tacacccgca gcccccatcc cagcattggt ccaagaacca 27360
gtgaagtgag gggacaacct acggcgcatc atcgggagga taatgttacc ataagctgct 27420
ggataaccct tcagaagtcc gaacatctgg acgttaccat tactcatcca cataggcttg 27480
tcagcgaaag ttgggtcaag gaccgtctgg tctacgaacc tacggatagc tagggtctta 27540
acgttgttgg ccatcagaac ctccgaaggg gttgctgggg ataccaatct gagggcatcc 27600
gcttggctac tcacgttcac acccatttcc ctaagttgag caaccttaag ggcaccatta 27660
gctgagctaa agggaagccc agcggcgagg tccatgaggt tgttctgata gacccttttg 27720
gctgtctctg tggcgaatac tcggtcaaca tgggtcagga tggacagacc gttgaccaag 27780
aacatcccac gtacagcctt ctgagtgtac tggttgaata cctcagcacc aacccgatct 27840
gccatcaaag aggtagcaga ggccagtgtg tggttcatat cactcatgag tctcccagtc 27900
tcagatttag gaatgctgct gaatacacta cgagtagcct gacgggcaac ttcacccact 27960
gttggaagaa cagcacccag ggcagtcatg ggaccagcct tagcaaatgg gatactgaac 28020
tcggtcaggg tagagagacc agccaaagga agacgagaca agaccaatcc accagacact 28080
acagcagcca gcttcttgat attcggatct tttatacgac cgtgcatacc gttataggca 28140
tccactaggt catacatccg gtctacttct tccttagtta ctcgcttacc attttgttga 28200
gcctctgcta ctgccgaagc aatcatagca ttggccttct ccccgttgat accaaaccgc 28260
tcggtaaagg caatccggtg ggaagatccc tcaaagtaat cccggatctc ttggatgagg 28320
tctttagggg attcattgag ggtgtattta ttaaggatct cctgaggtac agatccaaag 28380
gaacgacttt cctccagttg accatactta ggtactgcat cactctgggc aaaccgtccc 28440
cgaagggtat caggatctcc ctggatacga taccgtggat ctacctccca agcacctgta 28500

gtctgattct gagtgactag gcgggtaact tctggggcag tattacctcg ggtatcatcc 28560
gaagtctctg ccagccaatt agctacagcg tcctcagcag cctgacggtc ttggaaataa 28620
ggagtaatat catctaggaa ctcaggggat tctaccttct tacgggacag tccaaagggc 28680
atatagttga cgatattccc aacggacata ccacctcgac cactagcctc agccctaaca 28740
tcattcaaga tatctcggag ggcggtagcc tcaggagtat tgacgcccat agaggcatca 28800
ttgatgatct ggcggatctc cttattggac ttgccttcga tgatgttatc cagggcagtg 28860
ttccacttac cagcctggag ttcctgatcc tcgaagatgg tcttaccaga agccctctca 28920
ccactcatgt cagccctaaa ggtctcagag aactcacggg ctatcgggga ggccttagca 28980
agtggctcca ggagggccgt agcttcatta cccatggcat cccaggcctt cctgatagta 29040
cccctaggct ccatttcgga agccgtagga gcagccgaag gggcgttagg atcggtatgg 29100
gctgagcctg ctgaatcctt gttgctggcc atagtgttaa gaccagagga tacagcaccc 29160
ccacctacac ccatggcaga tccagcgaat gcagccgtca gtaggttatc catgaactct 29220
tctggacgtt gtacttgtcc tactgcatca taagcaatgg tgtcctgtag ggcctgttgg 29280
gcaccagagg taacaccctc agccacacca gagacagccg catgcttacc agcctgagta 29340
acagcctcaa tggcagtctg cttaggtagg cccgattgga ccaacatttg gtaagcgcca 29400
tctttaccga tgtgtttgag gagtggggca gcgataacac cagcacccgc agtatctagt 29460
accgagaggc cagcaccacc taggacagca gtccaggggt tactaccctc ggggtcaagc 29520
tccttcatct ggttgttcag agcacccacg ttgatcccca tggagctcag gaaagaacct 29580
atgagggcac cacccatacg accaggggca ccaaacatgg atccagcctt agcacctgcg 29640
gcaccaccag ccattacggc tcccattgag gggagggctt ctgagatatt attctttagg 29700
aaagaaccaa tggagggaat atcttggata tcggcaaaag accgaacatc tggggttccg 29760
tactgtgacg cctcctgggc attctcctca gccatctgag tgccgtaatc tttgaggtag 29820
tcattaccag tcagctcacc aacagtagca atagtgccac cgatgttaga ctgcatggta 29880
tcgatcccac gattgatcgc agaaccaata gaattaggat cagccggagc agccaaagcg 29940
ctcaggtctg gggcagcttc aggtacaacc tccggggtta cttcctgggg agctacctct 30000
acttcttctg gctcattcaa cgaagccaac tcagcggcta catcgaggtc ttttacctca 30060
gccagttccg cagcaatagc ggctttcatt tcaggggaaa gagccataag tcaccttttt 30120
gctggaatag aagataagca ccttaagact atcttaagat aacctaaaga tttaattaat 30180
aatacaataa gttattaaga taatcttaag tgcctataga tctattatac cacagtttta 30240
ataccttgtc aagtaccttt tatcacttag gggcaggacg actcaaaata cctgaccccc 30300
agccaaaaag acccgacggt tgcttctgta gcttagccgc ttcctgttgt acgaaggtaa 30360
cggggtcagc cccaggattc tgcctcaggt gattccgaag cttctgggag atagccgact 30420
ggtcttgttc accaatctta gcaccaccta ccagatccga accagagaca cccttgacca 30480
actcattggc atccttggta gtgatagcct caccacggga agccgaagca gcagccttac 30540

gtgcagcagc actgatcttg gcagtcttga gacgtaccgc agaactaagc tgagcattct 30600
cttgagacat atcctgtcca cgtctagcgg tttcagcctg gagctgagcc cgttgggtag 30660
ctaggtcttg accacgctgg gttagggcaa gcttggcccg ttctacagca gcctgttttt 30720
cccatttaga cagaagaccc tcattcttcc gagaggtttc ttccagagtg ccccgcttaa 30780
cctctaggtt accaatcccc agacgctcat cagacttatc cttagcaacc ttgcgttcca 30840
gatccttctc cttgatctgg cgatcagcag cagcagcagc catcttctgt tggttgatgc 30900
cagcctggag gttacgatca agctgacgtt cataggaggc agcgaacatg tccccagcct 30960
taccagtctt gtctagggcc gaagccagaa gaccagtacc aatgagagcg taagatacat 31020
aacgagacag gtcatcatta tccatagtcc tcatctgggt gagctcttca ttaacccgag 31080
ttttaagctc ttcgggttta agctctacac cttcccgttg ggcatcagcc tcgactacag 31140
cctgggccat ttcagggcgg cttacggctc cactacggag accttcgata gcaccctgtt 31200
ggatcacctg acggttagcc tcttcttcct ctgctaccgc tgcaccggcc tcagaggcaa 31260
cctcaggggc tatctcaggt tcgatagagg gttggttagg ggtaacacca taagacaaca 31320
gaccggctcc ctgggggcct cctgtggcct tctgagctgc cctctggcct acaccctggg 31380
cccattggtt agcactctgg gcaacctcag gacccatatt ctctactgcc tgggcagcct 31440
gagccatttc ctcagcacga gtacgttctt ggtcaccgat atcagcagga gttacagcag 31500
cctgaacacc tacaccaaca ggaccaccaa ggatacctgc gatgcgaccc aaggcaccac 31560
gaccagtagc ttcagccgca ggtgcaacct cttgggctgc acgggaccaa cgctctgggt 31620
tctccagggc tgcctgagca gcacgacgga cacgatcagg aatacctgag ccctctacag 31680
ccagcggcag tttgttcctt gcgattcgcg ccatatccag ttcattctga gcagcccgaa 31740
ggatgctagg gcgaattggg gacccttcat aagccatgct ggccatagga ttaacctcca 31800
aagagtttac ttaggattcc tgactttggg agttgtccaa agccgaggtc ttctgcgcta 31860
ggaagaatgc ccattcccgc tcccatacct gctgcaacat ttgctgcgcc ttgctcgctt 31920
gctcccctag ccccttcgac agaaaggctt ggtactgttt ctggagtaga gtcacttcct 31980
ttcccgagga gagccgcgag tgctgctccg cccattgcgc caaaagggtt tgattgtcca 32040
ctactagatt cctcacttag tcccgacccc aggtggacag ggcggtagga gccttgacga 32100
ctcaactgta cccgctcctg cccaccaccg atgttctgtc caatggtgaa cagcttctga 32160
gttaaagagg ccattagaac aacagacccc caactcgacc acctgcgttc atacccacag 32220
aagctcctgc ggggcctccg aagagtgctc ccagggaggc accacccaga gcacccaggg 32280
cagaaccgag gccaccacca ccgcccccag agcccttggt gacagatacg ttagtaccac 32340
ccatgtcacc cgaaatcagt tccttatagg cgagaaggtc gttaaggcta acgttgttct 32400
cataggccca cttctggaga gcaccattga tctcctgttg ctcctgattc tgtagcatag 32460
aaccagcgtc aacctgcatg gcattaccag agcccagacc tttagcgata gccgaaaggt 32520
tacccaaggt attcagacgg ttctggttgt aagcctgctg atcttggaag gccagttgag 32580

aggcgttatt ctgttgattc tggagcagcc tagcggtagc aataccttcg gctacacccg 32640
ctcgggaact gccatactgg ccagcattgg tcgctcctgc acgcaggtct gggcgtaccg 32700
tagtgtcgaa gtcccattgc atctgctcgt tggctgcacc aatggcgttc gccaagccag 32760
ttttattggg atcgtaagga ccaaggtaat cagccagaga gctaacacct gagctaccca 32820
ggagagactg gagggctccc ccgagattac ccaacccctc gatgccaccc aattggagag 32880
cattttggtc agccactgga tcaaagttcg gatcaccacc gtagttcggg tcaaagcccc 32940
cattatgtag ccaagaactg gcacccgaga gtagttcatt gtaattgccc tgctgatatg 33000
gggtagaggt ggaggtagtg gtttgcttct tactaccccc cttataagcc cgactatcaa 33060
gggcatcctc tacgttgaac cccatcagcc gcttaacgtt aaattggagg aagttcatct 33120
ggagttacct ctctatagaa ggttacggag tcctcagtgt acccaagctt ttcaagggta 33180
ggcttccagc cccgacgacc ttcacattgg ataaaccgac agcccatctt ctgggcaaac 33240
tgtcccaggt actcatcaac ctcggggtag tctatcggag tttcattccc aggcatctta 33300
ccactccaga agaagtgtag gatgttgccc aaaggggctt gggatacttg caacacccca 33360
gcatacccac tctcttcctg gtagaagaca taggcctcat aattaaccag ggaatgcacc 33420
aagtgctcaa agtcccagaa cttacccaag tcagtcctgt taaaagcacg ggctagggct 33480
ggaactacgg tcgggaggga gtcaatattc tcacgagtaa tcaaatgaat catggagtca 33540
ccactagggg ataagtaaac gtacccccga agatagcacc ttggggaatt ccttggatta 33600
gaacacggcc attagctgtg atcttaaaca ttgcttggtt aaccacacca gtctgaatag 33660
tagccctgtt gagaacatca aagacttgat ccaacggagg tgcgttaggg tcagacggag 33720
gtggataagt aatagtgctc tgcgctggga tgatgctgga gtaagccggg ataaacaact 33780
cagcaggagg ccaatacgcc tggggaagat ccaggacagt agctccatta gtgtagttac 33840
caccagacat gagcatggtt atccacactt catccttagc agtgttcatc ctataggcac 33900
aggtacccat cggctgatgg ttgttctgtg gggtaaatag gataaaatcc cccggcaggt 33960
ccttgggttt agtacctgcg agtctccatt ggttatccaa gtcgtaatga tagatgccgg 34020
atactggacc taccactccg ggggcaaaat actttacagt ccctggcttg agcttcttag 34080
ggggctccat agagaccccc cagtagccgt cagctaggtc attaagagtc tgtccaaccc 34140
taacgaattc ttcattaagg aagggcagca gttcctcctc ttcctgtggt ggaatcgaag 34200
ggctgtactt ttgactcatc gcatacctgc cttcggggcc atttcaatag tgtatccatt 34260
gaagtaccaa tcaccctccg aagagaagtc gaacttcaag gctatatacc ggcccacatg 34320
tttagtgtca atcttatagt cctgaccaat ccggtatggg taagggcctt tccatcgaat 34380
accagaacct tgtacctgag cgttacccac ccagatgtta caagtaccgt taccagtaat 34440
gtgtggaatg atggcactca ctgtcttcat cattcgatca tccccgagat agatatcgga 34500
tctctcaagg gtactgacga agttctgccc agagaatgta gagttattac cgaagaggaa 34560
caacttctta tcctggaaag acgagaagat catactggac tttgttgggt tataagaacc 34620

ttcaccccag accgaagtat cggtatccca ggggttgggg tcgtcatccc agaggttaga 34680
caccttagga tcgatgatcc cgtaggctcc actgagaacg ttgggaaggt ctcggatact 34740
ccaagtattt tccttccagt tccagatgat agccctgtcg cagtgcttac ctggctcaga 34800
cctagtggaa gagtagcata cccacatttc agtattcacg tggtctgcaa gtacgaatgt 34860
ccgttgatag ttgtcagggt taatatccga gaagaagaac ttacggacct gggcatcaat 34920
aacagactgc ttctgcacac cgttgtggac atatacatca ccgtgaccta ctacaaagtg 34980
gttaccatcg aactctactg cacagttggg cccgaggata cctacgtcgt taaacaactg 35040
ctggaactgg aagatgaaca atccaccgat ataccgcatg gagtatacag agtcttcctt 35100
gtagatgatg aaggagtcac gaagcttcac accatccacg atagcaccat tggtatcagc 35160
caaggtgttc tgaccagcat ccttagtagg gtccgttggg tcccaagaag cgggtacacc 35220
accagcatca gccgaggtac tccaccagac catctgtggc atttctacag agttacttgt 35280
agcgtttaag ccaaccagga agttcttaaa agacttaagc ctcttaaaag tagtattcgc 35340
tgggaagtta gggagtaccc taaaggttga ttctgacggt ggaagatgat gaggagggtt 35400
aaccccatcg ttagcaaaga ttaccccgtt gaacgatcct acagaccacc tgttagttat 35460
actagcagag taaggtcctg ggatacatcc gatgattgta gtcccgtcgg ctagatacaa 35520
ccgttgttca gaacacagga gccaataggg gatgttattc cggatgaaag gaaacatatc 35580
caagattggg gcctgggctg tatcaaagat aggcgtatgg cccagagtct tctgagcctt 35640
gccgttctta aaccggacgt tgttcccgaa ggaccatttc tccagtggca ggtcagcggg 35700
ggcgatatcg gtcacaatcc ccgtagggtt cttgacctct tgtctctcta gggccattgt 35760
atacctcagt tcttaatgat gaagaacaca gaacagaacg gtgggatatt acccaacggc 35820
atgttgatct ttactgcatg gttgtgagtc tgaccttgac cagcagggcc agtctcaagg 35880
ttggtattgg ctgcgttacc agagccaccc gtcagagcac cagagtcacc cgcagaaccg 35940
gtgagggtag tagcccctcg ggatctccaa gtgtgggtgt gcgatgggat ttgggctagg 36000
gtaagggcag tcccttcagt gaacccatcc catacgatgt tagcgctacc acctctagtc 36060
cctacagcct gggaagtacc atcgataccc caggggaatg ccccaatgag gttaggaaca 36120
gggataccat tagaggtggt acccacgcca ttacagagct tccaacccgc tgggatctga 36180
gctagagacc cagcccacat gataaccatc ccaggtttaa catactgggt agtatcagcg 36240
actgcgttta gctgggctgc cgttacagtg acagcctgag aaatattggg gaaggtattc 36300
ttaatagcac tcttaatgag tcgcagatgg tcatccccaa aggacttcag atcagagccg 36360
gtagggttcg taggcaccaa ctggttaata taagttgcga cctcaagacc cattcttggc 36420
ctcctttatc tcttctttca tttcttttcg ggtgacatac cgctccccaa agattgccat 36480
agaaatctgg agatcgctca ccgcttgggt gagcttctcc gtggcctgga tgtttctttc 36540
aagcagagcc tgatgaacat tctgaccgac aaccgaggaa cctaccgtca ctaccgagga 36600
tacgaccagg gcactgacga tgctgcccag gttatcagtt agaagttgca tcctccgact 36660

tctcctttag attgtcctcc aggactttaa caaactcgtc atcaactttg gagttagtct 36720
tctcagccag ggctttagca ccagtcacga tggacttagc gattaccttg gttgggaaga 36780
gggttgccag gagattgatt gcgagggttt tgagaaagat aggcattgga tgctccttaa 36840
cgttcgatat ccttgatcgc cagtcgagag ctagcgaagt cagcggcatt ctcttcattc 36900
tggagttcca tgactgcccg ttctagtttc tgtccccaga acatcgaccg ctcttcatcc 36960
atggtgtaca gatatatctg ctcaaggaca ccatagaggt aaatctgcgg gtacttagtc 37020
agggcccaag ttgttgggtt agccaagctc agctctggga gaacagtcca gtagttcaca 37080
atgaacgggg caccgtcagg aacaacaggg tatactcgcc agaaattccc caatcgagtg 37140
tagtaagtta caccctgggg ctggtagttg tagttaacat agtgggagaa gatatcctgg 37200
gtgatgtact ggagggtacg tccaccgatc aaggcatccc ctgtgataga tcgaagggca 37260
acaaagtgct cagggatctc gatgcctcca ccaaaggcca tcagggtttc aaagtgttca 37320
ttctcacgta cccgcaggag acggttgaga cggtcagtag tgttgccaat gaacaacatc 37380
agaagttctt gggtaaggtc ctgacggtca gaccactgga ttgcggcaat agcgagatca 37440
gttacgttat taatcgtagc cattcttata cccgtgcctc agaggtccgc atacgatagt 37500
tgtctcggtc gttaagccaa cgggtaaatc gagcggcatg gtcagggtcg caaccaatca 37560
ggttaagatc gatggggcca ccctcagaca ttggacgatt ccgtagtgcc tctacaacaa 37620
ccagcgggat actagcaacc tttcgaaggt tatccttacg gttagtgttt acacccgaat 37680
gacgctcttc ggcgttagcg gacaggattg actcaacatc ttgagtatcc tttcggataa 37740
agagcccaag gtcttcgtca attgcataag tcgattggat actcatgatg atctcctata 37800
ggaaaacttg gggccccgaa gggcccctct agattacaac tgggctacga cgtcgcggat 37860
cagagcaccg gacttctcgt tgtttacacg gaaggtgtat tcaaccagga gttggcgctt 37920
ctcgctgtca ccggtcttag ccagctcatg ctggaagaac ggacgcaggt agcagaggct 37980
gtgcatcttc ggatcgaaga cgaacagggt gttctcgtgg aaccaacggt tagcacggat 38040
ggtgtactta ccgaagtcac tctcgtagac gtctacggtc tgcgcgatgc ggttatccga 38100
agcgtccagg gtgatctcag tagcacgacc cttcatgttc ttgctgatgg ctttcttgat 38160
cgagctcgaa gtctggatcg agttagcctg accaccgtta cgccagatcg actccgaggc 38220
gttcaggagc atatcttcgg tcagcagtcg cagatcccca gcggtaccag tgttcgaacc 38280
gtcacctaca ggagcaacac cgttggcacc cagggagccg ttggtcttgt agtaggcgaa 38340
gatgttagcc atctgacccg gagtagtggt gttacgctgg accttagcct gaggggcacc 38400
gaccagggcg tattccatgt ccagcttcag ttctttggac ttcttagcca gttggtaagc 38460
cagttcgttc ttacggccag ccttcttaac gcgatccgca gtaccggtga cttgcagggt 38520
ctcgtcagag atttggcagt agttgttcag catggtggtg aagctaccag ccttgatggt 38580
tgcatcctca ccttcgactc gggtgttctt acccggctga cggagctcgt cagtctgcca 38640
ttcgtgggtg atagcagtag ctacaccttt gccgatagcc gacataaacg gggtgtcgta 38700

gggagcgatg ttgtagatga tatcgataag gtcttcgcgc ttaccgttga tctctacagt 38760
ggagacggca ttagttggag ttgccatata atgtacttcc ttctattaga aaatgtcgag 38820
agaggagaag agagcagcag cggcttcaac cgactggtct ttcctcagat tagcacgggc 38880
agctttaacc cgcttagaac cctctgaggc ttccgcccta cgagctacag gcttaacagc 38940
agcaggtagt tcagtctcct ccttcttctc tagggcagcc ttgcgacgga cctgagactc 39000
agcccactta cgtgctgcat caagtacagc cagttggcgg gcatcagcaa taccccggat 39060
ttcatcctcg gagtagccaa ttgatttgcc gtaagctacg atcttatctc cccaggtctc 39120
atcagtagtc atttcaggga tcagcttctt agccaattct gtctgacgtt tgatgtagga 39180
gctgtggatt atctcggctc gcttctcctg catagccttg atatcgttac gacgtttaat 39240
aagtgcctgg gcttgatccc gagcctcaag agcctctagg cgaagcttct gatacttctc 39300
cgggtcttga gccttaagtt gctcccagtt tatgctatca tactgattag caccagcaat 39360
agcggtaaca gtgtactgct caagctcggc cagtagatta gcacgttcag catcaactga 39420
ctcaagctta gctgcatact cttcctctaa ctcagcctga cgggttacaa actcttcatt 39480
acgaaggtaa ccactcttga gttcttcgaa gttgacctca tagatatcat ccccgatcgg 39540
aatctcaaag agtttctctg agaggtcatc ctcagcatca acctcaggtt catcttcgga 39600
ttcaccttca ggatcttcct cagactcttc ctcaggctca tcctcagatt caccctcggg 39660
ggtttccttt tcaacctctt cctcttcttc ctcacctacg actttaccat ctacggtttc 39720
atcaccgggg gccaggaggt catcaccgag caactcacca aaggcttccg ctgcctcgaa 39780
ttcatccatg ccttgattct caaggtccat tacttatact cctttagggt gatcgaatcc 39840
aagattgcct ggatacgaag ttgtacgcgg ttgagggcgt ggagctcgtg gtatatcgcc 39900
tcccgagtct cactatcttt gggggccgta gccttccatt cgccctcaat ctcctcttgg 39960
acaatacgga aaagctcggg aagaacgttc tcacgtacca tctgctgtgc tgcatcagtc 40020
agcactagac tatagccaaa acgttctccc aaggttattt cctcactgcc ttagcaggct 40080
tcttagtctc aggtacttta ccgtctccga tataagcagc ccgagcctga gtagcctcaa 40140
gatgatactc cgcttcgtta cgagcccgtt cccaagtgaa acgatcacgc tcaagttgta 40200
gctccgcttc cttaagggcc atttctctct gctgaaggac agcttcttgc ttcttcagtt 40260
cgatctcagc cagtcggatc tgagcctcta cctgcttcat ctgggcctcc gcttgcttag 40320
ccaaggcgtc cgattgggca cgttgggcat cagcttgggc tttgatatct tcaggtttag 40380
gttgggcttc cttctgctct cgaattgcct tggcttgtag ggcctcggga gagttagggt 40440
tagtccagaa gcgatccggg tctttgtacc cagcattctc tgtgacttcc ttaaggatgt 40500
tgtaaaggtt ctgctcagag acaaggaccc cgagaccacc acccccgact acagcctggg 40560
ccatttccca gatacgcatc aggtggagca tctgctggtc tttgttcatg ttgccaatac 40620
caacggtaac cgtcagatcg gatctctctc gccagttggc agggttaacg gaaacccact 40680
tgcctcgtag ctggaagacc tcttcctgat tctgatactt aatggcatgg tcatgcagaa 40740

gttggaacaa acgcttaaca ccagtctctg caaacatccg ggcaatcagg tcaatctgtt 40800
gctcagccgc agtcatcaac tggttaacac tcatagccgc ttggttagag tgcagggtgt 40860
tctgatctag tccgcgagta cgatcagtaa taccagtacg cttaccccgg tctgcctcaa 40920
ggcggtcaag catcccatag acttccccgg acaactgagg agtctccaga ggggtgatgc 40980
tattcatgga tttaactcgg acgataccag ccgcctcatt ggtcagcaag tcttccaagt 41040
taacctgacc atccaagact acagaacgac cttggttggt acggtagatg ttatccatga 41100
tgttccgcat gagtaccgaa cggatctctt gaatgtcacg gatcttatcg tagacactca 41160
tcccgtggaa cttatgggca atacggtagg cattcaggtc agcgaaggga cggcaatccc 41220
aaggctcgtt actgataatg taatcaccta catagaggat acgacggagt tcagagatac 41280
catcaccatc tacgtcaagt agagtgtagc actcagaggc ccatacctca cggttagcct 41340
cagcgtcatc cccagagtta tactggagtt gacccgtcat atcaaaatta tcccggacta 41400
ggcgctcagg ttggctatca gagaactcat actcatcata tggaagttct tcgataacat 41460
cctcgggaac ccccaagagt cggagatcac ttacggtgta cttctcacgg tgacagagga 41520
atcgggcatc atcgatacag gtcgctagtc tatccaccag gaagttctct ggcttaacac 41580
acaggacttt gatttccctt ttcttcttat ccttacgaat cttaatagtg taggtgccat 41640
catcatctac actttgggcc agtatagagg tatctgggtc agacagaatg tccgctacca 41700
tatcctcaga gaggcctgag aatcgttcga aggtgggctt tagaacctct tccacgtaga 41760
ccttaacaac cccagtcttc atcatgaggg tgtcttggaa ccagtcgaac attaccttga 41820
acccctcatt cttacgcatg aagaggtagt tcacatactc agtctcttgc tctgcctgct 41880
caacatcctc ggcagtatca ggctcatatt taactacttg accaccagac gtgaacacct 41940
tcataagaga aggcataatc cagtctacgg tctcttggac gtccctagat acaatcgcgg 42000
acttacccgg acgctcatta ccgaagggct caccgaagta atacttcagg gcctcagagc 42060
gttgcttaga tagttccgac gagttgaaat caagggcgtc gtttacaagt tggtctagat 42120
gacgaagtac ctgttcatca tccataggct tgatcttacg acgcttagcc atttagacaa 42180
tactcccaaa ccaatcaggg atcaaccttg cggtatcact cctgtagtat ccactgtttc 42240
gtacagcacc gggacgagcg tgacgggaag ccatcagaag ggcataccga gttgctgaga 42300
tcatgtcatc attacgatcc acaatcttac catctttacg gtggtacatc ttcatttctt 42360
tgaggaagtt agtacaagta ttaaatacct tgagatcccc attctccata cgagtaagca 42420
tccagttgac accaaactct acagagttac ccccgtgttt accatcggga cctggggggt 42480
tgctgaaggg ttcataaaca acattgaggt tgtggtcatc cttcaggagg tctacgaagc 42540
gacgacctga ggtagctcca tcatgcttaa acgcatcgtg ggggactaca acagggatct 42600
ggtgaccacc cttgaggtag atagcatcag cgtgcatgcc gagggtttcg ccagattcac 42660
ttcgttcatc atacaggtaa tacttgtcct tctctgcatc ccaggctaca caggcgatag 42720
cgttagggtg gtcaaaccct aggtcgatac caatgatcct atggaagtga tccgggatat 42780

caaaaggctc acatacgaac ttctcttcca gaatagggaa gactacacca gagccgagca 42840
taggaatacc ctcagccctc atcctgcgtt ctgctggaga gtataccgag agtagctgct 42900
ctttaacttc tggactgagg tgtggggcat cttcccagct tgcatggatc aggaactgac 42960
caggtttaag atcctggagg aagtccttca cgatttccgt gagaccatgc tctggggtaa 43020
aggttagata gacaataccc ccagtagtag ccgttcgggt tacacactgg gtataaatat 43080
ccttggggca ctcctcgtca agccagatga cgtcaatagc agtacccatg aatttgtctt 43140
gggacatttc gtaggacttg aagattagcg aggacaaacc cccatatgcg tgccttacca 43200
ttacagcttg gacgcatcca ggtttgcctt ccctacgttc agtctttacg atatcctctt 43260
tggggatcat cccagtgcca aatgcctcgg ggttcttcca atcacctaat agttcagact 43320
ggagaatgtc ccgagtagta tctgtggaga tccctgcggc ccagcagttc actggcttgt 43380
caaactttct tccagtccac cactcagggt aacgaccggt aagatgacag gccatgatga 43440
aggccccggt gtatgtctta ccacagcggt tgccagtcat agccagtagc tgagcacaat 43500
tagaagaggc tgcaatgaac ttctcttgcc acccataggg ggtgtactgg ttcatgcgga 43560
agtacttctg ccgctcagcc aattctctga ctaggttacg caaccgctct tgggtatcca 43620
ttaagtcacc gtgctaccta tcgtagatgc tcgccaaagg gctcgttgga ggtcccttac 43680
gcgaactgga gtttgtttag cccaggcact atcctcggct tcccaagctg ctctgtcgaa 43740
ctccccggct ttcagaaggc tccacgtctt ggggaacttc ttagtccatg ctgtaccaag 43800
ctggaagtta acgctcacta gggcatcgaa tagttctgga gtgcaaaaag gcagctcaga 43860
cacttgccgt tgggctgcat cgtaggctgc ctgactgtca ttctctagcc aagtctctgc 43920
tcgggctagg gtgatgggtc catcctctcc gggacgctgt aggtggccgt agcctccggt 43980
ccaatatccc aaggagtctt tgtaccactg gagtactaac ccctctcgct tcttaataag 44040
actcaggaag tcaagcggtc gggaaagaaa ggactttagc caagtctggc tcttcgtcga 44100
gtattcgtcg aacttcattc atcagatcct ctgcggacat atcttccaca ttcttagtgg 44160
taagctccac tttctgctta gcaccgaagc cacctcggtc caggatatcc tgggctgcct 44220
tgaggcggat accacctttc tcatttggat cttctgcaat agagacgatc acacggagcg 44280
ccatggggac atgactcccg atgcgctcag agataaaggc attgatgtac tcggcatgtt 44340
tccgatggta agctgcaacg ttgcgttgag catgattggg ggagaaccca gcggctacat 44400
aggcttgggt tttgtttaat ccatcggcta gtccttcgca ataaaggtct agtttttctt 44460
tggcagaaag aggagcggca ccaatcgaga caaccttttc gtctgacata cgcacgctcc 44520
tatctctcta atgtcacccc ggtaggattc gaacctacga cctcctgctt ccaaggcagg 44580
cactctaacc agactgagct acgaggagtt gttggcgacc cattggggat tcgaacccca 44640
gacttctccc tagacagggg agcactctta ccgctgagtt aatgggccat aatggctggg 44700
atggtagggc tcgaacctac gaccggatag ttaacagcta tctgctctac caactgagct 44760
acatcccaag gaaatcttaa gcacccttaa ggttaatttc tcaaccttat aatactatta 44820

tatcacactt ttgggttttt gtcaacccct aatttcatct tttttattat tttttgctgg 44880

aaagtggaat ttcttaaggt tcttaagact atcttaagat aatctataga ttaagtattt 44940

aataagtaat aagtaaatta agataaatct taagtgccta tagatctatt ataccacaaa 45000

ttgaataccc tgtcaagtac tttttgatat ttcttaagaa ttccccctga ttgcccgacg 45060

aacggtatgg atagggttct ataggtatac cctaagacta cccttcttat gttactggta 45120

atggggttat aaggtctatt gatccgggtt ctataggtat accctaagac tacccctctt 45180

gtcgctatac cctgggtcta gttcttctta tcccagcaat ataccacaag gtatagtcta 45240

cggggtgcct ttgatccggg gtatagcacc tagagtaccc caaaagtcta ttttatcctc 45300

cggtgtatct gtggaatatc cccccagagt cccccaggct aacgttgggg tggccctctc 45360

ccaggggtac tagcccctag ggctccccag gataccataa gactagtccg ggggtcaacc 45420

ccttgagata ccgttcatcg g 45441

<210> 6
<211> 66049
<212> DNA
<213> Pseudomonas aeruginosa phage

<300>
<308> NCIMB 41730
<309> 2010-06-24
<313> (1)..(66049)

<400> 6
ggaagagttt ttcagctgga gtctcaaggc tcctggtttt atcaggacgg ctggggagtt 60

tgcctggccg tggatatcgg tgcggccaag ttcaccgatg acgggaaacc gactttctag 120

gtggtgtatg tttgacggcg aactgatagc gaaaatggtt gtcgagctga atgcggcgat 180

ggcatctgct caagaggctt tgcagttccc ggattttgaa gtcgtccaga aagctcagcc 240

gacccaacag ggggcgtcaa ccaggccgac catctttttc cagaaactgt ttgacgttcc 300

tcgcggctgg cctgccaccg attggtacct ggacaacacg gcccgcaaat atgtagaaat 360

aactcgacag catgtagaga cgactttcca gatcagttcc cttcattggc agaatcctga 420

aataactcac gtggttacgg cttctgatat cgccaactat gtgagggctt atttccaagc 480

tcgatccacg attgagcgcg taaaggaact ggacttcctc attcttcgcg tgtctcaaat 540

ttccaacgaa gcattcgaga acgacaatca ccagttcgaa ttccacccaa gttttgacat 600

gattgtaact tacaaccaat atattcgcct gtacgaaaac gcagcatatt cggccgatgg 660

ggtattaata ggcatatgag tctgaggcgc gattcagagc taatcgccgc gcacctccag 720

atgttaagag ccatgcgcgg caggtccgtt tcagccggat ggtattccac ggctcgatat 780

cctgacaagg caggcggatc ggtcggaata caagtcgcga gaatcgcacg tctcaatgag 840

tacggcggaa ctatcgacca tccgggcggg accaggtata ttagggacgc cattgttcgg 900

ggccggtttg ttggcgttcg gttcgtcaga aacgactttc cgggagaaac cgaggtaact 960

aagcctcaca gaattaccat cccggctaga ccgtttatgc gatatgcttg gaacttgttt 1020

tccgcagatc gcgccgcaat ccagaaccgg atagccatga ggctggccag aggacagatc 1080
actccagatc aagctcttgc ccagatcggc ctggcattgg aaggatacat agccagaagc 1140
atcaggaccg ggccatgggt ggctaactca gcatctacgg tcaggagaaa gggattcaac 1200
agaccgctgg tcgatacggc gcacatgctt caatcgatta gcagcagagt aacataacca 1260
ggagatcatc cagtgatcag tcagagccgt tatatccgga tcatttcggg cgtaggcgca 1320
ggcgctccgg tcgcaggccg aaagctgatt ctgcgcgtca tgactaccaa caacgtcatc 1380
ccgcccggaa tcgtcatcga gttcgacaac gccaacgcag tcctgtcata cttcggcgcg 1440
cagtcggaag agtatcagcg ggctgcggct tatttcaaat tcatcagtaa aagcgtgaat 1500
tcgccgtcca gcatcagctt cgctcgctgg gtaaacaccg ccatcgcgcc gatggttgtt 1560
ggtgacaatc tgccgaagac catcgccgat ttcgccggct tctcagcagg ggttctgacc 1620
atcatggtcg gcgcggccga acagaacatc accgccatcg atacgtccgc cgcgacttct 1680
atggacaacg tggcgtcgat catccagacc gaaatccgca agaacgccga cccgcagctg 1740
gcccaggcta ccgttacctg gaatcagaac accaaccagt tcaccttggt cggcgccacc 1800
atcggcaccg gcgtcctggc tgtggcgaaa tctgccgatc cccaggacat gtccactgcc 1860
ctcggctggt ccacctccaa cgtcgtcaac gtcgccggcc aggctgccga tcttcccgac 1920
gcggccgttg ccaagagcac caatgtcagc aacaacttcg gttcgttcct gttcgccggt 1980
gcgccgctcg acaatgacca gatcaaggcc gtgtcggcct ggaacgcggc tcagaacaac 2040
cagttcatct acacggtcgc aacttccctg gcgaacctcg gcactctttt caccttggtg 2100
aatggcaacg ccgggaccgc cctgaacgtg ctgtcggcga ctgctgccaa cgacttcgtg 2160
gagcagtgcc ccagcgagat tctggccgcc accaactacg atgagccggg tgcttcgcaa 2220
aactacatgt actaccaatt ccctggccgc aacatcaccg tttccgacga taccgttgcg 2280
aacaccgtcg acaagagccg gggcaactac atcggcgtca cccaggccaa tggccagcaa 2340
ctcgcgttct accagcgcgg cattctgtgc ggcggtccga ccgatgctgt ggacatgaac 2400
gtctatgcca acgaaatctg gctgaagtcg gctatcgctc aagcgctcct ggacttgttc 2460
ctgaacgtca atgcggttcc ggcgagcagc actggcgagg cgatgaccct ggcggtgctg 2520
cagccggttc tggacaaggc gaccgccaac ggcacgttca cctacggcaa ggaaatcagc 2580
gccgtccagc agcagtacat cacccaagtc accggtgatc gccgcgcctg gcgtcaagtc 2640
caaaccctgg gttactggat caacatcacc ttctccagct ataccaacag caacactggc 2700
ttgaccgagt ggaaggccaa ctacacgctg atctattcga agggcgatgc catccgcttc 2760
gtcgaagggt cggatgtgat gatctaatgg tttgcggcgg actcgatccg ccgcgacctt 2820
ccataaatgg agtgaggaat aaacaatgat caacatttct gcgttcggct cgatctgcca 2880
gttcacggca agcagaactt tcccgaacgg attcaccgtc accgagtttg ccgacgatgc 2940
agaccccatc gacagcccgc cgttcactgc ggccgatacc ggcgtcggcc tcaatggtga 3000
catggtagtc tggaaccggg cgaacatcct ggaagtcgtc gtcaacgtca tcccgaacac 3060

cgagggcgag cgcaacctgg cagtcctgct ggatgccaac cgcaccggga aagacaagtc 3120
gggcgctcgt gatgtcgtcg gtttggtcgt ggcgatgccg gacggcagca aaatcacctg 3180
caccaacggc acccccatcg acggcgttct gatcaacgcg gtggcaagcg tcggccgtct 3240
gaagaccaag ccgtatcggt tccgattcga aaaagtgatc aaagccggta ctagctgatg 3300
aagaaaattc cgctgacagc agtccccaac caggcgatct cattcaacgc cggtagcagc 3360
tattggaaga ttcgcctgta ccagaacatg gacatgatga atgccgatat cagccgcgac 3420
ggcgtgatcg tttgccatgg ggtccgctgc ttcggcggga ttcctcttct ccaatacagc 3480
catcagtacc gacctgacta tggaaatttc gtcttcgacc gcgacgccga ttggacattg 3540
ttcggcgacg gcatcaacct gttctatctg gacggcgccg aattcgccga gtatcaggcg 3600
cttgccacga ggaaagaatg agcacatcaa cgatcagaac cgggacgaac aacgatatcc 3660
ttttggacga caatggaaac atggttatcc tcagggatgt cgaagcgtgc gcccaggacg 3720
ttcgggcggc gatgctcatg cgcaccggcg aaaacatttt cgatgtgaac tccggtgtgg 3780
gatatttcga atacatcttc tcgccgcaga aaagctatga tgacgctcgc aaatccatcg 3840
cggatgcaat tttatcctca ccggatgtga ccggcatcga gcagcttgac atcgacataa 3900
ccggggaagt cttcggcgtc gatgcgaaag tcatcaccat ccacgggcct gttaccacag 3960
gagtttgaaa tgagcacaat ccgcatccaa tacgccaacg gcacccaact gttcctggac 4020
ggcaaaaatc cgccgcccct ggacccgctg ccctcgttca acccgtctgt cgaagatctg 4080
gaaggcctgg accgcgaaaa gaacaccgac aagggcgact cctcgccggc cggtcttccc 4140
gttcccccgg taaacgtcga ttccaatgtc gacaacggcg gaaccatccc ggctccggta 4200
tcgaccgacg ctcctgcggc cgaatcggcc ccggaaggcg ccaaggaagc tcctgcagcg 4260
ggccaaggcg acgagaaagg cgccgaggaa gccccgacta ccgccccagt agaaaaggcc 4320
gaggaaacgt cctcgccggc cgctgaagag gcagccccgg ctcccgccaa ggccacctct 4380
cgcaaaacca ccagcaagta aggactcgac atgatcaacg tcagcggctt cggcacagga 4440
attgtaatag tttccgcctc atcgttcccg atggggtttt ccttgtcgaa gttcgctgat 4500
gatgagagtc cgatatcctc caaagagctg gagccgttcg gatatgagat gctttatgac 4560
ggcggcctat tcgcctttga caaggctgct cctctggaag tatctgtatc cgtcatcgca 4620
gggagcgagg atgatattaa ccttcgcatc cttctcaatt ccaaaaaggg atcattcaga 4680
tttcttccgg gaatcatccc ggacatgacg actctcgtgg ccactcttcc tgatggcggc 4740
cgcaccgttc tgtccaatgg gaccatcctc aagggtccgg ccatcgacac catacagaac 4800
accggacggc gcaaaggcaa cacgtacact ttcgttttcg gtagctatct tggcgcccag 4860
actgcgcgtc aagccatttc taacgttatc caatcggtac tggaggtggt ctgatgttag 4920
ggattttcac cagcctccta agttcgcgat ctttttcgat tgtggaccaa aacacaaacc 4980
agctagttgc tgcggatttg aggataagcc gggtcaacac ccggttttct tctgtagggc 5040
aacgccacat gctggaagat ggtacgacca agatggattc cagaacgatc caccctatgg 5100

aaatcatcgt cgaagtattt tgcccttcaa ttgatgtcgt cgatcagatc aatcaattgc 5160
tcctggatcg cgacacactg tacaaagtca tcactcgcgg catggtattc gaacggatgg 5220
tgtgtaccag cgaagcgctc aatcagactc cggatatgat atcggcgact cctgcgcggc 5280
tgacattctc ccaagttctc gtccagaatc ccaagcctat aatgttcaga aatgcagggg 5340
actcttctat gatcgaccga gggctggccc tagctgaaga cgtggttggc tcggccggcg 5400
atctgttcga ctacgcagta aacggcgtcc agaacgccgc agacttgttc tgaggtgcca 5460
attgaactct ttcctcaagt ctattctcaa cacgcctact ctcaccatac gcgatgatgt 5520
caccaaactt cctgtctgga agagtcttca agtcaagaaa gtggaaattt actcgccggc 5580
ttccgtagtg tcgaaacctc tggcgacgaa agaccagacg gaagctcagg tgtataccga 5640
agcgctggac gttgatgtaa agaacgggaa gatcattcag ccagtgcgtc ttcgcatcaa 5700
tgccatctgt ccagacttgt ccacagttga aagtatcatg aatgctttca atgacaatac 5760
ctcgactttc gccatcactt ccaagtcgat attggctgat aaaatggcca tcatgacgct 5820
cgatgtagat caatctcctg acatgctaaa tgcggctgag atcaacatgg aattcgagca 5880
ggttgagcct ccagtattga atgaatttga tccggctttc cctcaagatc gcccaactta 5940
tggcgtgcag attcagtccc tttccgatgc aaatttgcta gacttgggag ccaccggcga 6000
ttcgatatct tcggccgcaa aatcgctata taatcgcgtg accagttatt tctgaggatg 6060
tatcatgctt gaaatcaacc ttcccgatgg ccgccaaact cgcgtacaaa tcgaggcgtg 6120
gtcggcattg gacggctggg aactccagcg ccgtttcgtc gaattcgccg tcagccaaga 6180
ttccgatctc cgccgctctt tcaccatgga aatccttggc tatgctaaag tgatgcttgg 6240
cgacgacgac accggtattc cgctgaccac tgcggcggtc atcaacaacc atctcggcca 6300
ctggaagaac gtggaactgg ttttcaattc tgttctcaag cacaacggca tcgacccggc 6360
cacgcacgcc gaccggccgg actattggga acaagtcgga tcgcagatgg ccatcgcatt 6420
tctggccgag gcgtccaagc tcattggtcc agcaatgaag atcgccgaag gactcgccaa 6480
caagccggag tgattcatgt ctagtgattt ggatgaattc atacttcggt atgaggccga 6540
tactgccaga gccgagcgca atctggagcg cctccagaac cagatcaggc gcgtgaacag 6600
cgcatcaacg agtggccttc aggatttgcg ccacttcgca gacggcgcgg ccactgaact 6660
cggccgagtc gttccgcaga tcgattctgt gacgagcgcg attcgcggga tgaacgcgca 6720
actggcgata ggcgccactg gcgtggccct ggtcgcggcc ggcgtcaagg cgttcatgaa 6780
caccagggac cagtacaacc agcaacgcat ccaggcgatg gatatcggca tcgccccggc 6840
gcgactggaa gagtaccagc ggaagtttgc tcgccagtct ggaggcacga tcagccgcga 6900
gcagggcgcg gagatgacca aaaatctggc agacactttc cggcgtgctt atcgcgacat 6960
cgggcgagtc ggcccggaag cgcgaattct gcgtatggcc ggcgttgatg tcgggagctt 7020
ccaaaagggc atgcggccgc tcaacgacat cattactgat ctggccacga aaatggccaa 7080
gctgaagccg gacgaaattt ctgcctacgc tgatgccctt ggcgtctcgc gagactacct 7140

gagcaccctg gctaagatcg gcccagccat gggaaaagtc accgagatga cgactgcgga 7200
actccagtcc agggtccagg gcgagtccaa cattcagaaa ttcaacgatg ctctggcgaa 7260
tctcaaccag acgttcacca ccctggaaaa ccgagtcggc gaaaagctcg cgcctgcgtt 7320
caccaagctg atcgaaatca tcgacaagat agtccaggct attcccaatg aagtggaaga 7380
attcgccaag gacaccaaag cccgctggga tgacggcatc accggaaagg ctacggttgg 7440
tggtgatatc ctttcccttc tcagccctgg cgccctgcta ggccgcctgg cctcctgggg 7500
aactcggcgt gggatggaag aggcaggcct gatcgacaag tcgaaggttc ccggcgctca 7560
aaccagcgaa gacctggcca agaaacaaga agaccaggac aaggctacta agtccatgaa 7620
agagctagag aagctggctg accagaccac gaagtctacc aatgattttg cggtggcgat 7680
caacatgttc agtggcgcgg tatcgtcgtt cgcgaatgcc gttgacgagc gccaagcttg 7740
ggcagcctgg gcgggggaaa ttggtcgggc ggtcggtatg ggaagcaccg cgccgacttc 7800
gcgggccacc ggcgtctatc cgcacgcgat ctatgatcag tcgaagagtg gcgcggccgg 7860
acaggtcttc ggcgagccaa tcggcgccca gtctcttcgc aatcgcatgt tctcgccgca 7920
gcgcaaggcc gaaccggtca ccgttccatc gtacatcaac gatatcatca aagatgcttc 7980
gaagatgtac aacattcctg agctggacat caagaaactc atatacaccg aaagccgatt 8040
caacgccagg gccaccagcg aagccggagc taaaggcctc atgcagctga tgccggaaat 8100
cgccaaggcg tatggtatca ctgacgtata tgaccctcgc cagaacatcc tcggcggaac 8160
gcgcctactg cgggaaaacc tagatcgagc aaatggcgac atgcggttgg ccttgaccta 8220
ctatcatggc ggactcgatc cgaagaactg ggcccaaggg actcgcgcat atccgggttt 8280
ggtaatgagc gctccaatcg agctgatgga agaggctcag cgcaagcaga aggctgcggc 8340
catgaccgtc gccaacgaga cgttcgcccc ggaaggcggc gacatggaca ttcgtcccta 8400
tgatggcggg cgactggaaa ccccagacca gggcaagaag gaagatgagc gccgcgaagc 8460
ccgtcgatat gacgacaggg ttgtgcgacc ggaaattcgc atcatcgacc gcatgccaga 8520
ccgcagcgac ggcgaaatcc tcaagatgtc tagacgtcaa gaggccgacc gggcagattc 8580
tggattccgg aagttcccga atcaagttcg cggcgagacc aagcagaaca tccaggctca 8640
actcactgca ggagccatcg cgcaagtgat cggcgttaac cccaaccaga tcatgcgccg 8700
cgaaatcagc cgttctgact tgctgttcgg atacaaccaa gccatcctgg gcaagcaaca 8760
ggagatcaag gccgccgcga ccgaagccaa caacgtattc ctgtctccag ccaagcttgc 8820
cgaagccacg gccaaggtga acgccgcatc gcgagaaatg gatattctcc ggacgtacgg 8880
cgagcaactt ctgaagagcg ctccagagcg cggccaggag ctgaccatcg gtcgaatcga 8940
catgttggtc aacgtcaccg gcgcgaattc tccggaagag gctcgcgaga tattcagcaa 9000
gcagactgca gaccagctta ctacggcaat ccaggacgct caaaacgatt ccgcaactaa 9060
gatactctac tgatgaaaaa gagaattctg cgagtaacat tcaacatgcc ttatggaccc 9120
gaagtcatcc gcgaagatct ggatgttcgg gtccggatta tgaaggctgc gttgcggatt 9180

cagaaccggg cgacgatgga aattttcggc ctcaccactc agctgcgcga gtctcttctg 9240
tcgcagttca ccgcatggaa gcaccggcaa cgtcaagtgg gcagggaaga tgaattgatg 9300
atcaaggtgt cggtggaagc cggctactct gaccaggggc gcgagcaagt ttccagagtc 9360
tttgtcggcg aagttgccat tgtcgatatc atttctccgc cacctgacat tggaatccgc 9420
atccagtgct ataccaggca aatcgacagg acgaagacca ttcggaatat gccgcccgcc 9480
aacacgacgt ttgtcaagtt cgtcgaatgg ggcgcgaacg aaatgggatt gaacttcatc 9540
tgcgatacca gttacaacga tcaagttctg aagaatccgg gccggtctat cactgtcgcg 9600
tcggcaatcc tggcgtcgat tcaggacatg tacatgccgg acgtggccgc gttcgtcgat 9660
gatgacatat tgatcgtgaa ggaccgcgat aaagtcattc gtccggatga ggtgaccaac 9720
gtcaactcgt ttgttggaat tccatcttgg tctgaatggg gcgtggaatt ccagtgtctg 9780
tttgagccat cgattcgtgt ggctggaggt gtagcggtcg aatctctcat gaatccaagc 9840
gtcaatggta actatgtaat aaccgctctg gaatacgatt tggcaagtcg ggatcggccg 9900
ttctatatca aagtcatggg gagtccagca gcataatggc cagggaaatc aaatcgttca 9960
acatgttcgg agttcactat acttcgcggc aattctctgc tgtcgatggg ctcagcatga 10020
tgtcggaaat tcagagcgtg ccgccagaag aattgctcaa gggcaccgat gtattggcgc 10080
atccggaaga ccatccggaa ggcatctggc ttccattgac cgctgcgaac atcaatcttt 10140
atgtcgttga tcgagcgaaa gtaatagctc ccgtgcaagt tcttgcactt ctgtcagaag 10200
tggtaatcga ctggaacttt ggttttctca aagactggac cggagtcaag attccatcta 10260
gatttgtcga agatatcaaa agtgtgaaga cggcacattc accttctgta gttgcgagtt 10320
tggtagcgaa cggttcggcc tctatgcgcg agttggaaga gtattattcg actcaagatg 10380
cctttaagat gatagacatc atgacggcga agagcgtgaa cgaggccttg gcgtccgaag 10440
catcgcacaa cagaatcaaa aagggataat tcctaaccgg gcctgggaag gctatactag 10500
acctgccaaa tcagaggctt tcccatgtcc aatatttctc taacatccgc aaaagctccc 10560
gacaggacgc gactgatcgc cgctcttgac gctcggtcgc ggcgggatgc tctagatttc 10620
gaagtaatga taccggctca ggttgttcaa tatgacaggg ccgagaatat cgcgacgatt 10680
caacctctca tcacctgggt tgacacggaa cacaacgccg tccaaaggca tcagctggtt 10740
gatattcctg taatttccat gggggctggc ggcttccaca taagtttccc gattcagcaa 10800
ggtgacatag gctggattta tgcggccgac cgcgacacgt ctcagttcct ggagtcgttg 10860
tcaatgtcga agccgaacac cggccgcatc cataagttcg agcatggcct gttcatcccg 10920
gacgtattcc gtcgatacac catcaattcc gaagattcgg ccgctatggt catacaatcg 10980
acttccggag cgaccagaat ttcgattcgc ggcgataaca tcaagataac tgcgccgtcg 11040
aacgttactg tggatactcc gcaggcgaac ttcactggca atgtgactat cgctaacacc 11100
ctggtcgtaa acggcatcaa cgtgaacaac cacggccacc tcgaaaacaa cccgcctgat 11160
gcccggacga aaggcggcat gattgcttaa ggagattttc atggcaagtt ttgatttttc 11220

tgatttaaca gcgggggggg ttgtaatggc taactacaac tacatcgtcg atactggtgt 11280
gatagtcgca gataccgccg acgttctgag cgacgttgag gccgagttcc gcgccgccct 11340
cggtgccaat atcaacttgg ccgcgagcac tccgcaggga tcgcttgtcg cggccgaggc 11400
cattgcccgt tccagcgtca tgcggaatga agcgcgaatt gccaatacca taaacccgaa 11460
cgtttcattc ggaacgttcc tggacgcgat ctgtgccttg atggggatcg agcgtggttc 11520
tgacctgtca accttcggct atggagttca agtcaccggc cgcagccaaa cccgcatttc 11580
cacagggtct agggtccaga ctccggccgg cgcgatcttc acagtgctga gcgatgtcac 11640
gatccctgct ggtggtgtcg cgaccatcga tatcaaatcg caggagtatg gcaacattcc 11700
tctgccggtc gggaatctca tcatcatcga cggaacaatt ggctggtccg gagcgaaagt 11760
catcgcctcc actcgcgtcg atccgggcag ccgccaaatg agcgatgcag agttgaagaa 11820
tgcccgcgtt aatcgattgg ccatccaagg ccgcaactcg actatggcca tcaaggcgta 11880
tgtgagcgcc gttccaaacg tcacgtcggt gaacgtaatc gagaacaaca ccggcgccgt 11940
tcaagtggtg aatggagtct cgtttaccct tccgtatgct gtttgggtct gcgtcgccgg 12000
aaacccggat aaacaagcag tcgccgatgc gttgtgggcc gcccataacg gcggaactcc 12060
atgggactat ggtgcgacga acaacggcgt cccggtggac ggtccgaatg gcgttcctgt 12120
gcgcgatccg gcgtccggtc gaaagtatgt cgtgaagtgg accactccaa tcatgtacga 12180
tggatatgtt aacgtaacag tccagcaagg ttcctcctct gtagctccgg aagccattca 12240
gaacgcagtg gtcaattaca cccaggggaa agtggaaggg gaagaggggc tggtggtggg 12300
cgcgagcctg tcggcattcg aagtcgccgg agccatcgct cgcgaaattc ccggcatcta 12360
catcaaactc tgccaggtag cgtgcgtcgc ggctggctcg ccggctccgg ctccgggcga 12420
tttcacttcg gaatacgtca tgagcgcatt cggccaggct accatttccg ttggtaacgt 12480
tcgggtgact ttcgtatgac tctgcctgcg tacaattcgg acatccaaca agctctgaaa 12540
tggctccata accaggcgcc tggaatcacc ggcctgatcc agagaaaggc gcaatggtat 12600
gacagattca gccgccaatt ctgggccaac tgggagcgcg acgttttcaa cttgaagacc 12660
gccaacccgt tcggccttat ggtttggtgc atcatcctcg gcacgccttc gaaagggttc 12720
ggcctttatc caaagaacag ttcttgggca ttcggtcggc tacgccagaa cttcatctat 12780
agcggtacac aagtcccgcc accggcagac gcatcgcctg gcggcaactt ctacggtggc 12840
ggcaatgccg aaattctcaa cttggacgaa atcaggaaag tgcttcagct aagatatgta 12900
gcgctgattt cgaacggctc gattgcatat atcaatcgca tgcttcgtta catattcaat 12960
gatgatgagc catgggacga ggcgaccggt ctgtacttct atctcatgga ctcaaccggc 13020
gagaatggcc ctgtggagaa cttggcagta tatcggaaag attgggaagg tatggtgttg 13080
ttgtccagtt cgcccagaac gaaccacgtg ctgacatcga cccctgccag cgacgccgat 13140
tggccaggag tcgatccggc cgcgagcggt cttccggtaa cggtcgaaac ggcgtccgct 13200
acggccccgg acggctccgc tacggtgtgc aagcttacta agccggccgg gagtaccgct 13260

tacgtctccg cgccgataga tgggccgctg gggtccggta gcactgtaac gttctcgttc 13320
ttcgcgaaag ccggctccac ccgtttcatt gcaattcagt cggctgccga tttccccagt 13380
cgagccgatg ccgttttcga cctggattcc gggaacgtga tcagcgatca gatgttggac 13440
agcagcgtgg taagcgcccg aatgattcgt ctggagaatg gctggtggcg ttgcgttctc 13500
acgaccaaga ccgtcagctc ttcgttccgc gcggcttaca tcgctccggc agaaaccaac 13560
ttcagctgga ttgattcgaa ttccagcgcg gcgattgatg tgcttatctg gggcgctcag 13620
atcgaacttg gtgatactcc aaccggatac ttggagacta ccggaacgcc cgtaaccatc 13680
accgattacg ttctgcagag cgcccagacc ggaacggtca agttcacaca gcctcttccg 13740
accggagtag aagcgtattg gactggagac tggaaaggtg ggtctgcgac cgagccggcc 13800
agattcgcag taggggatgg gactcaagat acattcaatc tgtccagccc tgcatacatc 13860
ggcctaccca ctagtggggc gttcaagcta gaatacagag ttggtccggc gcttaatttg 13920
tcgccgcaat tgatcaacct cataaatgac cgggcggtcg gtatcatgcc gacttgcgcc 13980
ggttgcgatg taaaagtcat tcaggagtaa tgacgtgatc acacccgaac tgatacccag 14040
tccgtttgct gcgcagggcg acaaagaccc gatcccgcag acctcttcca ctggctttgc 14100
caaccttcgc gacggctaca cgccggacta cgaaatcagc ctggcgtcga acaacccgca 14160
ggccaaagcg gtcgagcgaa agattcaaaa ccaactcttc ttcatcgcga cccagaatgc 14220
gcaagcctgg cagcgtcaaa tggcgccgcc gtggtttcag ggtatgcccg gtgggtatga 14280
acggaacgcc gaagtcgtgc gcgtcggcaa tgacggaatc atgcggcgct atcgctccat 14340
ggtgaacgcc aacgccagcg atcctctcag cagtaccacc tgggaagagc aacccgcctg 14400
gtcagttatg cgcaccaaca taccgatgcc ggctggcggc cccggtctgt cttcgggcgg 14460
cgaagtcatc accaccggcc gcaacttcaa tgaattgctg aacggaacct gggaattttt 14520
ctctgatgca atcgtcgtgg cctctcagaa cgctccggtg tatccggcgt ctgccggcgc 14580
ggcagcagga atgctggagg cgaaatcttg ggtgtccggg tcaaatacgt tctgcgtcca 14640
acgctacact gatcgcgtcg gaaacgtcgc cgtgcgcggg ctcaatgccg gggcctggac 14700
caactggatg tacgctgtaa acgtcatggc cctccaacac ggccgtgtaa cctatggaac 14760
cgcggccggt ccggcgaatg cctacacctt gactctcgtt ccgcagatcc aaggtggttt 14820
ggtagatggc atgatccttc gggtcaagtt caacaccatg aacaccggcg ctactaccat 14880
caacgtctcc ggactcggcg ccaaagccat cgtcggcgcg gccaacttcc ctctcactgg 14940
cggcgaactt ggccaaggac tcatcgctga gcttgtcttc gacgcagcag gcgaccgctg 15000
gaggattctg gcaggcgcgc cgcgcatcca agtcggcaac gccgatcagg actaccaggc 15060
gccgagttgg aagcaagtta aagactatgt cgcgtcccaa aagctcaccg aagtggattg 15120
gacggatgtc gtcaacaagc cgaacgtcgc tatccaagat acaacgccgt ggttcgccaa 15180
tctggagctg tccgatgctc ggcctttcat cgatttccac ttcaacagca atcgcgccaa 15240
agattttgac tatcggctga tatctgaagc agacggatcg ctggctttct attcgcggca 15300

ggggtctgct gggcctaccc aggacatcct gttcaaccga aattccgtga ctttcttcca 15360
gccgcgactc gatgtggcga aaaacctcgc gtatatcgcg aactctggcc ccctttggca 15420
gaacaccacc gccgatcagc ccggttggaa attcaccttt gcacaaggcg tggacgcgaa 15480
caacaacgcg gtaatcgcag tcaataccac caatccggac ggttcctatc gttcacaggt 15540
catgcgatgg gactgggcgt ccacgaacgt catattcaac aaccgtccgc tcttcgccgg 15600
tcaatacacc ccttgggatt ctgggaactt cgatccttcc accaagttga cggtgagtgc 15660
cacgaaccaa atcgccggcc caaccgggat tcggaatacc aacggcaaca ccggcaacat 15720
gaacacctgg ggttccggct ccacgacggc atcctatggc aatgctgcca ttcaaatctt 15780
cggaaaaggg ggcggtgagc ctgccgcgat ctatttcgac aactcccaga ccggatggta 15840
tctgggcatg gacaaggatg gacagctcaa gcgggccggc tggtcgctcg gcaataactc 15900
ctatgtgatc accgacgaat ccaacatccg aacccatatc aacacaatgg ctgcgcgccc 15960
gatttggggg aatgtcgagt tctggggtcc gtggaacttc aatcctaaca cgaagttgac 16020
gcttggctca ttcaatgaca gccagcatac caggatggtc aacagcgccg cgaaggatgt 16080
aggaattgcg tccatgacga gctatgcgga cgcggctatg tcgttcttca actatgaggc 16140
ttcgacgccg accgggaatc gtgctgctgt aatttcgttt gttcgtaacg gggcacgagg 16200
cgttctgttc ggcctggaca cggacaacaa gctgaaatgg ggcggctatt ctctaggtgc 16260
cgtcgcgttc gagattgccg actccaacaa cctcatgagc ctgtggtcat cccacgctgc 16320
cgcgccgaac tggaacgggc agaccatctg gaggtcggga aacttcaacc cagacaccaa 16380
ggcgactttg gcagctcgca atacgacgtc atcccctaca atattcagtt atggggcgtc 16440
cggaatcgca tcaaccggac aggtcggtgc gttggttgtg gaaaacaaca gcgtcaccaa 16500
taccgcagcc gccatcacgt tccattcgcc gcagaaatat caggtcaact tcggcctgga 16560
tgcggacaac gtggtaaaga tcggtggcgg cacaatgggc ggcgtagcat atcccatcat 16620
ccactccggc aactacaaca actacatcaa ccaggcgctg gttcaggtag gtcttggcgg 16680
cgtcggttcc tacgcgatcc ttgccgtatt ggacacctcc gcgccggcag cgtccattgc 16740
ccccggaacg atcatggaca gttccaagct gttctattcg tcctgcgatt cgacctatcg 16800
cagcagcgcc cgtccgacgg gcacctggcg ctgcatgggg tatgtgtata accgagactc 16860
caccaacggc gactcggcat ccctattcca gcgggtaacg taacatgaaa tggacgcgga 16920
tcagaaaccc acgttggctg gacgcggtaa acatccacgc catggttact ttcgagggga 16980
ttggagaagt tcctttcacc gccaatccgc acgacgtgga ggcccacgga agggtcattc 17040
acgctgcgat cctgtccggg gcgcacggac ctatcgcccc agtagactcg acgcgggagc 17100
aggccttgca ggacgctata cgagacaggg aaaagcgggc tatccttcgt gatacccgct 17160
gggccataga tcgtcacgac gagcagaggc ggctgggtat cgaaaccacg gacggacctg 17220
agttgatagc agcccttgtt cactggaggc agcagattcg cgattggaat agcggggatc 17280
ggccgcgact tcccatggct ttgaaaacaa tgttcaaaaa tcaggagtac tgatgaaaat 17340

aacgaaggat attttgatca caggaaccgg atgcaccacg gatcgggcga tcaagtggct 17400
ggatgacgtc caggcggcca tggataaatt ccacatcgag tcgccgcgag ccattgcggc 17460
ctacctcgcc aacatcggcg tcgagtccgg tggcctggta agtctggtgg agaatctcaa 17520
ctacagcgct caaggattgg ccaacacctg gcctcgccgg tacgcagtag acccgcgagt 17580
ccgcccgtat gtcccgaacg ctctggcgaa ccgcctggcc cgtaacccgg tcgccatcgc 17640
caacaacgtg tacgcggatc gcatgggtaa tggatgcgag caggatggcg atggttggaa 17700
gtatcgcggg cgcggcttga tccagctgac cgggaagtcg aactattccc tgttcgccga 17760
agactccggc atggacgttc tggagaaacc ggagcttctg gaaactccgg ccggcgcgtc 17820
gatgtcttct gcctggttct tctggcgcaa tcgctgtatc cccatggcgg agtccaacaa 17880
cttctccctg gtcgtgaaaa ccatcaacgg cgccgcgcct aacgatgcga accacggcca 17940
gctccggatc aaccgctata tgaagaccat cgccgcgatc aatcaaggct cctgatattc 18000
cacccaaaga aaaggccgct tattcagcgg cctttttgct ttccggcttt gcctcttcaa 18060
ccatcttggc ctcaaccggc gcggcggact cctcctgagt gatcgagtcc acatagttcc 18120
ctagtgaact cagaacgccg attaacagcg ctcttaccac cttgtccttg actgtctcgc 18180
ctatgatctt ggtcagaacg gatattaact cttcccggag tctggggctg attcttggcc 18240
gaaagcgctt gcgatgctct ttgcgtttca tgtttagtcc tctgtctgcg gtcttctcct 18300
caccccgata attgcttggg gatgctttgt gttaatcgga agggtcgggc gctattataa 18360
cccgtcgaaa atgctagcgc ttaactgttt aacgatacgc accgcgatat taaatcgcct 18420
tctttctggc cgaggaactc tggcggccgg gtccggtcta aggcctaatt tgtcgacatt 18480
aaaacgagaa aacccggatc gcctgtaggg taaggcgtcc gggtttattt cgatctagtg 18540
tccgctagaa tcagtggctt ccgccccatc cgtccagcca gcaatcgaag acggcgtgtc 18600
tcggcttgtc cttggcgcca tgggagaagt gcttgaaccg gatgacctgg cccttgagat 18660
actccctgtc attccagcgg cgctgtttct cgtcgtgggt caggctggag gccgacacgt 18720
tgaaggtgac tccaggccac agacgttcgt tgcggcagac gaacgcgccg accattccgg 18780
acggcgacag gttttccgca tgactggagc gggccgtgtt accaagttca ttgatgaacg 18840
cttcattgtt gttgtgcatc aactcttcca cgtcgatgat ctcagcttca tcgtagtcgt 18900
agcgcttgac tttcacacag tgcccttcct tggcagtcga gcgaccgaac ttgtacagtc 18960
catcagcgcg cttgcccatg gaaccttcga atcccagcat tgtgtggcgg cgctcgactt 19020
cgctgaactg ttcgatggaa gtcaccagtt cctgctcgac caggtgaatc ctctcatagc 19080
cgaggcagtg acgaaggaag ttcacgcgct cggccgccct ggctaggcga tcttcggtcg 19140
gtgcgcgagg atcggtgaaa tcatcgaaca catggaaaga ccagtccggc tcaccgctgt 19200
ggcggcgaag atcgccggac gacttctgga aaactttcgg gtcgctgatg tcgccgcaga 19260
ccagctcgcc gtccaggccg ttgaacagtt tatcgctgag gtattcgtag atcgattgat 19320
tgttctgccg cttgagttga cgagtcagcg cctcgccttc gaatacgaag cagcgaaatc 19380

catcgatctt cggcgagaaa tacatcggca gttggccttc cagcagcttc gggtcaaaat 19440
tcgatgcgag catgggtttc atacagttct ccagaaaaga agcccggcga accgggctaa 19500
atggcggtaa gccggctcag atggtttcgt tggcgtggtt cagttcggcc atgatcgacg 19560
catagcgctc gtccgactcc ttgatgaaca cgccgtcgta catgacgcct ttgcgatcct 19620
tgatggtgtc gtaggccgca gcgtagcagg cgagcatggt agtgtcgtgc tcttccgtca 19680
catcgaacag agtcatgacg gccaagacca ggctcttgat ggctaatcca tgatttccgc 19740
gtgccagcgc gccggccaga tgtccgaggt gctgcaaatc gtcgccgtag gtgggatgaa 19800
cttcgacagt ctcgctgaag accgatagat gatcgaacag attttcaccg agttgcgcgg 19860
ccatgatcgt ggcaaccacc attacgtcgc cgatgccgtc tttcacctcg actggtttgt 19920
tataaaccca ggcttcgcaa acttctgcga actcttcgac caacttcagg aattgatcct 19980
tggccgaaga gcctttgatc aggttccggt cggaacccca tttaacaacc aggtcgtgaa 20040
gttcgcgact catgatttgt tcgatattca ttctttcgat tccttttgga tttgggattt 20100
gactgcgttg attatggacg ccgtgctctg gcgcgatcca tccttggtgg tgccgaagta 20160
gaaggccatt accgacttca gctcggcgaa ccaatagccg atgatggtgc cgatggcgac 20220
cgaggatgtc gggtccatca gagcctcgcg gccgaaggtg aagattgcga tgatgatgag 20280
aatggagcct gtcagaagag cgaatgtaat cgccgggcga acgaaatcgt tctgctgcgc 20340
agcaagcctc ctcgccgaat ccctgtctgc cgcctctgcg gcgaactggc taagctcggc 20400
ctggagctga ttctgctcag actgaaggcg attttgttcg gcttgaatcg caagctcctg 20460
gagacgaacg cgctcggcgc tctggagttc ggcgaggcgc gctagagctt ccggattcgc 20520
gtctagagcg ctcgcgactg aggctgggtc ggccttcgac cccagcgccg tcgcgacgat 20580
agagccaacg gcggcgcctg caggcccacc caggagcgat cccaacgccg gggctgccgc 20640
gccgatctta ctacctatgt ctttccagtc catttcgact cctcaaaaga aaggcgccat 20700
tacagcgcct ttctccggcc ggtgatttag aactcttcgg cttcggtcgc atcgccgacg 20760
ccaccggcgt cactgcgagg ctgttcctgc ttgctgtagt ccaccttcac ctcgccgccg 20820
acgaacgact tgtacaggtc ggccgcagcc ttgaagtgat ccgggttctt caccaggcct 20880
tccagctcga actggacgcc ggaccagctg cccttgtcgt tcgacatgcc tacggtggtc 20940
atacggacca ggttggcgaa agtcggcggg gtgcgcaggc cctgcggggt ctgaaccttt 21000
ttctgggaca gcgcggtcat gagcttcttc gaggccttga tctgcgaaga cgacagggag 21060
atcagagcct ggccgaaatc gccggtgtcc ggatcgatga cgatgacgta atgaccacgg 21120
gtgtcggcga agtagtcaga cttcttgtcg ctgaccgaac cgtcttcgtt cggcgcgtac 21180
aggcgtcctt cgacttcctt caccttggtc gggtctttca tcatttcctt gaagtcttcg 21240
acgctgatgg cgcccttgaa accgccctcg gcctcgcgac cggcccagcg gatgaactcg 21300
cggcggtacg cggccggaat gatcagcagg ccggtcttgc cgtcgtaaat cttgccggtg 21360
acggtgttca ggaacatgcc ggccttcgcg ccttcgatgt acttcgggtc atcttcatcg 21420

acctggggag acatcttctg cagaacctgg atgaagggga tggcatagga ctcggcatcg 21480
gcgccttcga agcctgcgcc gtcatacgat cccaaatcca tgaagtcggg aacttcggta 21540
gttgcgacgg cgccgccacc ggtggcaact gctacggcct tggtttcttc ggttgcttcg 21600
gaagtttcgg ttttcttacc agccatgtta ggctccttgt ttgtcgaatt tcagttatcg 21660
ctaactgtgg gtttataata acggaagttg caactaagta aagcaaatta catattaaga 21720
tttgctcttt ttcaccttcg gtttcgtgat tttggcctct ttgtattcgt gcacgccgat 21780
gaaatctggc agctcttcgc ccttctccaa atactcgcga ccgaacgcct ggagggtctg 21840
gtagtggaca tcgcggttga tggtggcgtc atagccggcc tcaatgatag cctcggccgc 21900
cttcttcgca tcttccattt cgccgcgacc gaattcggcc agaaccttgg tcttgatgat 21960
gccgtcgttg tcggtttctt ccagccactt ccagaacttc gatttgttct cttctttgac 22020
ggaaatgatg gccttcggct cgactttgac cgtgcgaccg tcagccagag tagtggtctt 22080
ttgaccaagc tcctccagca gctccggaat ggtgttgcgc ttgagggttt tcagctcctc 22140
ttccttttcg gccagcgcct tttgaagatc gaggatttcg acgtccagct gcgaagcctt 22200
gtccaccagg ttcaacagtc gatggccgat gtcggtggct tcaaccgcca tttcatccat 22260
gacgccaaaa tagtcgattt cgcccggcgc gttctctttc aagtattccg ggatttcaag 22320
ctcttgctct ttcatgtccg cctccaactt agtgatgttc ccttacttga accaagtatt 22380
gagtagatat tatgccgcat cttccttgat acggctactg atttacatat taaatttcgt 22440
cgcgagtgct aacgtcagcc tcaaacacgc catcaacgac ataacttgcc aggttgcgtt 22500
tccactccaa gctgacctgg attttctcgt cgatggaatc cagacagatc aggtcgaagt 22560
acagaaccga gttgacggtg ccgattcgat gatttcggtc ttcggactgc atccgcagct 22620
cgttgtcttc gtcggtcgtg tagtagatgg ccacgtctgc ggctgtgagc gtgatcccga 22680
tccctgctgc ggccgggttg cccaggaaca cctggacgcg cttggcctgg aaatcgtcga 22740
tcaatttttc ccgctcggcc tctttggtct cgccgtagta ggtaccaaac gaaatccctt 22800
gggcctccaa atacgccttg atctggtcga tttcctgaat gcgcatggcc cagacgatga 22860
tggaacgctc cgggtcttcc tccagcagac cctccagaag atcagtaaac acggcgaatc 22920
gcgggttgtc ttcgggcggc aggatcaccg gctcaccata gacgttgata tagccggacg 22980
ccacttgctt gagcttcgag cgcgctgccg cagcatcgaa agatacatca agcatgaaat 23040
cttcgttctg gagaacaaag tggtagtctt cttccacgcg ctggtaaacc tttcgttgct 23100
caggcgacat ttcgaagtat atgcgcttgt acactttgtc cggcaggaat gggagtgctt 23160
ctttcttcgt tacacggaaa ctgtgcggct cgatcaggga ccgcagcttg tccaggttcc 23220
ggaataccgg gcgcccataa tcgtcttttt cgacgagctg gggcggcatg gtactcttac 23280
cgtccaattt tcgcatgatg gctaccattc gcggatcgtc actgggaacc agaacggaaa 23340
attcagccac gaacgcccga taggatttcg tccccagaat accatcacgc aggaattgaa 23400
actgcatgaa caaatcggtc ggcgctcgcg tcagaggcgt gccagaaagg atgcgccgtg 23460

ccacggcctt ctcgcccaac ttaacaatct ttttcgcgcg tttggcttgt gggtttttga 23520
tcctcgtgga ttcgtccaca attgcgcaga ctttgaacgt cttgaggaat cgctccactt 23580
cgtcatagcc agcctgatgg ttgatggcat cgacgtttat ggcaaaaacc cgaagaactt 23640
tttcatctgc gaacgtctcg gcatacagac gatccaagcg cgccctggcc tttttggaag 23700
tcggtcggcc gcgccaatcc acgctcaggg tcttgatggc gacgtgtgtc ggaatttctc 23760
gaagaatcca gttggtatgg acaccctttg gggcgacgat gagcaatgcg tccacccggc 23820
cctgtaggaa gaggcgaacg gcatcggcca aagtcgtcca agtcttcccg gtgccctgct 23880
ccatcaggta tgcgaaattc cgtttgttca gggacgcggc cagggcattg aactggtgct 23940
gcatggcctc ggtcttcatg cccttgacgg gatatgtttt ggctttcatt tgttctccag 24000
atcagcgagg aactggacga tgttgtccag tccttccgca tagctcgcaa cttccaccaa 24060
atcgcggctg ttaagctcga acaaatcgag cataggattc agcagcagcc aatcggttcc 24120
gatcttggcc aggacgaagc cccggccacc ccagccgatc cgctcccgaa gaaaagggat 24180
ttgcccaggt tcgaaacatc tggccattgg gcaggtggaa gcgcgtttcg gccactcctc 24240
caaagccttg aactcgaccc agaactggac acctcgacga ttcaggcaga tcgaatcgga 24300
catgccagac cgccgcgtct ccagaaaatc gatcaggatt cgaccgagcg agcgctgctt 24360
aaacgcattc gcggctttcg tttcgcgatc attcatcgcc attcccctct tcggaatttt 24420
tctctgcttg cgctgccaac tttgacttct cgcgctctgt caatatccgc ttgacggcct 24480
tcacgatgaa catatcgatt ccgctgagct tccagccttt gatgaggaac caagaacctg 24540
tcggcgtccc ttcagcaatc tgctttccgt attgcagata tttctcaggc cgaattctga 24600
aacgtatcgg ttgatcaaca gaatcgtcca cgcacatgat gtcgaggaac tgcgactggc 24660
ctttgtacac cgggtttttc ccttggtcgg ccctcttctt ctggcgaatc ggttcgttct 24720
catcagacaa aactttcttc accatcttgg cgatgaccag gccatcgtcg ccatcgcgga 24780
tgtcacgaat gttctgaatg gggtttccgg aagtcactcc aaccagctcc ggattgtcat 24840
aagcgtgacc ccagagcgta tgagcttcgt tcaagtctgc gaactgaact tcagagtttg 24900
acaaactcgc ggcgactttc tcccaatcct gaagggtctt caggtgggga ccggccagct 24960
ctttatattg agccttcaac tccttcagat cggctttcaa ctccttcagg tcggacttca 25020
acaacttctc cagttctttg tcttggctga ttttcgccga gagaatctgg gcttccagcg 25080
ctgctacatc atcagactta tcctccacat cttgcgccga tatcggacaa ttggccaggg 25140
cgatcctcgc ggccttgaca tcctcgcgaa gacgcaggaa cctctcggcc ttagccgggc 25200
cgaagccttt ggcgttcatg atgccgccga tcaagcgtcc gtccgccacc acccagttga 25260
gttcggaatg ctccgggtcc agggccgtat attctacgcc ttccttggcc aattcgcgaa 25320
ggatagacac agtttgctgg tcgtccttcg ccgcccgaag gcacgcggcc gcgtactcca 25380
accgatggta tcgcttcatg tagcaggtcc agtacgtcac cacggcatag ctgaccgagt 25440
gcgagcggtt gaagccccag gcgccgaacg ttaccatttc ctgccaaaca cggtgagcat 25500

cgtccggggc gacgcctatg gtcttggcgc cctcgatgaa caattcccgg cgcttgttga 25560
aaaattcttc gccctttcgc gcagacatgg ctttccgaat cgccgacgtt tgttcccagt 25620
cgaactgccc aatgtccttc acaatggaca tgatctgttc ttggtacagg aaaactccat 25680
atgttccaga cagatactgc tcgacctgcg ggatggtata ggtcacaggc tcgcgaccgg 25740
ccacgcgctc gatatacttg gttgccatac cagaagacaa cggacccgga cgggcgagcg 25800
ccgtgatgtg gtcgatgttc tcgaacgcgg tgatgttgat cgcgtttgct accgagcgaa 25860
cggcctgccc ttcgaactga aagatgccgg acattttgtc ttcattgaga acgtccagaa 25920
ccgccttgtc gttcaaaggc aagtcgtaca actcttgcgc ggtcacgcag ttcgcgtcct 25980
ggatgacgcc cagagttcga aggccgagcg catcgatctt gaggaggttc aaatattccg 26040
aatccggctt atcgagctgc gcgacgcctt cagaagttac cgtacagaag tcgataactt 26100
catcgttgca gaccaggatg cctgccgcgt ggactccaga gtgggatggg tgaatttcga 26160
ggtcgcccat gcaggcggac gcaatctcat acttttcacg gaagtcgcgg ccgggttgag 26220
ttttttcgaa agtgtcctcc aatccttttc catatcgttc gtccgccgat gtatattcga 26280
tgatcgagtt tttgatgttg tcggtatcgt ggaaggggat gccgaagcgt tttccgacgt 26340
gagcgataac cgacgcggcc ttgagtgtgt tgatgttacc caacttcaca acgttccaag 26400
tgccatactt ctgctggaga tattcgaaca ctagatagcg atgggtatcg gcgaagtcga 26460
tatctatatc gggaagatcg gaccgggaaa tgtcgataaa gcgctggaag agaaggcgat 26520
gagggagcgg gtcaacctcg gtaatgccaa ggagatagca gaccaaagag ccggccgaag 26580
agccgcgagc tggaccgacc agcatgtgct tcttggcaaa cgcgaccaga tctgccacga 26640
ccaggaagta gctgtcgaaa tctttcagct gaatctgctt gatctcttct tggaatcgat 26700
cctcatactc ctgggtccat tccttgatgt ggccgcgact gaggcggtag gcttgcccct 26760
cgcgagccag agcgacgata tcaccatcca agtggatcat cggcgccttc gccagtttca 26820
catccgccaa ccgctcgact accgcacgag tattggtgat ggcggaatcg aactcttcgc 26880
gagtcatgat atggcgtaaa cggctccaca actcctcttc agtggcgatg tggcgaaggc 26940
cgaccgactc ccgcaccttc caggccgacg cgaaatcagc atggtcgatg gacggcatgt 27000
cgttgtaaga ggtaatcacc accggctttc cgaatgctct ggacgtctcc atagcaccgt 27060
gcgcggcgac catcgacgcg ggattgatgt caatgtagtc gattccggcc agatccagat 27120
gggcataggc ctcgccagcg aatttgatga cgccgtcagc ttcctggaat tcttgcggag 27180
tcaatccttg attctggacc gacttggaag tcaagcggta gaacttcttg gtgtccttgg 27240
cgagcaccca ggctttgagc ttcagctctt tctcgccatc gtcggcgcat ttgatcggga 27300
tttccatgcc gaatccgcga ggcagttctg ccttggtagc ggcttgttcc caacggacgt 27360
ggccccatgt tccatcatcg acgatggcga cgaatgggga ttcgatctct ttggcgcgct 27420
cgatgatctc cgggaatcga ccatatgcgg cgccgtagga gtaaccagag cgaacgcgga 27480
gctgagggaa agacatcatg cggcctccat tgcttgatac gcccgataca ctcccatgcg 27540

cttgcagact tcgtggagca gccgcacgtc gtcgagcgcc cggtgtttct gtacatatgg 27600
gccgcagtag tgctcataaa gatgttgcag ccgcatgcgg tggccgaaca acggcgccga 27660
ctcttccaca gtacagatat cgagcgatgg gaaattgact tcgtccaggc cgaacttgcc 27720
gcgagccaga tcgcaggtca gcatgaactt atcgaatggc aggttgtggg caatattcgc 27780
gtcggctctg gaaaagaagt cgcgaacttt ctggcgctga tcgaggaaag atgggtgctt 27840
gaccaggtct tcgttcttca acccggtgat cttggtgatg atttcctcga taacgattcc 27900
aggattgcag atgaactcta cttcatccaa aatcttttcg ccatcagtta tcacgccggc 27960
gaattcgatg attctcggct gctttctcag actcaccctt tggtggaacg ggagtcctgt 28020
ggtctcagta tcccatacag cgaatatcat gtttgttccc tcttatgtcg aaaggccggc 28080
tgctttcgcg accggcctga ggagtatacc gcgacggctg aagatttacg ccttctgtcc 28140
gtctttcggc gtgatgcggc cggagcgcat ggtggcgtga acaaacgccg aatagttgat 28200
cgagtccaag gccgaatcgg catccttgaa cccgctattc gccaggcgag ttagtttgcc 28260
caccatatgc atcacgaaca gggcgagccg atggtcatct gcggtcttcg ccaccaggcc 28320
gttcgggaag aggatttcca tgatcttgcc atacatcaga tcatttcggc cataagcgct 28380
ctggcggtcg cggaaaatgt ctgctgcggc gtacagattg ttgagaacat cttccgcgaa 28440
atcatctgga tgggaatcat cttcgcccgg ccagacggat tccatggcga acggcgcggc 28500
gtcttcggtc gggtcttcgg actgagccgt ttcagcgagc ggagacgggg ctttcgcaaa 28560
cgccttgtcg agggtagggg cggagttcgg gacggccggg aacggctcgc ctgccatgtc 28620
gttgggcgcg ctatcggggt cgctatcggc cgcgacggcg aagaacggcg catcgcaacc 28680
ttccaggttc aggatgtagg agtcgatccc caggctattg taggcatcga tgatgtcctg 28740
gcgatcatcg aacgccgcga cgatcttggt aacgccttcg attttcttca ggatgtcgag 28800
cgcgactgcc cgcttgaact gaggcgccgg ctcggtgtta ccatacggcc gcatgatgag 28860
ttcatactcg cgatgttcgg cgataccgag ttcgcggtga atcttggccc tggtctggaa 28920
aaagtggttg tcggttcggc cggtgatgaa gaagatcatg aggtcagcat cgatagcatt 28980
tctgatgcga cctacagcat gcgggttgag agcgtctttg tcgagacgag aatgatactc 29040
gtcccactgc cgttccaagg cgaagctctt acggtggcta tcgtcaaaga cgcagccgtc 29100
caggtcgaag atgatgatgc cattcttggg tttgcgttcc atattcagat ttccttgatg 29160
gttgctttct gggtgacggt tttatcgatg aacgtcccat cagaagtgag tcggaaaact 29220
tcgcagttct gagtgaaatc gaacgattgt acgttcatcg tgatgcgaat ggattcgctg 29280
ccgttggtca cttcgacttg tgcgatgtcg ccaactttct cgacaatgat cttcatttta 29340
catggacttc ccgttgatgg ccacaggatt ggcctcttgc ttttccgatc cccagaaatc 29400
gcggcgcaga gcttcctgct cggccgagcg atccatccag ggcgatagaa acttacactg 29460
gtagataggt tccaacggaa catcaaccac aggaatctgg ccgggcttcg tctccagagc 29520
ggacatgaac cggtcatagg atttctgctg gatttcctct tcattcatca ccttcgaccc 29580

atagcgcggg aaagcgcagg agccggtggc gacgcagtgc ggctggagca ggctgtcgaa 29640
catcggatag acttccagaa ccagccggcg catctcgcgg aacacttcct ggtattcacc 29700
ctgggtccga acacacaggc gaaccttcgc catgtcactg agagtccgca gattgaactt 29760
ggccgcgatc ttcgtttcca tgttggaagg aatgattgca cgagcgtcct ggagcgacgc 29820
gccggcctcc agcagcttct ggtaactggt ctgcgcgtcg gctatggcat cgtgccacag 29880
gcggttcagc tcttcgcggg cgtggtaggt cgggtcaggc tcgccattta ccgtagccgg 29940
ttcgtcgaat tcccagcgga atgcttccgg ctgaacgacg gcgctgattt ccagagcgcg 30000
actggtttcc tgctggtaag ccccggtgcg agtccgaacg agttgatggg tgaaattctt 30060
gctgactccc tcgatctgga aaatgaagtc cacgaactcg aacggcgagc ggatggtgtc 30120
cagcatgtac ttccagtggt cgagcttttc ggcctcggtc atggtcgccg ggtcttggcc 30180
gcgcatgcgg gtggacttcg ttcccaagag caattcccag gagttctggg tgtaactgat 30240
cagagaaatt ttcatcagaa atcttccgga attggcgtga agtggaattt ttcggtgaga 30300
gccagggcca ggccttgggc ttgctccctg ttaaggccat actgctctat ctcttccttg 30360
agcagttcgc aggcctccag acgcgcctca tactcttcag cctcatggac ggatgagaag 30420
acggctccat cagaggttcg gtacacaagt tcgatcgcca tggtgacctc agtagcagcg 30480
gatgatttcg gcgcggatgt cgcgacggtc gaggtagtgc tcacgaatct tgtcgcgggc 30540
acgctcggcc tcttcgcggc tgccgaacga caggttgaac gacgtgaaag gcttatcgtc 30600
cagttcagcg ccggtctgat acaggattcc taccagaacg aaagacggcg cggtcttcgg 30660
ctgctggtcg atttgcattt cgaggaaaga catgggaacc tcttcaggat ggtctggtga 30720
gtacatcaat agcgctcctg ctgagcagct acggtttccg gctcgtagat gagcatgtca 30780
acgatttccg gacactggcc tttcacccag tcgatggccg cgaccagtgt ggtggaagcg 30840
gagaaagaga aagtgcggta agactcatgg atgtacggag accccataga atcgcgctcg 30900
gtggtgcgcg aaatgactgc tttgatatta atgatccggc ccatcttcgg cctccacttt 30960
agcgattata tctgacaggc ttaacttctc gccattcagg aaataactgg cgcgaaactt 31020
cctgtcgccc tctggcccga cgattcgggc cgttatggtg agactcccgc cgccaagggc 31080
atcggtgtcc cggaagaaag ctagcagcgc ccgcttgagc gctgcttcgc gaggatcgac 31140
ggccattaac ctaccacgtt ccagccgtgc tgagcgcacc acacggcacc ggcccctgca 31200
ggaagactat tcagaaggaa cacaggagtc aggcggccat cctcggtcat atggacgaag 31260
tagcgggcgc cttcaccgag ccactcggcc ttggcgatgg cacgttcgag attggccttg 31320
gtggcgtagg tcttggtgtg gttcttgtcg gtggagaagg ttacttcgcg ggccatttgt 31380
cgattccttt tggttgaagg gtttcgcgtt tcgatgaggg aatattacgc ccacctgatc 31440
cagaagtaaa gcacttttgt aaattacttc acgaacattt tcttggcctt ctgataagac 31500
gaagaagtca tcaggcgctc gatgacgtcc atgtcagaaa ccagatcgtc cagaaggacg 31560
ttgcgccagg tagcgaaccg gccgagcgag aagatgccag cctcatgggt gagattccag 31620

atcatggatt cgcgctcgtc gcgaccgagc ggaatgattt tgcctttggt ctggatggtc 31680
ggctcgccat cttcgatgag atgccttttc ctgatgccga aggccgcgca aacgtaatcc 31740
atatcccagt cgctgtccca ttcgatggtt tcgattttac catctaaagt ctccactatc 31800
cccttagtga tggattcgac gatcagagta tcaccggtga tggacgctcg aaacgttccc 31860
actttaggac cagggaaata cacggtctgg aagacatcac aggggatgga aagcttgtat 31920
cgactcacga tgatggaggt gccttcaccg aatgacgggt cgattcccag gtccagccct 31980
gccgcagcca gattggcgcg gaacggcgcg gtgctgatga tattcacgtg gtcatcttgc 32040
cggcgaagat actggaagaa agaggcgtcg aaaggacggc tccaagtgat acgattcgca 32100
agcttagcca ccagctgctc atagtagtcg gccggtgcga tccaacgctt ttcagtcgcc 32160
agattccaga tggaccggtc cgacaggccg cccgttactt tcctggagta catgttgcag 32220
tggtcgatac gcggctggga aatgaactcg ccgtcgatgt agatagcctt gtgtacggtg 32280
acttcgcgga atgggatgcc ggtgagctgg ccgatgactg gcgagcggaa tcgcagaagt 32340
gcgttgtggc gctccttatt ctccggcgtc gccgcgtcga tgatttgggc ttgaggaaag 32400
cgatgcgcgg cgatcagtcc ggcgagtccg gcgcccacga tgataacttt ctgatcagga 32460
atcatgatgt gtcccttatg agtgtacaaa acttgagagg ataaaaaagg gacccatttt 32520
catgagtccc ttgaagagct agacgattca ggctcagagg agcggcggct tactcctcct 32580
caccttcgga accatcggcg ccctgatctt caccgtcgtg ctcctggcct tcgtcatcgg 32640
ccttctcgtc atcgccctgg ccagcttcgt cttccttcga agcgatggca accagatcga 32700
cccagcccat gatttccagc ttgctcaggt agctgcggac agaggtgcca tagagcagat 32760
gggccacctt ctcgccgaag gcttcgattt cgaccggctc accaacggtg cagtgctcgt 32820
tgatgtaagc gaacaccttg ccgcgagtcg agaaggcctg cggagtgcca tggccgtcgc 32880
cggtcgggat gaagtgggtg gcgcgggggc gacgcgagcc attggacttc aggtcttcgc 32940
ggcgggcttc ggccttctta cggcgctctt cctgctcttc cttgcggcgc tgcttctcgg 33000
cttcgcgctc ggccttcttc tgctcggcca ggcgcttgcg ctcttcttcg cgagcgactt 33060
tctgagcttc ctgggcggcc ttcttctctt cggccttttt ggcgcgctcg gcttccttct 33120
cggccttctt ctgctcgcgc tcggcttcct tggccttcgc cttctcggcc tgctcggctt 33180
ccttcaactt ggccttttca gcacgctcgg cctccttggc ggcggccttt tccttcgcct 33240
tctcggcgcg ctctgcttcc ttcttctcac gctcggcctt gcgcttctct tcgcgctcgg 33300
ctttcttctg ttcggcttcc ttggccttgg cctcggcctt ctcggcgcgc tcgcgctctt 33360
tacgttgacg ctcggcggcc ttctcggcct tgcgcagggc ggctgcttgt tccttggtca 33420
gctcttcgcc ttgggtctgt tcgttctggt ccatgttctt actccaggaa tgtttgaaat 33480
gatggcttat tggcctgtga gaggattatc tctaaactaa ttgaagaagg gaataccttt 33540
cgcctgaact tctctaaata ttttttcttt cgggaaagtc cagactccag ggaacttatt 33600
tatgttagcg aagtttttaa ctcttacgca aaaacaacaa gtattcaatt gcgcgagtta 33660

tcccagtata catcaactga ctataaggga tggacggcaa gttttcttcc aacatggcga 33720
cccgtttcca ttccgatccc tgcgacttat ggaacgtcat cgcccagccg aagtcgaatc 33780
cgccaatggc cttctgtgcc tccagccgca catcttcctc aaccgaaaag ctaagaggat 33840
tgaacttcac ccagcgctca tagttcgtgc cgataatgcg aactttggca aacagcattt 33900
catccggctc gtcatcatct tcttggcctt cggggaccgg cttgaagtcc agcagaattg 33960
cttgctcgcc gttcatgatg ccatattcgt gctggttccc ggtgcacacc agcttctcgc 34020
cgatttccgg ttgtacaccc ttgtagccga ggatgcggcg cgccctggcg ttcaaacggc 34080
ggcgagtatt gttgtaggcg caaaggatca cgccatcatc gtccaagaac gtccgcattt 34140
cgtcgtccga catgtcgaat ccggcccgta ccaggatgtc gtcatactcg cggcagggta 34200
ggcgcttgcc ctggcggacg aacatcgacg cccgaacgat attgccggcg ttgcgctcga 34260
tttcggtcat gatggtgtca cagctgttct catggaaaat ctggacgccg cgcacaggag 34320
gaacttggcc aaagtcacca atctccagaa ccggaatccg gtgcgacagc aagcgctctt 34380
catcccactc gccgatcatg gacgactcgt cgagcacgac caacttcggc ttctcgtcga 34440
gcgagtcttt gttggcaaac atgatttcgc cgtcttcatc ttcaccaatc ggccgataga 34500
tgaagctgtg gagggtccgg gcattggtgc agcccttctc gcgaagccgc gctgctgctt 34560
tcccggttgg cgcgacgaag actgtccagt ccatcgagca gcaaagttcg gcgattatct 34620
tcgcgatgga agtcttacca gttccggcga aaccagcgag tcgatagacc tggcggcggt 34680
gcgctcgatc acaccaaccg cgataccagt ccacaacgga attgatcgcg tcgatctgct 34740
gactgtttgg tcgaaagccg aatcgctctt cgatctgatc gacggtgaag ttagatgctg 34800
acatatttgc gttctccaac gctaggttta attgaatcga ggctcagttt aagcacgccg 34860
tccacagacc agccagtatc acgacgatac ttgcggcctt gcggatcgac atagaagttc 34920
ttcgtgcgcc gcagcagaac ataatgccaa gcagctccaa gcgcgtggac ccttccttta 34980
tacgggaaag ccttcgcctg ctctgcggcc tctttggccc cagggagcca gcggacggtc 35040
gaaaggacca ggaccgcacc cttaatagcc tgggaagcgg cgcggccgtc ctttgggcta 35100
tagcgatgtc tatcgaagtc tacccagtgg tggttgccgc gccggagctt gaccgttcgg 35160
gatcgcccct cgaacaccac agtgccttcg tgagttaaaa tatgttccgc catcgaatgt 35220
tcctttataa cgtacagtta tgctttacct ctgcgcagga agagtatact atcagctgac 35280
tcgccaaagc gagcgaattt aatccaactt tacttcggca ggaaagtggc cgatactagc 35340
gccgccgcct gtactgccct ccaaaacaga ggatacatta aatgcaagaa tgcaagattt 35400
accgcgacca actcccggtc ggtaacccga atcccaatgt cgacaagacc cgcgacccga 35460
accttaagcc cggtttcctg cgtcgcagtc gcgagctgga cccggcgctg gccgttcgca 35520
tccgtcgcga gctgatccac gccgaagcat ccgacttggc caaggccgga tgggtcaatt 35580
cacagtccag cctttatgga tcgaaagcct tcccgcgcca ttccgtcgtt cgcgtgaccg 35640
gagttccgga agatggagct ttcatcggca tgctgatcgg cttcatcgag catcgcgagc 35700

atggcgaatg ggcggtcatg gaagccggaa cgaaagaagg cggcgccgtc attattccag 35760
tcgatcacat catgcgagcg tcattcgccg aagccgaaga gttctctgaa aagtgggagc 35820
ggaacctggg atggcgtctg ctgcgccagc tccgcgagtg cggcgccctt gccgggactg 35880
aagacgagtt tctgcggcgg atcatcaatc gatacgttcg tgatcgcacg atcctcgatc 35940
accacaaagt cggcgcggac aaaatctaca ctgatgcagt gctcaaaagc atcggcgaaa 36000
catggccgaa gattccttcg gggaaattcg tcggacaccg agtcgcgcag ctcctgatcg 36060
gccacaagct aggtcgagcg gggaccatcc tgaatgacct ggtggacttc ttggagaagt 36120
tcgcggccgg gcgcgataaa gttctcaaca tcgccatctg taattgaggt gaatggcatg 36180
tataataaac cgactttgaa tcaccaccat caaaccgcat tgttatatct gtataacaat 36240
cccgatcagc cggccttcac agaccccaac aatcaggcgc taaatgaact tcggcagatg 36300
gggtatgtaa aggcgaaaaa attcgaaaac tgggcaggca ccggccatct taggatggaa 36360
tggacgctca ccaaggccgg gattgagcgc gtcgaagtcg ggtttctggg gaagtgcgcc 36420
gcttgcaagg gaatcggtca gacgttgctt cgggggaagt gtactgtttg caatggccgc 36480
ggccaggggt ggatcagcga atggtcgcag aagccaatcg aagataatcc tcaaatcgtc 36540
ccaaagttcg aaaagacaga tgcgaatcgg ctagcagacg ccattgaaga aatcgcccgg 36600
ctggaaaagg cactggccga atccgaaaag cgcgggagcg aactggccgc gagctattgc 36660
gatggcgtga tcggcgatga atacggccat cctcattgtc gttataaagt ggagcgcgac 36720
gccgccctgg ccgaagtcga gcgcctgcga gaatcaaaag gcgatccctc tggcagcttc 36780
gacagatgta tgaagatgat gtacgagcga gacgagaaag caaaacagct ggaagtcgcc 36840
ctggccaggg tcgcggagct ggaaacggct ctggcaccat tcgcagcggt tgcccaacca 36900
cagccgtgca cgcaaccaca ggcccatcct gctcgctgtg gttgcgagcg gtaagtgcca 36960
agtaaggagt tcatgtaatg gaacagaaga aaccttcacc agtagatgga gtcatcatga 37020
ccagcctcga cgttctccgg aaagcgaagc ccgaagcaca ggacgagtat gccgtgtcca 37080
tgttcgcaac ggcgattcgc cagaaactgc aacgctcccg cgataaaggc cgaggcggct 37140
ggatcgactg cgacgaagat attctgatca acggattcgc cgaacatgcg ctgaagggaa 37200
atgagaacaa cctcttggac ctggcgacgt tcctgatgtt catgtgggtg cgcggcatcg 37260
atgatgcgaa gattcccccg gcgctcgaaa aggcgacaca gcacaagatc atggaagcct 37320
ggagtcgaat ccatgaagat ggcctaaact ccgccagaaa ggcgagtgct gcgcgacagt 37380
tcgtggaagt gcctcgacgc aaggggcgcc cggagcgact tgcatgaagc ctcacgaaat 37440
aagactggca caggccgaag agttcctgcg cgaactcggc cgagggattc cggacgacga 37500
acgggtaatg gtcggctacg ctgaagaggc cacagtccaa accgacgaga acggccgcaa 37560
gctcaacgcc ggctggtggc ccgtgccctg gaaggaaggc aagtacatca attccagatc 37620
caacgcatat gcctgtatct cgtcgtccat caagacgccc aacccgaaga ccggccagat 37680
gcgatactgg cgcggcgagg cctctttcgg ccacggcctg gcgttaatgg tcgatgacat 37740

cggctccggc aaagggtcca agggcgactt cgaccgcgac gagttccgcg agcgcctaga 37800
gccgaccgcg attgtggaga cttcgccgaa caactaccag ttctggtatt tcttcaaaga 37860
gccgatgtcc cacatgctcc agttcaaggc gctgctctat tcgttcgtgg accaggtgct 37920
aaagaaaggc ggcgacaaca ccgtcaagga cgtcagccgt tacggccgga tgccattcgg 37980
cttcaacaac aagcgcgggg aagacggcaa gttcaaatat gccgacgaaa acggcaagcc 38040
cgaactcgtg cgactgttca gtgccgacta ttccaagcgc tactcgccag aagagatcgc 38100
ccaggcattc ggcgtccgca tcatcatgcc acagatgaag aaggtggaga taaaccgcga 38160
cgattggttg tatgaccaag tatggttgaa gtatgccgag cacatctgca cgaaatacaa 38220
gatgggagaa gcggcaggcg gccaggtcca gcagaatatg tccggcaaat accgcatccg 38280
ctgtccctgg ggcgacgagc acaccaacgg cgatcctttc ggcgcatact ttcgcggacc 38340
gatacctggc gccgagcacg aatatgtgtt cggatgcggg cacgatactt gccgcaaaga 38400
gcatcgacgg acatgggcgg ccttcaccga tgaagtcgtc ctgccctata tcgtagaaca 38460
attggaacgc attaaccgcc gtcacatcgg tgaggagtag acaatatgca aaacgatcct 38520
ggaatcctga tcacagccat tggcttgctg ttcctcggcc ttatcatctt cttcgaaggc 38580
ctaaaggggt ggaaaataca agtcgcaaac ttcctcgcgt cgcttctgtg ctttttcttc 38640
ggcctttctg ctttgacgtt ctggttcgtc gttgcgtttg acgtatttta atcgacgaac 38700
ggtacagaaa ttttcggatg gggacggaac ttattagcta tgccggttta ggtaggagat 38760
aatagccgtc cctttcgcct caatatgtag aggcaatgtt gaatccgatc atgtaaagca 38820
gaaggcggca aacctaacat gattatcgac gaagataata tttttgatga tggcgaatca 38880
gggtccagtg agtttgatct cacacagata gaagatgctg gaatggaccc tttgatgacc 38940
gccgcgagca aggcggccga tgatgcgatt gcgaggaatg agacgtaccg agcacaaaag 39000
gcggcaaagt atgccgaggc gtatgcggaa ccagatctga agaagcgagc gcgattgttg 39060
atgctcgatc aggcctttga tcttccggtc agccggctgg tgaaaggacc gttcgacgac 39120
ttcatcacaa agtacagctc tacttcagac agcaactacc tcgcggtcta tgatactttg 39180
ttctgtaagg gcgacggaac tgtcccacac ccgcacttcg acgagtttcg cggccggctg 39240
gtggaccatc gcggcgtggc gttcaacaac aagaccctcg atccgattga cctgatgggc 39300
gccctcgcgg ctgcggcctt ggacgatccc tcgattaaga agacgattga gacttgctgc 39360
gtttgggcgc gtcgatatcg ccgcaactcg ctgatcgaga cgtttgagaa gaagataccg 39420
gattgggacg gcgaagagcg gatcgaaacg ctgctgatcg acttgttcaa accgttcgac 39480
accgagctta accggatggt gagcaagtat ttctggctga gcttgtactg ccgcatcaac 39540
taccctggaa tctcggcgcc gatttcgttg gcgttgattg gtgggcagga tgcggggaaa 39600
tcatatttcg gcttgctgat ctgtaaggaa ctgtccggtg ggcgcgatct ggctccagtt 39660
cagctcgacc tgagccgaca cgaccagacg ccgttcctgc gcaacatcac cggcaactcg 39720
gttattgcga acgtcggcga aatgtccggc ttcaaaaagg gcgacatgga acgcatcaaa 39780

gaattcctgg tgcgatcttc ggatacattc gatcagaagt ttgagcctgg cgaaacgatc 39840
aagcgacaat ggatcaccat catggacggc aacggatacg atggactcca gcgggacgac 39900
tccggcaacc gtcgattcta tccgatgttc gtggcacaac ttcccgacga agatggaaag 39960
ccgaactggg ttaagccggg cgatggtaat gaaccgttca aggtggactt caccgacttc 40020
ggccgcaaat tctggcaagc gatggctgaa tgccgcgcat ggatcgaaga gcatggtgtc 40080
gatggatacc tggatatggt gtcggaagcg aaccgcgaag tgcagaagtt ctctatttcg 40140
gaaatggaga atgcgcgtgg cgtggttcgt gatgacacga ttgatatgta tctgatcaac 40200
gtgctgatca gttgcgagtt cgaagagatc aagcctggca ggaattcaaa gaatcctgga 40260
tggagagttg acactgtagc cattctgaaa tggttcgata ttctcgccag gaagaagccg 40320
atttctcgcc acttaactcc gcacctgaag gcgctggggt tcgtcccgaa caagaacggc 40380
ctggctggtt ggtgcctgcc cgtcgataag gttgcgcctg gatggacgaa tagtatgcaa 40440
accacgctgc cgccgttcaa tgatgcgctt gtgtacttgc tgaggaaggg cgacccggat 40500
atcactgatg aggccgccat ggcaaagatt cgggcagtcc gggccgagcg agccaagatt 40560
ttgggcgagg atttctgatg tagtggttga gtgtacttgg actaggccgc cttcgggcgg 40620
tcttttcttt ggtgcggcgt acattagatt ccggcgttgg gcgggattgg tagaagtgaa 40680
tacagtaatt tggaccagtt tcgagcgcga ataatggaaa gtcgatagat tttcggtatc 40740
ggtttagcgt gtatggtgga tggattttgc gtgaaatgtt gagaaattgc gtgtttgagg 40800
tggacttttc gtgggaaata gccgcaaatt actgtatagc gattctgagc ggtaatttgg 40860
gaagcctact gccgcgtggg ttagcgggcc aatttcctaa tttccggttt ttcgagcatg 40920
ggttaaaact attctacagc gaaaatcgat tgcacaatcc taatagaaaa aatctatcac 40980
ggacgttacc tatctttaaa attaataaaa ttaatagtaa tttggtaatt tggtaaactt 41040
tagtatttga aagcctcgcg gcactaagct tgtacatatc ccgtcaagtt tccgattcca 41100
gctagctcgc ggcagggtcg ccggaaactt ccggacttac aatccatggg tcgcggcaac 41160
accacggcgg actaagcggc aagtgccaaa actcgacgaa cggaaccgga aatttgggag 41220
cgcgacagaa tagctcagct ggacatattt ctaacattcg atttaacact caatccaaac 41280
actcaccacc atcgtctccc accaacagcc gactcgaccc tcacccacca gcagaccgcc 41340
catataacat cctataacac cacctaacac tcattcacca tcaaacccac ccagacctac 41400
agcccaccca caagcagccc atagacgcga tccctggccc catagtacaa tcgcgccata 41460
ctcagtgtcg cggcagagca ccaggggcca tccgccaacc aagccaccac gacgactcca 41520
gaatcgaact ccagggacgc agcaacaaat gaccgccaaa ttctacagcc ccgacgattt 41580
agtcacgcca caggaattcg cagacccgca tttcgcggcg atcaaccaaa agcgattcga 41640
cctgtacatc gacctgcgcg tccaaggtta cagctcctgg cgggtcttcc gggccatctg 41700
gggcgaggag catatggacg gtcccgcgca ggcccgcatc ttcgcgatgg agtccaaccc 41760
gtactatcgc aagcagttca aggccaagtt gaacgcgacc aaaacgtccg atttgtggaa 41820

tccaaagacg gcgctccacg aactcctcca gatggttcgt gaccccaccg tcaaggactc 41880
cagccgtctg tcggccatca aggaattgaa cgttttggct gaaatcacgt tcgtggacga 41940
gtcgggcaag accaggatcg gtcgcggcct ggccgacttc tacgcatcag aagccgaggc 42000
tcagaccgcg accgtcgctg ccgcggccga agccaatggc tatgttccgg aaggcgaaga 42060
gggcgatttc ccgtccccga cgccggaacc gaccgaggaa gatcgcgcca accccattta 42120
gacataaaat aacatcgttc taggcccgaa tcggaccgaa ctaaggcgac ggtagcggga 42180
aggaacgaaa aaacgattct agggcggttc taggaagtcg tagcctaacc ctcagaaacg 42240
acaaagcccc cggactctag ctcagaatcc gggggctttc ttttgggcgc cttattcgcc 42300
agcgtcgatg atttcgaagt tgtatttgac gccttcgtgc tcgaaagtca tcttgccagc 42360
cgccttcagc tgcatgcgga agcggatgtg tttcgaagaa ggcaggccga actcgatgaa 42420
tgctgcgttg gtggaccgga actcgccgcg cttgcctttg acggttacag ccacgccatg 42480
gcgctgggtg cgtttcttgg aaacttccgg gtctttccag gagttggcga tggctgccga 42540
caggtctttg gtctctttgg ctttctccgg cgcgttcttg gcctcttcgc gcatcttccg 42600
gatttcttcc agcgcttcct cttcggtgat ctcttcttcc ggcttcagat tctcttcctc 42660
gaccttctct tcttccttct tcttggaagt gcgggttttg taaaccttgg ccggggcttc 42720
ctcttcctgg aaggcttctt cctcggccgg cagagcattc agaatggcga ggcagcgacg 42780
ctcagcagtc ttgcggtcag agaaacgctt gacagtcgca tcggcgttgt gggcgttgta 42840
gaaggcgacc agttctttca tttctgcgtt ctggatgtcg ccgaaggttt tgatggagtt 42900
ggtcattttg cgatcctctg ttttggaaga tttcgttcgg gcttcggttt gtcgccccgt 42960
tgaaagagat tatgcctagg tcgatgctgc gtgtctacat ttatttcatc agattgatcg 43020
caaagccgac gaacggttgt ctgatgtgaa aacaccgcag agcaggctgc ggtgtttggt 43080
gggcgagggc gatggtcaga aagtcgggac tgtgatcggc tcgattggcg ggtcgccggg 43140
cttgtcgttg tccgatgcgg gagctggact ggacgggatc gcgccggacc gaagcgacag 43200
agattccgac tgcctgctca cctgcggagc tggaggagct ggaggcgctt ttggcgcatg 43260
aggcgcgggc ggaaccggca ggcctggagg agccatcggc gccctgggcg gaaccggaag 43320
atcgggcaga tcaggaagac tcggcgctat gccgccaaag gaccgctgcg actgctgcgc 43380
cggattgtcg ccgaacattt cggcggatcg gttgtccttg atgacttgac ctatggccgt 43440
cttgtcgccg cccggctctg ccggatcgaa gcccgtgaat ggagtggtaa cgcagcgcat 43500
gttcggatgg gtaggcagat ttttcagaac tatggaatct gcccagacat acggattctt 43560
gtggttgccg ggtccgtggc tgatgatctc ggcaggaata tggccgttca tggtaagcca 43620
ttccgccatc ccttgccgag tcagcgggct taccacgatc tgaccagtgg actctttggc 43680
gatggcgaac agatcaccga attcgaatcc aatgattgtg tagttggacc tggactcgcc 43740
gacgaaagcc agagtttcga tgttgtggtg gatatcttgt tcttcccaga tgtaaagcat 43800
gatgcgctcc tcaataaggc tgcttgttgt attcgaccag agacataccg gccggaactt 43860

tgaagcgtat gtattcgatc atcatcgcag tgcgacgccg attcgtgcca gactcgtcgt 43920
tgacgaatag ttcgcactgg tgcggaatga ctcgcgtcac caggccttcg cctttcgagc 43980
cgtagaactt gatgtaggcc gcgacgcctc gcgtcatgtt cagatggatc gtttcgcaga 44040
gtcgctgcgc ggccgacgcg ctggacagat cagtcgggct gaccgtcacc cagtaatgtg 44100
cgttttgttc tttatcggac atcatctgcc ctccattgat aaagcctctg ccgagtagca 44160
gaggctggtt ttaatcgcgc ttcatcagga cgactttatc atatgctcga tacttcccgc 44220
gccagtcttc ccagtaatcg ccggccgggc aagtcagctc cagagtgatt tcgtctcgcc 44280
agcattcgac ggaaaggaca atcgccttgt tcctggctgc ggaatccgct ctgagcttaa 44340
tcagaatttc gtcgcccgtt ttcagctcat caactcttac aactttggcc atgttgttct 44400
cctattgggt agcgccacga gggggccatg tcgcttcgat gtcctgtaag cagatggtgg 44460
gatagtctct ggccagcttg agcttgaagt ccatccagtc ggcaccgatg agtgccgcga 44520
cttcgatgat gacttcgttg tgcgacgccg cgctagattg caggactata gcccgacgcc 44580
gaacgggatc attgacatcg acatgagtta gtatttcgtc gccgacgagc aactcacaag 44640
ccatcaatat tttggccatc atacactcct atgagaaggc gcggccagga cgatcccagc 44700
cgcgccgatg gattaacgtt tgtgaaggat ggacactgcg tccacgtcga ggatgctgat 44760
ggtacggcga cggcgcggat tgctgcgttc atggatgcga atcaggttcg cagctggagc 44820
tttgtccacc acccatacat cttccggctt ctgcttccgg agcaggaccg tcacttctgc 44880
gttctgggcg aggccgttgc agatgaacgt gaacttggac gagggagttc tgtacatgtc 44940
gagattcctt tttggacttc gggtccggct tttcagtcgg tgaagagatt atgcctgatc 45000
atcaccgtcg agtaaagcac ttatggacca attctccagt taaatggaac caaagcgcgg 45060
tatcgctgat cgctacgctt ccacgccatg gcccttcttg ctcacagact tcgaaccaca 45120
agctaatttc catcctggcc aggactcggc cgaaggattc gagccgacgc cggctggcca 45180
ggatgtcgac attcacccgt cgattaattt cgtatcggcg accgttgatg acgaagacta 45240
ggcgtagggt ctgtccgtcc agacattccc agtatttggt ttcataccga agccagtagc 45300
gcttgccagc cgggcctacc atggtcattt aatcgatgct cctggccgcg ccacgcggtc 45360
ctgtcggcgg cgcaggatcg aaccgcccca ggatgtagtc gggccggcgc gcttcctgag 45420
gacacacggc gagaaggcgc catcctgcgt ccagggcata ctggagttcg tccgtgcagc 45480
agtcttcctt gagcagcaga cggttgacgc tctggagatt cggaccaggg atggccgagc 45540
tggtgtgcga gttccaacct tcgatgccgt ccaccatctg tggttggttg atgtagcctt 45600
cggacctgcc agccaaccgg ctcgcggcca gctccaggcg ctcaagcatg ggtcgcagag 45660
cagcttccgg gtcaacatca tcccagagca ggaccaggtt gatgatggtg tacggatagt 45720
ccttgtccag atcccaggcc gaagcggtca ggcggcccag gccgatttca ttacacatca 45780
cgggaacgtc gttgctccat gtggacggct cccagttccg ctcaccagga ttcccgattg 45840
ttacgccttc caggttcccc aggagaacgt ggagtttgct gacgtattcc gcctccagcg 45900

ctttccgctc ttcgtcggtc tggttgtggc gatagaagga tggagggctg acttttgcat 45960
ggtagagttt catggcggtt cctcggtttt tgaaggcttg aacgttagag aatggtgtcg 46020
cagtatttct cgaaaggact ctggcgcttc ttctcgcaga tcgcgcaggt gatttccagg 46080
tcgggcagct cgctgtaagt cttgccgaga tacagccagc gcttgcacag actgcggccg 46140
tccaacgtga agaagtggac tttgcgagca ttgccgggtt gcgcccagcc accttgatcg 46200
tttttgcgct tgctcatggc gatatttcct ttggatctgg aatccatgcc gtccgttcgc 46260
tcacccaaac tccgtctaca tagatcagga ctgacagagc agtcgcgcca aacccgaagc 46320
ctacatagaa tgaaccggga tcgatttcca tggtggcgct attggtggcg ctattgaaat 46380
cgacttcaaa gtcgaacatc gaatggccac tcataccatc accatgtcga tttcattgat 46440
gaagaaatga acatcgacac catcggcgcg accggtgtaa cgcagccgag tcgtcccatc 46500
aatgtgggct tcctcgacgc cgatgacttc caggatggtg tcgttgcagt aaagcttgac 46560
gaacatcttg atgccggagc caatcgccgc gaaggcgatc tgcttgtaca ggtcttgctt 46620
gatcatgctt tgcgctcctg tttgcggatg tattctgccc tggcttcggc ttcgaacttc 46680
gcccaaagcg cgttgtcgac tggaccccag ggaccggtct gagtcccgtc caccggcgcg 46740
aacttgccac cggcgcgacg atacttggac agagttctca ggctgatgat tttctcttct 46800
tgggaatcga gttggacgat cacttgacat acttcccgaa gtagcaggtg tggagtattt 46860
ccaggccttt ggagatagcc ttcagcttga tgccgaaatt cttgatcgcc cagtccagat 46920
cgtggtgata gaactcgatg atgttgacga tttggccggc gctgtagtgg atgacgcgaa 46980
ccttgccgtt ccagcggaaa tcttcgcggc tgtttacggc ctcgacttcg atggtgtcgt 47040
ttctgccgat ggtccatttg gagctagtca tgtcgcaatc ctctttttgg agtgtttcgc 47100
gtttcgatga ggtaactata cctcagtcac ctcatcgagt aaagcacttt tgaacatatt 47160
atctgaaatt tctgtgagac ggatccatcg gcggattcag tcagtggaac cgagattcca 47220
tccgttgcgg gcgaaggccg agcggccaga cagcttgcgg cactggatgg agctgccatc 47280
gtcgaaggtg taagatacgg cccagtccag gcgctcgcga cgtactgccg ccgcagcgat 47340
atcttccaga cgggctttga gaagctgttc tgctttggtc atttcgagtt cctctttggg 47400
gcgattcgtg tttcgatgag atgactataa ctcagtcacc tcatcgagta aagcgcttct 47460
gaggaattat ctgaagtttc tggaggccag aaattgccgc cagagccagt caacgtgatc 47520
gttgaggtag cgctcgccat cggacgacag agccaggcag ccatccttta tgccgagctt 47580
ggagctggaa atccattcga actcgaactg gagagttcct gggcgaggga tatgggacat 47640
gctgtacttt tgcacggtca ggaatcctct aaaggtttgg agttcgcgat ggccgcgact 47700
ttctgcatgc cgatgcgata gaattcggcc tcttccctgg ccttggccag ctgctccctg 47760
gcttcgcacg ccagcttgtg ctggttattg gcattctgga tgcttccgtc cagactcctg 47820
tccaactctt cccgaagagc cttctgcgac gctagctcgg cctccaaatc ctggattctg 47880
atctgaagct ggcgattcct ttcagaaaca tcgttttcgc gagcgatgga attctgaagg 47940

cattcgtcaa ggcgagtatt ttcgagcttc agcttgtttt cgcgatccgc gtacgcagta 48000
agacatttgg tcagacgagc ctgttctttc acaagatcga ctttgcggag cttggccctg 48060
gccagagctt tgcgaagtga ctccagctca agcgggtgaa gatcagcatc cgggtcttca 48120
cgctcttcct tcggctccat ccgcaggtag tattggccgg cagcgaaggc caatagggca 48180
cggtcggaat tctctacgga agcggccatg aagtgggaat tataacgagg cagaaacttg 48240
cccgcctctg ggtcccagca caggctatcc gccataggcc agaatttccg gaagttatct 48300
gcgttcacgc ccaccttgac cgtttccggc gcacctgaat ccgtcgccac ttccaggttc 48360
aggtgaatgg tgccggtgat gtactggaag tacactatgc tagacataac acaaactcca 48420
tgagaagtcg cggccgggac catcctggcc gcgcatcgtg attactcttc gccttcgtcc 48480
gcgctcatcc actcttcgaa ggcgaaattc actttcgact cgacccaatc ctgcagctca 48540
tcggcgaact cgtcactgtc gatatccatc ggaacgccca gagtttcttc attccacaat 48600
tcagcattca gctcgaactg gaccatttgc tcgccatcga cttccatcag gatgcggtct 48660
gcgacttcat agtcgtctac atcgaacaga tggccgccag tcaggaggtg ctggacgaaa 48720
gcttcatcga gattggtgac ttcgatctgg acttgtttgg tcattttccg tctccttact 48780
tagcggcttt gaattgggct ttgaggttgg ccagttcagc tttcagatcg atcatcgcct 48840
gaccgcgcag gccttccatt tcggcctcgc tgatcgcgat cttggctgcg cggattttgt 48900
cgttgatttg atctttagtc attttcggct tccttttgtt tgaagggttt tgcgtttcaa 48960
tgaggagatt atgccgaaag tcagaatgga agtaaagcat cttgatgaga aaatttctgc 49020
ccaccgacga acggtcgtgc tcgaccgtcg attgagggtg tgcataatcg aagtcgcacg 49080
acgcgaccgg tgatgccccg gcgaaccggg gcggggtggt taggcggtgc gctcggcggc 49140
gcgttctgcg ttacggtcgt gcgccatgcg cgcccatact gcggcccagc gcgctttatc 49200
ggcagagttc ggcttgtaca cgtcgtcagg gggcggttcc gggccgtagg cgttgcagct 49260
ttcgtcgtat tcgtaggtca tcatcttgcc ctcaaggggg tcatggcccg gcgaaccggg 49320
cgggtgatta cttgcgatat ttcttaatca ggtcgtgtgc catggcgatg gcatcagaat 49380
aggtttctcc gccagcctcc atttccttca cagccttatt gcgcagtgcg ttcagcttgt 49440
tgccgttgat ggtgcctttt tcatccttgg cagcttcgca ggagtcgcgc agagttttca 49500
gggcttggta ggtggcgttc atcgtcttgt tctccgggtt tcgcgtttcg atgaggagat 49560
tatgccgata gtcagaatgg aagtaaagtg tctgaatgaa gaaatttctg aacaccgacg 49620
aacggttgtg ctcgaccgtc tgctgcggtg tcgatatact cggcctattg cgaacaatgg 49680
actgattaaa tgttcaagct caatcctgca ctgcgagcgg tctggcgaac tcgtgcccgt 49740
tacaaagtca tctatggcgg ccgggcgtct tcgaagtcgc acgacgctgg cggtatagcc 49800
gttttcctcg cggccaacta caagctcaaa ttcctctgtg ctcgccagtt ccagaaccgc 49860
atcagcgaat ctgtctacac attgatcaaa gacaagatcg aaaactcaga gtataatggc 49920
gaattcatct tcaccaagaa ctccatcaag cacaagatta ccggctcaga gttcctgttt 49980

tatgggatcg cccgtaacct gtcggaaatc aagtccaccg aaggcatcga cattctctgg 50040
cttgaggaag ctcactacct gacccaggag caatgggaag ttatcgagcc gaccatccgg 50100
aaagaaaact ctgaaatctg gatcatcttc aaccctaacg aagtcaccga tttcgtgtat 50160
cagaacttcg tggtcaaacc cccgaaagac tcctgcgtca agatgatcaa ctggaacgaa 50220
aatccgttcc tcagtgagac gatgctcaaa gtgattcacg aagcttatga gcgcgaccgg 50280
gagcaggccg agcacattta tggcgggatt ccgaagactg gaggcgacaa atccgtcatc 50340
aatctcaagt tcattcttgc ggccatcgac gcccacaaga aactcggctg ggagccggcc 50400
ggatcgaagc gcatcggctt cgacgttgcg gacgacggcg aggatgcgaa cgcaactacg 50460
ctcatgcacg gcaacatcat catggaagtg gacgaatggg acggcctgga agatgaactg 50520
ctcaagtcgt ccagccgcgt ttacaacctg gcgaagatga aaggcgcctc ggtcacttat 50580
gactccatcg gcgtcggcgc tcatgtcggt tcgaagttcg ccgagttgaa cgatgccagc 50640
cccgacttca aacttatcta tgatccattc aacgcgggcg gcgctgtcga taagcctgat 50700
gatgtctaca tgaagctgcc gcacacgacg atcaagaaca aagaccactt cagcaacatc 50760
aaggcgcaga agtgggaaga agtcgcgacc cgattcagga agacttatga agcggttgag 50820
catggaaagg tttatccatt cgacgaattg atttcgatca actctgaaac gattcacccg 50880
gacaaactaa atcaattgtg tattgaactt tcgtcgccgc gcaaagacct ggacatgaac 50940
ggccggttca aagtcgagtc caagaaggat atgcgcgaga aacgcaagat caagtcaccg 51000
aacatcgctg actcggtgat tatgtcggcc attctgccga tccggaagcc caaaggcttc 51060
ttcgacttct aaacatagaa aagcccggat cgctccgggc ttcgggtctt actcggtgtg 51120
gttcctggcg ctgagtgtcg acgcaacggc ctcgccgact cccagggctt cctggccggc 51180
cgcgagcgct tcggcttccg actcgacgat gaaatcatca ccttggccat cgcctggcgg 51240
aacctcgacc agcacggctt cttcgccttc gaaccgcagg tcataggtct tttcgacgga 51300
cagaccgtaa cgagcgttga gcgcgtccca gagctgggcc tcataggtcc gcaggtcttg 51360
cagagctttc tggtggctga gcatggccat gtcaacagcc cgttgcaggg tttcgtccag 51420
gacgttgaat cgcatgcgaa gagaacgaat ccgctcgacc acttccgcat ccactacatg 51480
cctttcgatc atcgcttttc acctttgctg aatgttacgt tgtagccgtt gtcggccaga 51540
taggtcaagg cgccttcgaa agaagttccg acaaggtgct tgagctgcat ttcgcgttgc 51600
gcggcgatcc agaatgccgt tccggagaac tctgcgcggc cttccgacaa aacctttcca 51660
tcaggcccgt cgatccgtac atggacggaa gatagcttca gcgccatcag tgaaccgtcc 51720
ctgtgaaggc gcgcgccgcc gcgcattgat cgcaagggca ctccctctgg atgcgcgcca 51780
ggtcgtttgc atccagggcg tagagttttc catctacggt atcgtgagcc attgcgatgt 51840
ccggatattc gttttcgtcc aggccggatg ccgcacggat gttgttccag atgccggcat 51900
gttccgcgcg atggcgcgcg gtcaactcat cttgctcttt gttcgccctt tcaacgaatt 51960
tctccaggag ttcggtgaat ttctcatcga tgactcgtgc agacttgaca gagctgagtc 52020

gaatcgggtc tttcttcatg gtgattctct tttgctggtg gttgttcgct gcccagacct 52080
attcaaagtc tgggcagcat gatgaaaatg aacacgaagg ctgcaaacgc caatagcgtt 52140
cctgtaagca ttttggcttg actctgcacc tcagcgtact gcttagtgga cattccgtgc 52200
tgcgccgcct cggccgcgaa gcgagctttc gcctcgataa cttccgggcg cagcgacagg 52260
acatattcta aggcctcttc ccgcgctttt tcggcctcga ccaacctagg gtcgcgggcc 52320
gagacttcgc tgtgccctgg cctcgcggga tgggcctgca gcgatggcgg aagttcggcg 52380
gccacgactc catagtcggc gcaggcccaa gcgatcccga tgaggatcgc gaggatggac 52440
tggacgattc gcaacatcac tttgtcgcta ggaaagctca tgatcaatcc tccaccgacc 52500
gaacgatttc catgttgcga ccggcattgg cccctgcatt gaaggcgcgc cgaccgtcgc 52560
tatcgtccag acgcatgagc ttggtgacat ccgacttctt gtagccagga tcgccgaaat 52620
gttcgtgaac cgcagcttcc ttcaccacaa ccagagacgt tccggcagaa gataccagct 52680
ccatgcgctt cctggttatg gactgaaggc gatagctgat ttcctgggtc gcggccagct 52740
tgaattgcgc ggcaaccttt acgttgaaac gttcgtaccc ttgcgccttc tgatactccc 52800
gacacagacg atcaatggcc tcgaccaggg agttgaacat gttcaccgcc agctcaacgt 52860
ccgacttgta gcctttgaag cgtacggcat gaccccagcg cttggtagtg cttccgtcgc 52920
gggcgctcct ggatgcctta gccgatgctc tgtggttgtt gatgccaccg gcgaaatcca 52980
tgatgcagtc attgtacgtc gccacggcca ctgagaaaaa cttcatccag ttcgggattg 53040
cggaatagta acgagtcgca atctgctcgt cgaactcttc acgaatctcg ccggtcgctt 53100
cgaagccgtg gaggtcatac ttgtccttca gcttcttcac gcgctcggct gcgatggccg 53160
cttcgtgcgg gctggaggag tcggctgcca tggcggtcag tttgcggatg cggtctttcg 53220
ccttctcgat ggcttccggg gtgaattcgt tctggtcggt catggtcggt tccttttgtc 53280
tgaagggttt cgcatttcaa tggagctatt ctgccttcat tcagaatgga agtaaagcac 53340
tttcttccac tatttcggca tcgactggaa gaaattccag atccaatcac ctgctaccag 53400
cgagaggatg agggcggcaa agaacaagac agcggccgcg agctgtgcgc ccggcttcag 53460
tttgggatgg ctgagcttgg gctctacggg aacggccgga gcgctagcgc tgcaaccggc 53520
gtctcctggg ccgtagccga tgccgcgttc acgcgcctgg agcgacacca gggatttaag 53580
atactcggtc tgcttttgcg actcttcgta gatgccggcg acggcgaacc agagggcgaa 53640
cactacggac gtacacacaa cccaggcgcc ggtcagcagg agggccagcg ggccgatgac 53700
gaagacggta gcagccagga cgattgcgcc gccccagatg atgaagccgg ccagaccatt 53760
ggtgatgtcg atacagaact tttcattttt cagattcctt cggttacggg atggagggga 53820
tttgaaactc tgcgccgccg agaacatcaa tgacgacttc ccagagcgtc ggaacagacc 53880
actggtaacg gtcgaagtcg gtttcaggat cgacgcccag cgtcacatag gaagtggcag 53940
gccatttatg aacactgcct ttccgcatga gagtcgccac gcggccgtca ccgagcggat 54000
tcgtcctgtg cggaacgtac gcagacatcg aatattgctg cccggcgtcg acaacaaatc 54060

tttgcctctg gcgcagacat acagagccca caggattcca gccgcgaccg ccgcaagact 54120
ggcgagccga agaatgctca taaccttcgt gagtcatttc gccgaggcaa gccgagcgat 54180
cctcgtaata gttgatgttc tccatcgatc caaccagcac cagagactcg aaatcgaatc 54240
gatgatcgtg gatggccgag tggttgaagc aaagccgacg cggcagctcc gggtgccaaa 54300
catggaggcg accggccgga agttggacct gaatgaaccc caggccgtgc agagtgatct 54360
tgtccttcat cgggtcatgg acggtgctca tggataatcc tcagtagcag aaatgtattg 54420
tgagagttac gattgataag ccggtcgccc atagaagggc gaaccaggcc atggctttga 54480
tggtcgtgta gatcatccga agaattttcc ggcacagatc gggccaatgc ccatttcgat 54540
ggatgcgtgg ttggtcaact cgcgaccgca gcaggagcac tgaccagtct tccgaccgta 54600
ggcgactgcc gattccatcg gcttttcgaa catcttcaga acgtcgtcgt gctcagtatc 54660
ggtgcagtcg cgactcttga tgaatttgcc attggtgatc cggccgaggt agatgtcgcc 54720
caggacgtac aggctaccgg cgttccggct gtgagcgcta gcctctttca caacaacgat 54780
gagcggctcc tcgccttcgc cagctaggcg gattttcggg cgcttgatac cagactcttt 54840
cgccttctca aacgctttct cgatgccgga aatgtccaga gtcggcgcag cagcttcctg 54900
cgcggccact ttctcgcgat acttggcgag gttttcgatg gcgcgcttcg cagcagcgat 54960
ctgattttct gtcaaggagc cgtatttata caacgactcc tgaaggctct gagcgaagct 55020
gaaggaattt ccagtccacc actcgatgat gtccgggtgc gcggcttcga aggccttaat 55080
tttgaggtcg cgctcttgcg cagcgctaca gattttctcg atgcgctttt ctgccgcctt 55140
agcacggctc ttggcgcgct gctccgggct ggttttgtac tctttgtatc cgacgccgcc 55200
gcaggcaaag caggcgcgac cataagacga agggccacgg tacaggccgg tgcctgcgca 55260
tttggtgcac ttgtcgcgat acagcttcac ttccttccgg gagttcgggc gggcacccat 55320
ggacacgtct tccagggtct tcggcgcttc gttgttgatc tctacggtag cgaagtcatc 55380
acccaggtct tcgaagccgg tgaacagatt ctctgctgcg ttcatgtcga ttctcctgtt 55440
tggaaagttc gtttcgatga gttgactata cgccagaagt ggaaaaacgg tagcgatttc 55500
tcactaccgt tcgtcgggtc gaagacaatc aataaatgta gctattgatc ttgaacccat 55560
gctcagcgcc gtcgttgtag tcgtactcaa cataactgtc gcagtagtcg ttcaaatgct 55620
gaatgatgcc tagaacgtcg ttcgcggcct tgtgcctttt cgcggcgatg gtcttggcga 55680
tgctgtcgcc gacttccagg gtttcggccg tccggcgatg gatcagcagg cgactccaaa 55740
gatagagccg gacgcgacga atgatgtcgc gatggcgctc cagtcgtccg tgcaactctt 55800
cgatttcgtg ctcgcgaaat tttaatactc gtcgaagctg ttgaacttcc aactccaaat 55860
cggccttagt agccatgttc acctcagaaa ggataatcgt ctgagactcc aggaagctcg 55920
acgattattg ttgagcctga tcggtctaga atgcatccga ccgagccagc cctgtgtgga 55980
tagggtctac acgaactata acaggtgact tccctgacta ttcgccaggt cgatcctcca 56040
caccatctgc aaattggcgg gcgcttcgac ttcagttttc gttcgtccag gacggcgcgt 56100

ctgtcgcagg aaaggcagcg tgccttaacc gacgtaacca tgggtatagt cgatcattcg 56160
gatgagctca tcatcctctg attctctcga cttcagaccg cccaaagagt ccgactggcc 56220
gtcgcatttc gcatcgatat ccatcgaaga caggaatcct gccgatactg cggtgatatc 56280
aactttgtcg ccggccttct tgaagccttc gcactcagca tcgagggcca caattgcact 56340
cgcggccatg ttcacatgac tgatgaggtc ggggaagatc acaggaactt cacgcgacat 56400
gccacggacc gtcagcttca ggactacata cttcatactc actatccctt ttgtatgtga 56460
ggaaagaact tgctgttttc cggatggtga aaacgctcgg tcgcaggcgg tctttcttcc 56520
ggacattgaa tcgtcgaagg cgggaaaacc gcgccggcga taatcgccgc gaggagtgca 56580
gcggatgtcg acatccagag ggcggtttcc aggctcaccc gaacttccgg acggcgcggc 56640
ttcattcgct ccaccctctt cctggctcat aggatggcag accgccgccg cgaatccaat 56700
tgtcccacat acggttcaga ccagtcggtg gctggggcgg cggactgtag aatccgggag 56760
tctcagtgct gctcaggaaa taaggcgtgc aaacggcctg gacgaccaat tggtgccgct 56820
cccacatctt atcaatcgct cttagcatga cgtcttcgca ctgagcctta ctgtcgaacc 56880
gtctgctagt atggtccggc atctggacgc agccgtctcc agtgcaaagg aaagcagtag 56940
caatccatac ggtgatactc gccatttcgt caccctcttt agttgatgag cagagtctat 57000
tccatctgct cgccaggagt aaagcgcttt tcgtcgggat aaatgccgat gatgtctgcg 57060
tcgagcatcc aaatctccac ggacggatcg tcgctgctga tttgatatag gttgtacagc 57120
tcccgtccgc cgacagcacg ctcgcctcgt ggctcaaccg ccagaacctt gccgtgtcct 57180
tctccgtgct cgtccaggta tatgacgtga tcaccgactt catagctttc tttcgtaacg 57240
aggcgtgaac gcgagctgtt ctgcgattcc accatccagg aatccagcac gccgcctttc 57300
atgtcgcgaa acaatcttgc cgatttgtcg caatcgaata cgcccagaac ttcgccgtct 57360
ttcaacacga tatgtacgat tggcaaaaga gtattcatgt ttaatctcca ttggttgata 57420
attagagtct aatctgccga aaagttcccg taaagaatta ttttctcata actgattagt 57480
tgcaactgtt aatctgatgt atatgtttga atctcttttg aacgtttgat gtttccccta 57540
taataagcgc acacagccaa caaccacgtg gaactacaat gtttaaactt tcctggatat 57600
tcgggcgcaa aaaggataat gctgcctgtt ctgaatcggc gccggagaaa gtcgcacaaa 57660
tccctcaaca cgatccgctc gaccccatga taaagctggg aaggattcgc ggctggaacg 57720
tcgagccgga gaaagccccg gtcattcgta gtgtgaaaga tttcctggag ccgggcctat 57780
ctgtcgcaat ggatagtgcg tatggtgatg gaccaacccc ggccgcgaag gctgctgcgg 57840
gcggccagaa tccctatgtc gtcccgacta tgttgcagga ctggtacaat tcccaagggt 57900
tcatcggata ccaagcttgc gcaatcattt cccaacactg gttggtggac aaagcttgtt 57960
ccatgtctgg ggaagacgca gcacggaacg gatgggaact caaatcggac ggcaggaagc 58020
tatccgatga acaaagcgcg ctgatcgccc ggcgcgacat ggagtttcgc gtcaaagaca 58080
acctcgtcga actcaacaga ttcaagaacg ttttcggcgt tcgcatcgcg ctgttcgttg 58140

tggagtctga cgatccggac tactacgaaa aaccgttcaa tccggatgga atcacgcccg 58200
gctcctacaa gggaatctcc cagatcgatc catactgggc gatgccgcag ctcactgccg 58260
gctcgacggc cgatccgtct tccgaacatt tctatgagcc ggatttctgg attatcagcg 58320
ggaagaaata tcatcgcagc catctagtag tcgttcgcgg accgcagccg ccagacatcc 58380
tgaagcctac atacatcttc ggcggtatcc cgctcaccca gagaatctat gagcgcgtgt 58440
atgcggcgga acggacggcg aacgaagccc cgctgcttgc catgtcgaag cgaaccagca 58500
ccattcacgt tgacgtggaa aaggccatcg cgaatgaaga cgctttcaac gctcgcctgg 58560
cgttctggat tgccaatcgc gataaccacg gcgtgaaagt cttgggtact gatgaaagca 58620
tggagcagtt cgacacgaac cttgccgact tcgacagcat catcatgaac caatatcagc 58680
tggtcgctgc catcgccaag actccagcta cgaagctcct cggcacttct ccaaaaggat 58740
tcaatgccac tggtgagcac gaaacgattt cttatcacga agaactggag tccattcaag 58800
agcacatatt cgacccactt cttgaacgcc actatttgct tctggcgaag tcggaagaaa 58860
tcgatgtgca gctggaaatc gtctggaacc ctgtggactc cacgtccagc cagcaacaag 58920
ccgaattgaa caataagaaa gccgcgaccg atgaaatcta catcaactct ggcgttgtgt 58980
cgccggatga agttcgcgag cgtctgcgcg acgatccgcg ttccggctac aaccgactca 59040
ccgacgatca ggcagaaacc gaaccgggca tgtctccgga aaacctggcc gaattcgaga 59100
aggccggtgc acagtcggcc aaggcgaaag gcgaagccga gcgagccgaa gcccaggcgg 59160
gcgccgtaga gggcgcaggc gccccggttc ccgccgctcc acgcgggact aagcctctcg 59220
cgaaagcggc cgaggaaggg gccagcgagg ccgctgaacc gccgtcgcgg ccggacccca 59280
aggccgagct gcggaacttg ttggtcgatc ttttatcgaa gctccaagac ctggacgaca 59340
ttaaggcgcc ggacggcgta gacatagagt acaatgatgc gcctggctta aagcgcacgt 59400
ccaaacctgg cgtgtctggc atggagcctt cggtgttttc gtccaaccgc atcgtcgggc 59460
ctcgtgatca ttcggaactc cagagaatca aggtgaatgg aataaccacc ttgatcgaaa 59520
atccgcgcgg aagcattcgg caaggaaagg atgggagttg gcgagttcag atgaaacacc 59580
actacgggtt catcaaggga acgaaaggcg ctgatgggga tgaagtcgat tgcttcgtcg 59640
gtccgaatct gggatccaaa cgggtcttcg tcgtcaacca ggtgaacaag gaaggtcagt 59700
ttgacgagca caagtgcatg ctcggcttca acaacatcaa cgatgcgaag tctggatatc 59760
tgtcctgctt ccgcccaggt tgggatggtc tcggctccat acatgaagtt gacctacccg 59820
cgttccgtcg ctggctggcg aacggcgaca caaccaaacc gttcgggggc gagtgatggc 59880
attcaaggcc tccaaaaagc gcgaacgccg ggcacctctt ccagtcggaa gagggaagcc 59940
cataattcca tcggcaggaa tcgaggcctg gtatcgaaag cagatgaagg atatgtccaa 60000
gctcatgatc gccgactatc gaagcgagat tgagaatgca ctgtcccagc ctgcggccga 60060
acggttcttc gccagcgacg aatccgttaa cgttctgttc aagatgaccc ttcgaagcct 60120
acagcagcga tggagccgca tttttgaagg cttcgcggcc aagatcgccc cggagtttgt 60180

taaccggacc gaagaagcgg ccaccgccgc gaccctgcac agcttgtctg tggccggcgt 60240
cgatcagcca agagctgcgt acaatgagag cgtcaggaat accctggagg cagcaactac 60300
ttacaaccat actctcatca ccaggattca agaggaagtc cacgagaaga tttacacatc 60360
agtaatgttg tctctgactt ccccaaatcc ggaagagcaa ggaacttccg gcataacgaa 60420
cgcacttcgc aaagtcggga agttctctga agatcgaatc gaactcatcg caagagatca 60480
aaccagcaag ctttacagtt ctctgagcga tgagagaatg gcggaaaatg gagtcgagga 60540
attcgagtgg atgcactctt ctgccggcaa gactcctcgc catacccacc tggagaaaga 60600
tgggaaaagg ttcaagctga atgaccccag actttgggag ggtccgaagg ccgaccaggg 60660
gccgccagga tgggcgatta actgtaggtg cagaaagata ccaatcattt gatatcgata 60720
ggagcgctat atgccgttag ttcatggcac ttccaatgag gctcgttctg aaaacatcaa 60780
gcgggagatc gaagccggta aagacccaaa gcaggctgcg gccatcgctt actccatcca 60840
gcgcagtgag aaagggaaga cggcgaaaga ttgttcgcct gagctcgttg ccgatcttcg 60900
cgccctggtg gacgctctgt cgaggctcgt gaaatgaacc gcaagacatg ccggcgccga 60960
ctcgtggtcg atgtaatcag ggccaatatt cacggcggat tcttcagcct gaagtttgcc 61020
gccatcgatt tggcaatcat cggcgtcgcc atcttgatga cttttggccg ataatgctga 61080
gaaaatctgg attctgacta aaaattctag tccggatagc cgcaagttac cgtttacgga 61140
aaatagcagt aatttggaaa gcctactgcc gcgaggcttt aacagagcta gttcctaatt 61200
tccgatttag ccgcgtgctt caaaagtata tagcctggga aattagaagt aacgttccaa 61260
tagaattcat ctataagtaa cgttataata taacgtcaat ctatatgctc tagacgtatt 61320
gaaattcaat ttttaattgg taaattggta atttggatta gtttaggagt tgaaagtctc 61380
gcggcagtag gcttagacaa atcccgtaaa gtttccgaga ccaaattacc ggattttcgc 61440
ggctgaggaa actggtaatt agatcataat ataaattata atgtaagtta acagtcgcgg 61500
ctacatctaa ttattgttcc gcttatttac ccttagatgt actgcgtata taatacagcc 61560
atagtccacg actcttcgaa ttaacgatga caaagtcgaa aagaaaaatt gacgaaaatg 61620
gatatatgac catcgagggc tgcccgatca gctcttatgg cgttttccag tattctgctg 61680
gtcaactcgg tcttccgggc gatccgacgc ggattgtcaa cgtatatcgc ccggagtctg 61740
ccgtcagcga tcctgagtac atcgaatctc tgaagaatct cccgttgatc gacgagcacg 61800
aaatgctgtc gggattcgac gacgatgacg acagcgtggc ccccgaagac aaaggggtgg 61860
agggcatcat cacctccaac gcttactacg aagccccatg ggctcgcggc gatatccgca 61920
tctattcccg caacatgcag aatcagctgg aaaggggcaa agaagacctg tccctaggct 61980
atagttgccg ctacactgag caacccggca tctggaacgg gacgccttat gaagtcgtcc 62040
aggacaagat gcgcggcaac cacatcgccc tggtaaaaga gggtcgtgtg ccgggggcca 62100
gagtattgga tggtctgtgt tttgaccatc tcagttttga tttcagacca tccgatgagg 62160
gtaatgaaat gagtctcaag aaagccaagc ggaagccccc tgtccagcgc gtagggcaag 62220

ctgctgactc ggcggtcgaa gagttgcgca ccctgtggcc gaaactctct gcgtctgtcc 62280
agaagttcct gggcgaagaa gagcaggagc cggagcatca ggaaggcgcc gctccggccg 62340
aaccgaccga cagcgagcac ctgaccgagc atccgactct ggaaggcgcc cagaaggacg 62400
acgaagagca ggaagaggag ccttccgttg tcgatccggc cgtggccgcc gtcgagccgg 62460
agcatcaaga aagcgccgca tccgaaatgt ccggtgaagg cgaagtcgcc gaactgatct 62520
ctcaggtcaa agccattctg gctcgactgg agggaacggt agccgaaggg gcagacgaag 62580
agcatggcga aggtcaagat gtcgtcgagg gtttggagga gcagagcagc ctcagcggct 62640
cgcaaaccgc cagcgacgat ggtggtgaga gcaaggataa cagcgaggaa cttcctgaaa 62700
tggcacagaa gaacgcgcaa gatgctgcaa ttcgcggtct ctatcgcgac attgctgcta 62760
aagatcgcct ctacaagcgt cttagctccg tggttggtgc gttcgatcac cgagctatgg 62820
actcggctga agtcgctgtt tacggcgtga aaaagctgaa catcagctgc gcgaagggcc 62880
aggaagctct ggcgctcgac atgtacctga aaggcgtcga agcctcgcgc ggcgcggcca 62940
gccgtcaatc gaaagcccag gattcggccg gttctgctcc gcagtgcgcc gagctggaca 63000
gctacctgaa gggggagtaa ctcatgttcc agaaacaagt ttaccgccag tacactcctg 63060
gttttccggg cgatctgatc gaggacggcc cgaagcgggc gcggccgggt cgaatcatgt 63120
ctctgtctgc cgtaaatccg gctgccaccg ccaccggccc caaccgcatc agtcgcgctt 63180
tcggttacgc cggtgacgtc agcgccctcg gtgaaggtca gccgaagacc atcgcggctc 63240
gtgcttctga agtcgtgatc ggcggcgcca acttcttcgg cgtcctcggc catccgaagc 63300
attatgccct gttcggttcg gccggcgact ccctggctcc cagctatgat ctgcccgatg 63360
gcgccgaagg cgagttcttc gacatggcca ccggcctggt cgtcgaaatt ttcaacggcg 63420
ccgcagccgc cctggacctg gactatggtg acctggtcgc ctatgtcccc aacaacctgg 63480
ctaccgccga caacgcgctt ggcctgccgg ccggcgccct ggttggcttc aagactggct 63540
ccatgccgac cggcttggtc cagattccca acgcacgcat cgttaacgcc atcagtctgc 63600
cggcccagtc ggcggggaat ctggttgctg gcgttaccat cgtccagctc acgcagtaag 63660
gaggcgtcat gagccagatc agcaagaccc attcgcgcct cgcaggccgc aatgcgaaac 63720
ctttcgacct gaagaacatc accaatgatg ccgtggcatc tctgcgccgc atcggactgg 63780
tattcgatca cgctgtcgtc caggaccaga tcaaggcctt ggcgaaggcc ggcgcgttcc 63840
gctccggctc ggccatggac agcaacttca ccgccccggt gaccacgccg tccatcccga 63900
ctcccatcca gttcctgcag acctggttgc ctggcttcgt gaaagtcatg accgcagcgc 63960
ggaagatcga cgaaatcatc ggcatcgata ccgttggctc ctgggacgat caagagatcg 64020
ttcaaggcat cgtggagccg gccggcactg cggtggaata cggcgaccat accaacatcc 64080
cgctgaccag ctggaatgcc aacttcgaac gccgcaccat cgttcgtggc gagctgggca 64140
tgatggtggg taccctggaa gagggtcgcg cctcggccat ccgtctgaac agcgccgaaa 64200
ccaagcgcca acaggcggcc atcggtctgg aaatcttccg caacgccatc ggtttctacg 64260

gctggcagag cggcctgggc aaccgcacct atggtttcct gaacgatccc aacctgccgg 64320
cattccagac cccgccgagc cagggctggt ccactgccga ctgggcaggc atcatcggcg 64380
acatccgcga ggccgttcgc cagctgcgta ttcagagcca agatcagatc gatccgaagg 64440
cggaaaagat cactctggcc ctggccacca gcaaggtgga ctacctgtcg gtaaccacgc 64500
cttacggcat ttcggtttcg gactggatcg aacagaccta tccgaaaatg cggatcgtgt 64560
cggctccgga attgtccggc gtccagatga aaaaccaaga gccggaagat gctctggtgc 64620
tcttcgtcga agacgtgaac gcggccgtcg atggcagcac cgatggcggc agcgtgttca 64680
gccagctggt acagagcaag ttcattaccc tgggtgtcga aaagcgggcg aagtcgtacg 64740
tggaagactt ctccaacggc accgccggtg cgctgtgcaa gcgtccgtgg gctgtggtgc 64800
gctacctcgg catctaacag atgcctattc accaaaggcc gggcttccgg cctttgttca 64860
ctctgactct aactcggttg taggggccgg ttagggcata attcctagga ctacgccaat 64920
gactgtttat atcgtttcct ccatgactca atcggtgtct tacaatgcgt atgacacctc 64980
tgatccgtcc aatcctcgcc tccagcgaaa gattctgatt cgcggccgcg caggcatcgc 65040
atccgaaact tccggcttcg gcgacatgat ttccgacgag gctgggcgtc cgatctggac 65100
cccacagggc gattgcactg ccgtgagcga ttcggatttc gagctgcttc aggccaataa 65160
gattttcatg cgtcacatgg ataagggcta tttgcgagtc gtgaagaccg acatcaccag 65220
cgaccaccag cggatttcca aagagactcg taccatggag cgcgatggat tccagcctct 65280
ggacgctact cgcctgcagc agaaaatcaa ggtgaccaca gccagcgctt cccaggaaca 65340
agagttccgg atttaaccga gggtttcggc atggtgattt tcgacgaaaa taagtttcgc 65400
acgctgtttc cggagtttgc tgatccatcc gcttatccgg acgtgcgcct gcagatgtat 65460
ttcgacattg cgtgcgaatt catttctgat cgcgattcac cataccggat tctcaacggc 65520
aaagccttgg aagcgtgcct gtatcttctg accgcccacc tcctgtcgct gtccacgatg 65580
caagttcaag gcgcggcggg aggtggcgtc acagcaggtg ggacccaagg cggtttcatc 65640
accagcgcta ctgtcggtga agttagcgtt gctaagctcg cgcccccggc caagaatggt 65700
tggcagtggt ggctgtccgg gacgccttac ggccaagagc tttgggcgct cctcagcgtc 65760
aagtctgtgg gcggattcta tatcggcggc cttccagagc gccgaggctt ccggaaggtt 65820
ggagggacgt tctggtgatc cctggcgcga atcttctgcg aatggccttc ggggtcatcg 65880
gcactcaaat tgtgagatat cgcaagtttg agcagcgagt gaagaatgat caagcccagt 65940
atgtctccat gttcggggag cctttcgacc tggcagcgtc tgtccagcga gtccgacgcg 66000
atcagtatgc ccagtttaat ctggagttcc aacggaatta tgttatgat 66049

## Description

### Field of the Invention

The present invention relates to isolated bacteriophages (phages) having lytic activity against *P. aeruginosa* strains, and the use of the isolated phages for the treatment of a *P. aeruginosa* mediated infection, for example in a mammal with cystic fibrosis. In particular, the invention relates to a cocktail of phages capable of clearing *P. aeruginosa* infection from the lung of a mammal. The invention also relates to a pharmaceutical composition for the treatment of an individual suffering from a *P. aeruginosa* infection.

### Background to the Invention

Cystic fibrosis (CF) is a common inherited chronic genetic disorder that affects mainly the lungs and digestive system (pancreas and intestine) of children and adults worldwide. CF also affects the mucus and sweat glands of the liver, sinuses, and sex organs causing progressive disability due to multisystem failure. CF is caused by mutations in the CF transmembrane conductance regulator (*Cftr*) gene. The defective gene and its protein product cause the body to produce unusually thick and sticky mucus and improper amounts of chloride-containing secretions into ducts and body cavities. This secretion clogs the lung, leading to life-threatening lung infections.

Lungs of CF patients are often colonised or infected in infancy and early childhood with *P. aeruginosa* that may damage the epithelial surface, resulting in altered airway physiology and impairment of mucocilliary clearance. Such chronic infection of lungs with *P. aeruginosa* is the main proven cause of lung function decline and ultimate mortality in CF patients. In fact, 80 to 95% of patients with CF succumb to respiratory failure brought by chronic bacterial infection and concomitant airway inflammation. Current evidence suggests one way in which *P. aeruginosa* persists in CF lungs is due to its ability to form biofilms in the lungs of patients. Another significant factor is the inherent resistance of *P. aeruginosa* to many antibiotics due to the production of exopolysaccharide. As a result the most prevalent and severe chronic lung infection in CF patients is caused by mucoid *P. aeruginosa*. Reports have shown that organisms in biofilms are able to tolerate 10-1000 fold higher antibiotics than planktonic bacteria and this often makes the antibiotic concentration needed to eradicate the biofilm above the peak serum concentration of antibiotic, rendering it ineffective in treating biofilm associated infections. The continued emergence and re-emergence of *Pseudomonas* pathogens which are resistant to one or more chemical antibiotics and have the ability to form biofilms poses a continuous challenge in the treatment of lung infections in CF patients. As a result, there is an urgent need for an alternative, non-antibiotic approach such as phage therapy.

A number of studies have investigated the development and differentiation of *Pseudomonas* biofilms *in vitro* using a variety of static and flow cell assays. Recently *Anderson et al.*, (2008) demonstrated a tissue culture model to grow GFP-tagged *P. aeruginosa* biofilms on CF derived human airway cells that promotes the formation of highly antibiotic resistant microcolonies (biofilms). A very recent study by Debarbieux *et al.* (2010) reported that the amount of light measured in lux-tagged *Pseudomonas* PAK strain-infected mice decreased to a minimum level in 6 h when phage PAK-P1 was administered 2 h after infection. However, there is very little information and understanding regarding the efficacy of phages in clearing *Pseudomonas* biofilms growing on CF lung tissue.

Bacteriophages (or phages) are viruses consisting of an outer protein capsid enclosing genetic material (single-stranded RNA, double stranded RNA, single stranded DNA, or double stranded DNA) in either a circular or linear arrangement, capable of infecting a bacterial cell, and may cause lysis to its host cell. Phages may also exist as pro-phages, which are phages that exist in a dormant state or which are even defective. In this case the genome of the phage is either integrated into that of the host bacterium, or is replicated autonomously.

Currently, there is a lack of information and understanding about the effect of phages on biofilms growing on lung tissue models. It is an object of the invention to overcome at least one of the above-referenced problems.

### Summary of the Invention

Lungs of CF patients are often colonised or infected in infancy and early childhood with organisms that may damage the epithelial surface, resulting in altered airway function and impairment of mucocilliary clearance. Such functional impairment to the lungs leads to increased risk for *P. aeruginosa* infection in CF patients. Biofilm formation by *P. aeruginosa* is considered an important factor as to why it is becoming increasingly difficult to treat CF lung infections using antibiotics. The invention is based on the discovery of phages that have lytic activity against a broad range of *Pseudomonas* isolates, including mucoid *Pseudomonas* and CF clinical isolates. This invention is also based on the surprising discovery of the clearance of *Pseudomonas* by a cocktail of phages from lungs of infected mice and biofilms growing on the surfaces of a Cystic Fibrosis Bronchial Epithelial (CFBE41o-) monolayer. p16S*lux*-tagged *P. aeruginosa* was used to monitor lung infection in mice and biofilm growth. A cocktail of phages were used to kill *Pseudomonas* in both *in vivo* and *in vitro* systems. The data presented shows that a phage cocktail of the invention can clear *P. aeruginosa* effectively from lungs of infected mice and from biofilms growing on CF epithelial cell monolayer.

Accordingly, in a first aspect, the invention relates to an isolated phage MR299-2 or phage NH-4 deposited under NCIMB Deposit Accession Nos. 41729 and 41730, respectively, said phage having lytic activity against *P. aeruginosa* strains, and variants of the isolated phage, wherein said variants retain the phenotypic characteristics of said phage and wherein said phage and variants thereof have lytic activity against *P. aeruginosa* strains.

Phages of the invention have been found to have lytic activity against a broad spectrum of *P. aeruginosa* strains, including CF clinical isolates and mucoid strains. The phages have shown utility in clearing *P. aeruginosa* effectively from lungs of infected mice and from biofilms growing on CF epithelial cell monolayer.

Suitably, isolated phage MR299-2, and variants thereof, have lytic activity against at least *P. aeruginosa* strains MR299 and NH57388A. Ideally, the isolated phage MR299-2, and/or variants thereof, have lytic activity against at least *P. aeruginosa* isolates CH001, MR299, MR325, MR326, MR327, MR330, MR331, POA1 MR299 and NH57388A (the characteristics of which are described in Table 6).

Typically, isolated phage NH-4, and/or variants thereof, have lytic activity against at least *P. aeruginosa* strains MR299 and NH57388A. Ideally, the isolated phage NH-4, and/or variants thereof, have lytic activity against at least *P. aeruginosa* isolates CH001, MR299, MR300, MR326, MR327, MR330, MR331, POA1 MR299 and NH57388A (the characteristics of which are described in Table 6).

Generally, variants of phage MR299-2 have at least >95%?? sequence homology to MR299-2 (SEQUENCE ID NO: 5). Typically, the variants will have a MW from 40kD to 50kD, suitably from 42kD to 47kD, and ideally from 44kD to 46kD.

Generally, variants of phage NH-4 have at least >95%?? sequence homology with SEQUENCE ID NO: 6. Typically, the variants will have a MW from 60kD to 70kD, suitably from 64kD to 68kD, and ideally from 66kD to 67kD.

Suitably, variants of the phage MR299-2 have the same lytic activity against *P. aeruginosa* strains as phage MR299-2. Typically, variants of the phage NH-4 have the same lytic activity against *P. aeruginosa* strains as the phage NH-4.

The invention also provides isolated progeny of the phages MR299-2 or NH-4, wherein said progeny have the same phenotypic characteristics of said phages and have the same lytic activity against *P. aeruginosa* strains.

Suitably, the isolated progeny have a RFLP profile that is substantially equivalent to a RFLP profile of the phage when prepared using the techniques described below.

In a second aspect, the invention provides a phage cocktail comprising (a) an isolated phage MR299-2 of the invention, or an isolated variant or progeny thereof, and (b) an isolated phage NH-4 of the invention, or an isolated variant or progeny thereof. Typically, the phage cocktail is capable of clearing *P. aeruginosa* infection from lungs of infected mice. Suitably, the phage cocktail is capable of clearing *P. aeruginosa* infection from biofilms growing on the surfaces of a Cystic Fibrosis Bronchial Epithelial (CFBE41o-) monolayer.

In a third aspect, the invention provides a pharmaceutical preparation comprising an isolated phage of the invention, or an isolated variant or progeny thereof, and a pharmaceutically acceptable carrier.

In a fourth aspect, the invention provides a pharmaceutical preparation comprising a phage cocktail of the invention and a pharmaceutically acceptable carrier.

In a fifth aspect, the invention provides an isolated phage of the invention, or an isolated variant or progeny thereof, or a phage cocktail of the invention, for use in the treatment or prevention of *P. aeuriginosa*-mediated infection, especially a lung infection, preferably a chronic lung infection, in a mammal, typically a mammal with an underlying disease or condition which renders them prone to contracting a *P. aeuriginosa*-mediated infection, for example a mammal with cystic fibrosis (CF). Thus, the phage of the invention, or variants or progeny thereof, or the phage cocktail of the invention, may be employed to prevent or treat CF in a mammal. In a preferred embodiment, the use of the invention involves administering the phage, or phage cocktail, of the invention to the mammal within 12, 10, 8, 6, 4, 2 or 1 hours of the establishment of the infection.

Thus, the invention relates to the use of the isolated phage, or phage cocktail, of the invention in the treatment or prevention of an acute or chronic *P. aeuriginosa*-mediated infection in a mammal. The invention relates to the use of the isolated phage, or phage cocktail, of the invention in the treatment of cystic fibrosis in a mammal.

The DNA sequence of phage MR299-2 is provided in SEQUENCE ID NO: 5. The invention also relates to an isolated DNA sequence having at least >95% sequence homology with SEQUENCE ID NO: 5. The invention also relates to a phage encoded by an isolated DNA sequence of the invention.

The DNA sequence of bacteriophage NH-4 is provided in SEQUENCE ID NO: 6. The invention also relates to an isolated DNA sequence having at least 98%, 98.5%, 98.6%, 98.7%, 98.8%, 98.9%, 99%, 99.1%, 99.2%, 99.3%, 99.4%, 99.5%, 99.6%, 99.7%, 99.8% or 99.9% sequence homology with SEQUENCE ID NO: 6. The invention also relates to a phage encoded by an isolated DNA sequence of the invention.

### Definitions

The phages of the invention should be taken to mean *P. aeruginosa* phage MR299-2 and NH-4 deposited with NCIMB, an international depositary authority recognized for the purposes of patent procedure under the Budapest Treaty, on 24 June 2010, and receiving NCIMB Deposit Accession Nos. 41729 and 41730, respectively. Said phages have lytic activity against *P. aeruginosa* strains. The phages of the invention also include variants of MR299-2 and NH-4 that retain the phenotypic characteristics of said phages MR299-2 and NH-4 (as defined below), and which have lytic activity against *P. aeruginosa* strains.

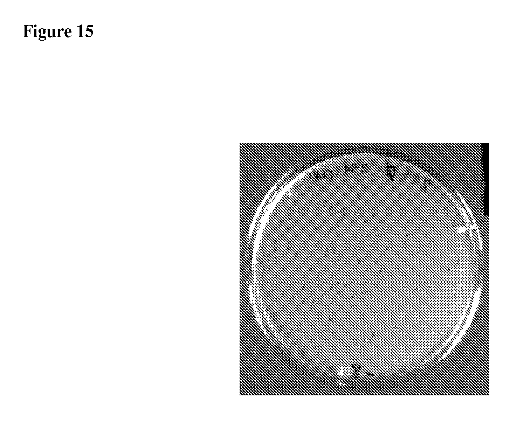

In the specification, the term "lytic activity" should be considered to mean as having activity against a target bacteria as determined by a plaque assay. An example of a plaque assay is one where phages are purified by successive single plaque isolation and propagation using Luria Broth double layer agar plates. In general, a single plaque is picked from a plate using a sterile capillary tube and added into mid log-phase *Pseudomonas* culture (10⁸ (colony forming units) cfu/ml) supplemented with 10 mM CaCl₂. The culture phage mixture is incubated at 37 °C overnight. The lysate is filter-sterilised through a 0.45-µm pore size sterile filter, serial dilutions are made and plaqued on a lawn of host culture. Single plaque isolation and plaquing process is repeated three additional times after which purified phages are obtained. Phage titer is determined as plaque forming units (pfu/ml) by plaque assay as previously described (O'Sullivan *et al.* 2001). Those phages having lytic activity against the bacteria of interest are indicated by a clear halo in the double layer agar plates (see, for example, Figure 15).

The term "variants" as applied to phages MR299-2 or NH-4 means phages that retain the phenotypic characteristics of MR299-2 or NH-4, respectively. The term should be understood to include genetically modified versions of the deposited phage in which the genetic code is manipulated by means of, for example, genetic engineering or serial passage.

In the specification, the term "phenotypic characteristics" as applied to MR299-2 should be considered to mean a PaP3-like phage having an isometric head and short tail, being attributed to species type coliphage T7 in the family *Podoviridae*, and possessing lytic activity towards *Pseudomonas* isolates MR299 and NH57388A.

In relation to phage NH-4, "phenotypic characteristics" should be considered to mean a PB1-like *Pseudomonas* phage classified in the A1 morphological group of the *Myoviridae* family of phages, and possessing lytic activity towards *Pseudomonas* isolates MR299 and NH57388A.

*Pseudomonas* strain MR299 was deposited with the NCIMB, an international depositary authority recognized for the purposes of patent procedure under the Budapest Treaty, on 24 June 2010, and receiving NCIMB Deposit Accession Nos. 41731. *Pseudomonas* strain NH4 refers to a stable mucoid Cystic Fibrosis sputum isolate NH57388A isolated from a chronically colonized CF patient attending the Danish CF Center, Rigshospitalet, Copenhagen, Denmark (N. Hoffmann, B. Lee, M. Hentzer, T. B. Rasmussen, Z. Song, H. K. Johansen, M. Givskov, and N. Høiby "Azithromycin Blocks Quorum Sensing and Alginate Polymer Formation and Increases the Sensitivity to Serum and Stationary-Growth-Phase Killing of P. aeruginosa and Attenuates Chronic P. aeruginosa Lung Infection in Cftr-/- Mice" Antimicrob Agents Chemother (2007) 51(10): 3677-3687).

In this specification, the term "sequence homology" should be considered to include both sequence identity and similarity, *i.e.* a phage sequence that shares at least 96% sequence homology with a reference phage is one in which any 96% of aligned nucleotides at least are either identical to, or conservative substitutions of, the corresponding residues in the reference phage.

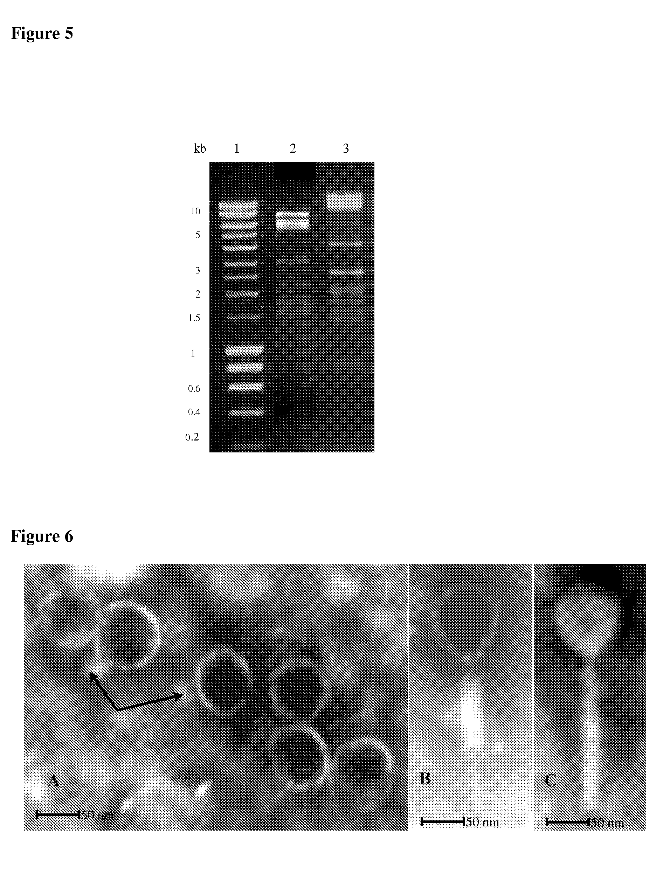

In the specification, the term "Isolated" should be considered to mean material removed from its original environment in which it naturally occurs, for example, in this instance bacteriophage specific for a particular bacterium. The removed material is cultivated, purified and cultured separately from the environment in which it was located. Thus, the purified isolated phage in this instance does not contain any significant amounts of other phages. For the term "isolated progeny", the term should be considered to mean replicates of the original phage, including descendents of the phage created by serial passage of the iophage or by other means known in the art, and isolated in the same manner as described above, and phages having a substantially equivalent RFLP profile to the deposited phage. In the specification, the term "Restriction Fragment Length Polymorphism profile" or "RFLP profile" should be considered to mean the *Eco*R1 restriction digestion profile of Figure 5. The term "RFLP profile that is substantially equivalent" should be considered to mean a RFLP profile describing acceptable variability between genomes of identical propagated organisms of isolated progeny as described by Tenover *et al.*

In the specification, the term "phage cocktail" should be considered to mean a combination comprising the two phages of the invention, or variants or progeny thereof, each of which have been isolated from the environment from which they were originally found or have been produced by means of a technical process such as genetic engineering or serial passage techniques.

"Treating" (or "treat") as used herein includes its generally accepted meaning which encompasses prohibiting, preventing, restraining, and slowing, stopping or reversing progression, severity, of a cause or resultant symptom of a *P. aeruginosa* infection. The term includes causal or symptomatic treatment. As such, the methods of this invention encompass both therapeutic and prophylactic administration.

In this specification, the term "prevention" should be taken to mean inhibition or prevention of the growth of *P. aeruginosa* bacteria, typically *P. aeruginosa* biofilms, ideally in the lungs of an individual suffering from CF.

In the specification, the term "mammal" or "individual" as employed herein should be taken to mean a human; however it should also include higher mammals for which the prophylaxis, therapy or use of the invention is practicable.

In this specification, the term "administering" should be taken to include any form of delivery that is capable of delivering the phage to a site of infection, including local delivery, intravenous delivery, oral delivery, intranasal delivery, intramuscular delivery, intrathecal delivery, transdermal delivery, inhaled delivery and topical delivery. Methods for achieving these means of delivery will be well known to those skilled in the art of drug delivery. For treatment or prophylaxis of lung infections, especially chronic *P. aeruginosa* infections in patients with compromised lung function, such as CF patients, pulmonary delivery is ideal as it delivers the phages of the invention directly to the small airways of the lung where the target bacteria mucoid infections exist.

In this specification, the term "pharmaceutical composition" should be taken to mean compositions comprising a therapeutically effective amount of a phage, and a pharmaceutically acceptable carrier or diluent. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the phage is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, *etc.* Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

"Effective amount" refers to the amount or dose of the phage, upon single or multiple dose administration to the patient, which provides the desired effect in the patient under treatment. An effective amount can be readily determined by the attending diagnostician, as one skilled in the art, by the use of known techniques and by observing results obtained under analogous circumstances. In determining the effective amount or dose of phage administered, a number of factors are considered by the attending diagnostician, including, but not limited to: the species of mammal; its size, age, and general health; the specific disease involved; the degree of or involvement or the severity of the disease; the response of the individual patient; the particular bacteriophage or combination of bacteriophages administered; the mode of administration; the bioavailabilty characteristics of the preparation administered; the dose regimen selected; the use of concomitant medication; and other relevant circumstances. For example, the phages of the invention may be administered at a concentration of about 10⁵ - 10¹¹ PFU/ml.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

### Brief Description of the Drawings

The invention will be more clearly understood from the following description of an embodiment thereof, given by way of example only, with reference to the accompanying drawings, in which:-
**Figure 1** Confluent CFBE41o- cell mono-layer (8-10 days old), A and 24 hr old *Pseudomonas* micro-colonies (biofilms) scattered on surface of CFBE41o- cell monolayer, B.
**Figure 2** Growth of *lux*-tagged *Pseudomonas* biofilms on surface of CFBE410- cell monolayer. Light measured over the 24 hr incubation period.
**Figure 3** Light (photon unit) measurements of biofilm growth on CFBE41o- monolayer (data from Fig. 2). Light reading was taken at times 1, 5 and 24 hr. A and B show respectively light readings before and after washing biofilm culture with MEM medium. values are mean ± S.D readings from 6 wells.
**Figure 4** Fluorescent image of 24 hr old CF *P. aeruginosa* biofilms formed on CFBE41o- cell monolayer following calcofluor (fluorescent enhancer) staining. A and B showing matured biofilms of strains NH57388A and MR299 respectively. C and D showing weakened and opened biofilms of strains NH57388A and MR299 respectively after exposed to phage mix for 22-24 hr.
**Figure 5** *Eco*RI restriction digestion profile of phage DNA. Lane 1, Hyperladder I marker, Lanes 2 and 3 phages φMR299 and φNH-4 respectively
**Figure 6** Electron Microscopy Images of the podophage φMR229-2, (A) and myophage φNH-4, (B & C), stained with phosphotungstic acid 0.2%. Arrow in A, showing a short tail of 10-20 nm long of the podophage, and B and C showing contracted, B and relaxed tail sheath, C of the myophage.
**Figure 7** *Pseudomonas* biofilm clearing by phage mix. A, Biofilms of non-mucoid MR299 strain and B, mucoid NH57388A strain grown on CFBE410- cell monolayer for 24 hr. Cultures washed with MEM medium to remove planktonic cells and undefined phage mix applied and incubated for 22-24 hr. Light was monitored and reading taken at times indicated. Wells in the top row (a) control biofilm cultures without phage added and bottom row (b), with phage added.
**Figure 8** Clearing of *Pseudomonas* biofilms on CFBE41o- monolayer by crude phage mix (data from Figure 7). Phages were was applied to a 24 hr biofilm of NH57388A and MR299 cells and light monitored over 22-24 hr period. Values are mean ± S.D readings from 3 wells.
**Figure 9** *Pseudomonas* biofilm clearing by phage mix. *Pseudomonas* non-mucoid MR299 strain biofilm culture, A and mucoid NH57388A strain culture, B. Undefined phage mix was added to the top row wells, a, and defined mix of phage φNH-4 and φMR299-2 added to bottom wells, b. Light was monitored at time 0 and 24 hr.
**Figure 10** Phage titer estimated at time 0 and 24 hr after addition of phage into *Pseudomonas* biofilm of MR299, A and NH57388A strains, B. values are mean ± S.D readings from 3 wells.
**Figure 11** Clearing of *Pseudomonas* MR299 strain infection from lungs of infected mice by a cocktail of φNH-4 and φMR299-2 phages. Top row, control mice and bottom row, phage treated mice.
**Figure 12** Clearing of *Pseudomonas* NH57388A strain infection from lungs of infected mice by a cocktail of φNH-4 and φMR299-2 phages. Top row, control mice and bottom row, phage treated mice.
**Figure 13** PCR reactions (16S rDNA) of CF *Pseudomonas* isolates. A, genera specific primers product (618 bp) and B, species specific primer product (956 bp). Lanes 1-9, CF *Pseudomonas* isolates, Lane 10, *P. aeruginosa* POA1 (control). M, hyperLadder IV DNA marker.
**Figure 14** Colony morphology of *P. aeruginosa* overnight cultures on Luria Broth (LB) agar plate of the mucoid strain NH57388A, A and a non-mucoid strain MR299, B.
**Figure 15** Plaques formed by phage MR 299-2 on *P. aeruginosa* lawn growing on Luria Broth (LB) Double layer agar plate (lytic activity test).

### Detailed Description of the Drawings

### Bacterial strains and culture conditions

CF *Pseudomonas* strains used in this study are shown (Table 1). LB medium was used throughout this study for the culturing of *Pseudomonas* strains. A standard system for identification was performed to determine metabolism and assimilation profile of *Pseudomonas* isolates using API kit (API 20 NE, Bio-Mereux, Etoile, France). API reactions were read according to the reading table and identification was obtained by referring to the analytical profile index provided. For phage isolation, a double-strength LB medium was made by doubling the weight of dry ingredients required to prepare single-strength LB broth. Cultures were grown at 37 °C under aerobic conditions and shaking at 180 rpm. Solid media and soft agar overlays contained 1.5% and 0.7 % agar (BD Difco, Oxford, UK), respectively.

### Transformation of Pseudomonas with p16lux plasmid

*P. aeruginosa* strains NH57388A (mucoid) and MR299 (non-mucoid) were transformed with p16S*lux* plasmid (Reidel *et al.* 2007) by the method of Shen *et al.,* (2006). In brief, *Pseudomonas* strains were grown in LB medium at 25 °C until O.D 0.8 (600nm) is reached. To facilitate electroporation, *Pseudomonas* exopolysaccharide was digested by adding Aliginate-lyase (Sigma Cat. No. A1603, Sigma, Japan) to a final concentration of 2 U ml⁻¹. The cell enzyme mixture was incubated at 37 °C for 30 min. Cells were centrifuged at 10,000 x g (4 °C) for 10 min. The resultant pellet was washed twice with chilled electroporation buffer (containing 300mM glucose, 5 mm CaCl₂ and 25mM HEPES in distilled water, pH 7.0) and resuspended in 0.1 ml of buffer (1 x 10⁹⁻¹⁰ cfu/ml). These electro-competent cells were mixed with 10 µl of p16S*lux* tagged plasmid DNA (10ug) and incubated on ice for 10-15 min. The mixture was immediately transferred to a chilled electroporation cuvette (0.2 cm electrode gap Gene-pulser cuvette, BioRad, Hercules, CA, USA) and subjected to a single Voltage shock by applying a pulse (settings: capacitor, 25µF; resistor, 200 Ω and voltage, 2.5 kV on ECM 630, BTX, Harvard precision pulse apparatus, Holliston, MA, USA). Immediately after electric shock, 900 µl chilled SOC medium was added to the mixture and incubated on ice for 10 min, followed by incubation at 30 °C for 2-3 hr. Cells were concentrated by centrifugation at 10,000 x g (room temperature) and transformants were obtained by plating cells on LB agar containing erythromycin (800 µg) and incubating at permissive temperature (30°C) for 24 - 48 hr. Ery^{r} colonies were checked for light emission using a VivoVision IVIS100 imaging system (Xenogen, Alameda, CA), luminescence was measured in relative light units (RLU, in photons s⁻¹) and the presence of p16S*lux* was confirmed by mini-prep and restriction analyses.

### Identification of Pseudomonas using PCR reactions

Colony PCR was performed using genus-specific primers PA-GS-F (5'-GACGGGTGAGTAATGCCTA-3'(SEQ ID No. 1)) and PA-GS-R (5'-CACTGGTGTTCCTTCCTATA-3'(SEQ ID No. 2)), designed to the 16S rDNA sequences of *Pseudomonas.* Positive isolates were further analyzed and verified by *P. aeruginosa* species-specific primers PA-SS-F (5'-GGGGGATCTTCGGACCTCA-3'(SEQ ID No. 3)) and PA-SS-R (5'-TCCTTAGAGTGCCCACCCG-3'(SEQ ID No. 4)). All primers and PCR conditions were according to Spilker *et al.,* (2004).

### Isolation of phages from sewage

*Pseudomonas* phages were isolated from fresh sewage obtained from a local treatment plant as described previously (Alemayehu *et al.,* 2009) with some modifications. Sewage samples were centrifuged at 3,200 x g value (Heraeus Labofuge 400 Centrifuge, Thermo Fisher Scientific, Inc. MA, USA) for 12 min and the supernatant filtered using a 0.45 µm pore size filter (Sarstedt, Actiengeselischaft & Co., Germany). The sewage filtrate was mixed with an equal volume of double strength LB broth, supplemented with 10 mM CaCl₂ and inoculated with a mixture of CF *P. aeruginosa* cultures. Samples were incubated overnight aerobically (37 °C) with slow shaking (20-30 rpm). Following overnight incubation, cultures were centrifuged at 3,200 x g value for 12 minutes to remove bacterial cells and debris and the supernatant was filtered through sterile 0.45-µm pore size filter. A double layer LB agar plate containing a lawn of host cultures and 10 mM CaCl₂ was prepared and 10 µl of cell free filtrate containing phage applied. Plates were examined for presence of plaques after incubating aerobically for 18-24 hr at 37 °C.

### Plaque purification and phage titering

Phages were purified by successive single plaque isolation and propagation. In general, a single plaque was picked from a plate using a sterile capillary tube and added into mid log-phase *Pseudomonas* culture (10⁸ cfu/ml) supplemented with 10 mM CaCl₂. The culture phage mixture was incubated at 37 °C overnight. The lysate was filter sterilised through a 0.45-µm pore size sterile filter, serial dilutions were made and plaqued on a lawn of host culture. Single plaque isolation and plaquing process was repeated three additional times after which purified phages were obtained. Phage titer was determined as plaque forming units (pfu/ml) by plaque assay as previously described (O'Sullivan *et al.* 2001).

### DNA extraction and restriction digestion analysis

High titer purified phage suspension was prepared by concentration of phage particles from 400 ml cell lysate in LB medium to a final volume of 1 ml in sterile ice-cold ammonium acetate (0.1 M, pH 7.2) according to Capra *et al.*, (2006) and Alemayehu *et al.* (2009). DNA was extracted from high titer purified phage according to Moineau *et al.*, (1994) and Alemayehu *et al.,* (2009). Phage DNA was digested with restriction endonuclease EcoRI (New England Biolabs, MA, USA) according to the supplier's recommendation and digested samples were analysed by gel electrophoresis using agarose gel (0.7%) containing ethidium bromide.

### Electron microscopy and phage characterization

For electron microscopy, a drop of high titer phage suspension (1-2×10⁹ pfu/ml) was deposited on carbon-coated copper grids, negatively stained with 2% (wt/vol) potassium phosphotungstate (pH 7.2) and examined with Zeiss Supra 40VP scanning electron microscope (Carl Zeiss SMT LTD Cambridge, UK) fitted with Scanning Transmission Electron Microscope Detector (STEM) operating in bright field mode at 25 kV accelerating voltage (National Food Imaging Centre, MFRC).

### Cell Culture and Pseudomonas biofilm formation on surface of human bronchial epithelial cells (CFBE41o-)

Cystic Fibrosis Bronchial Epithelial (CFBE41o-) cell cultures (Bruscia *et al.*, 2002, Cozens *et al.*, 1994) were grown according to Anderson *et al.* (2008). In general, CFBE410- cells were seeded in sterile 6-well, flat bottom tissue culture plates (Sarstedt, Newton, NC, USA) at a concentration of 10⁶ cells/well and maintained in minimal essential medium (MEM) containing 10% fetal bovine serum, 2mM L-glutamate, 100ug/ml penicillin and 100ug/ml streptomycin (all from Invitrogen GIBCO, Invitrgen, UK). The cells were grown at 37 °C and 5 % CO₂ using Jouan cell life, Incubator (Jouan, IGO150, St. Herblain, France) for 8-10 days until cells formed a confluent monolayer and tight junctions. MEM medium was changed every 2-3 days until confluent growth was achieved.

For biofilm formation, lux-tagged *P. aeruginosa* cells were grown on the confluent CFBE41o- monolayer using a co-culture model system (Anderson et al., 2008). Once the monolayer growth was achieved (between 8-10 days), the medium was replaced with 1.5 ml fresh MEM (without fetal bovine serum, penicillin and streptomycin) and *lux* tagged *Pseudomonas* cells inoculated (1-2 x 10⁷ cfu/well). Plates were incubated at 37 °C and 5% CO₂ for 1 hr. Then, the medium containing the unattached (planktonic) *Pseudomonas* cells was removed using sterile serological pipette and replaced with fresh MEM supplemented with 0.4% arginine and incubated further for 24 hr. Then, planktonic *Pseudomonas* cells were removed and the biofilm culture washed twice using MEM supplemented with 0.4% arginine. Epithelial-monolayer integrity and growing *Pseudomonas* microcolonies were assessed by phase-contrast microscopy (Olympus IX50, inverted system microscope, Olympus Co. Tokyo, Japan). Luminescence from biofilms was monitored by VivoVision IVIS100 imaging system (Xenogen, Alameda, CA, USA). Biofilm cfu estimation was according to Wirtanen *et al.*, (2001) with some modifications. In general, washed epithelial monolayer with 24 hr biofilms was scraped using tissue culture scraper and transferred to 2 ml eppendorf tube containing 1ml phosphate buffer. The tube was vortexed thoroughly for 1-2 min to release the cells. Samples were serially diluted and plate count was made on LB plates incubated at 37 °C overnight.

### Applying phages to biofilms and mice lung infections

Fifty µl (1-2 x 10⁹ pfu/ml) of a phage cocktail containing phages φNH-4 and φMR299-2 were applied to wells containing 24 hr old biofilms on CFBE41o- cell monolayer and plates were incubated at 37°C and 5 % CO₂ for 24 h. Biofilm clearing was monitored by measuring light and images taken for times indicated, using the IVIS 100 imaging system. *Pseudomonas* lung infection in 6 - 8 week-old conventional female BALB/c mice (N=8) was performed according to Riedel *et al.* (2007). Animals were infected intranasally with 50 µl *lux* tagged *Pseudomonas* NH57388A or MR299 in phosphate buffer (2-5 x 10⁸ cfu/ml). Two hours after infection, a 50 µl phage cocktail suspension (1-2 x 10⁹ pfu/ml = multiplicity of infection (moi) of 2-5x) was given intranasally. Fifty µl phosphate buffer was given to the control groups. Animals were anesthetized with isoflurane, light was monitored and images were taken using the IVIS 100 system at time points indicated. Animals were kept in an animal colony, and all experiments were approved by the animal ethics committee of University College Cork.

### Biofilm staining

*Pseudomonas* biofilms were stained with the fluorescent enhancer calcofluor, (Fluorescent Brightener 28, Cat. No: F3543, Sigma Aldrich, China). In brief, CFBE41o- monolayer containing biofilms were removed from wells using sterile cell-scraper (Sarstedt, Actiengeselischaft & Co., Germany) and mixed with a drop of 0.1 % calcocfluor on a microscope slide. Cover-slip was applied; edges sealed with paraffin oil and incubated for 1 hr (37 °C). Stained biofilm preparations were assessed by Olympus BX51 fluorescent-microscope fitted with U-RFL-T fluorescent power supply unit and images taken by DP50 integrated Camera (all from Olympus Optical Co., Japan).

### RESULTS

### Identification of Pseudomonas by PCR and API test

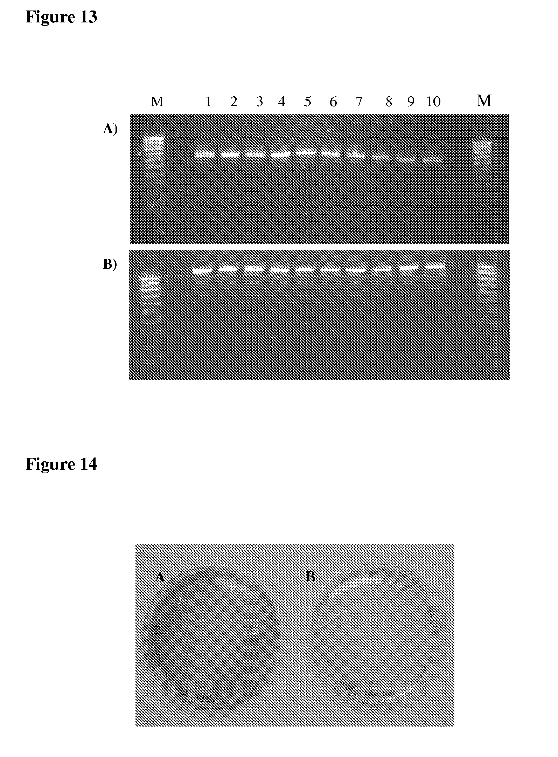

Plasmids and strains used in this study are shown (Table 1 and Table 2). The PCR assay produced DNA products of the predicted sizes. 16S rDNA PCR products of 618 and 956 bp were obtained using *P. aeroginosa* genera and species specific primer pairs respectively (**Figure 13****.**). PCR results confirm that all isolates belong to *P. aeruginosa* species. Substrate metabolism and assimilation ability of isolates was examined by API 20 NE kit (Table 2). The API test profile obtained for CF isolates was consistent with the API20 NE identification criteria and confirmed that isolates belong to *P. aeruginosa* species.

### Isolation of phage from sewage

Myophage φNH-4 and podophage φMR299-2, both virulent for *Pseudomonas* NH57388A and MR299 strains, were isolated from fresh sewage. A plaque assay performed on an LB Double Agar plate for podophage φMR299-2 demonstrated the lytic activity of the M29902 phage against *Pseudomonas* strain MR299-2 which is illustrated in **Figure 15**. The electron micrographs shown, obtained by scanning electron microscopy (**Figure 6**), revealed that phage φMR299-2 virions have isometric heads of 40 - 60 nm in diameter and very short tails measuring 10-20 nm. Morphologically, phage φMR299-2 is very similar to *Pseudomonas* phage PaP3, a podovirus which has an isometric head and short tail. Therefore, like φPap3, the presently isolated φMR299-2 should be attributed to type species coliphage T7 and in the family Podoviridae, as referenced in the Report of the International Committee on Taxonomy of Viruses (Van Regenmortel *et al.*, 2000, Murphy *et al.*, 1995). Phage φNH-4 is different in morphology from that of φMR299-2 and displayed an isometric head of 50-60 nm in diameter and a contractile nonflexible tail (non-contracted tail length =150 nm and contracted tail length =85 nm; tail diameter 20 nm when contracted). The tail sheath shows cross striations and diameter increase 1.5 times when contracted. In addition, a putative DNA injecting structure of 70 nm in length (narrower than the contracted tail sheath) and tail hair fibres are observed (<). Morphologically, φNH-4 shows similarity to phages PB1, LBL3 and SN, which are classified in the A1 morphological group of the Myoviridae. Based on structural characteristics obtained from microscopic analysis, phage φNH-4 can be classified as a member of the Myoviridae family according to the International Committee on Taxonomy of Viruses (Van Regenmortel *et al.*, 2000, Murphy *et al.*, 1995).

The percent (%) identity of the φNH-4 phage and φMR299-2 phage with known *Pseudomonas* phages are summarised in Tables 3 and 4. The number of open reading frames (ORF's) for the φNH-4 phage and φMR299-2 phage are 114 and 73, respectively. There are 8 and 3 unique ORF's for the φNH-4 phage and φMR299-2 phage, respectively (Tables 3 and 4).

The infectivity of phages φNH-4 and φMR299-2 was tested against 8 other CF *Pseudomonas* isolates (Table 6) using the plaque assay described above. Six (75%) were sensitive to both phages. However, there was significant variation in the level of sensitivity of the isolates to the two phages. Two (25%) of the CF isolates were sensitive to only one or the other phage (Table 6). This data shows the cross infection capabilities of phages φNH-4, and φMR299-2. It was observed that *Pseudomonas* cells exposed to a phage mix of φNH-4 and φMR299-2 produced very strong lytic activity compared to that exposed to individual phages. Based on this observation, a cocktail phage mix was used in all experiments to assess efficacy of phages on *Pseudomonas* cell lysis. DNA restriction pattern of phages φNH-4 and φMR299-2 are shown (**Figure 5**).

### Biofilm growth by lux tagged Pseudomonas on CFBE41o- cell monolayer

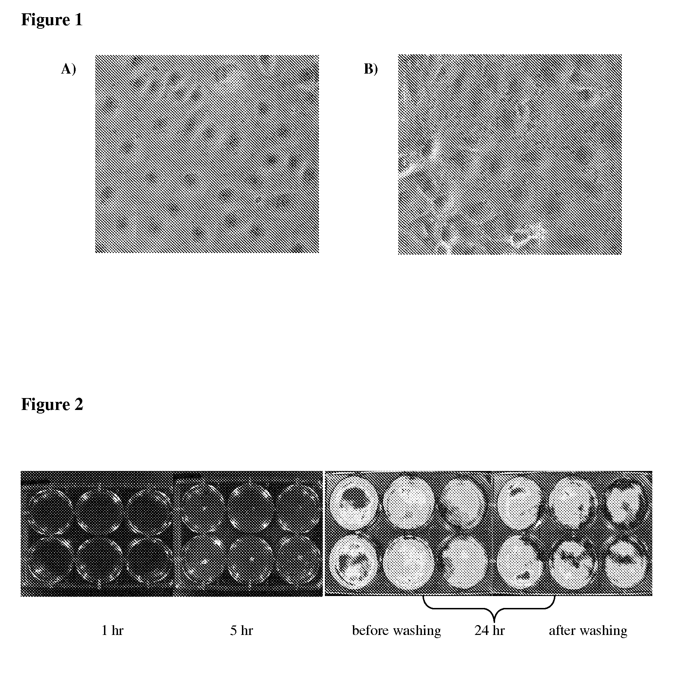

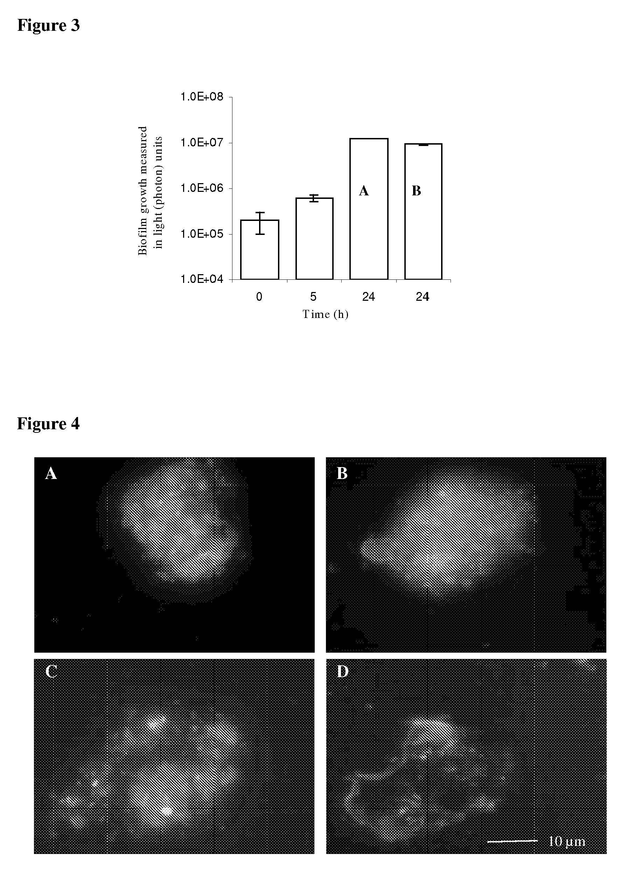

Cystic Fibrosis Bronchial Epithelial (CFBE41o-) cells were grown in standard 6-well tissue culture plates. A monolayer culture with a tight junction between cells was achieved in 8 - 10 days of incubation (**Figure 1A**). The monolayer culture was inoculated with *lux* tagged *Pseudomonas* strains NH57388A::p16S*lux* or MR299::p16S*lux* and biofilm growth was monitored in this static system for 24 hr. The addition of arginine in the minimal growth medium enhanced the formation of biofilms and helped preserve the monolayer integrity of CFBE41o- cells as suggested by Anderson et al., (2008). The amount of light recorded for the growing biofilms increased by 1.5-2.0 log units during the 24 hr incubation period (**Figures 2** and **3**). Washing of the biofilm-monolayer culture twice with MEM medium removed all planktonic *Pseudomonas* cells. The absence of motile cells and the presence of only adhered clusters of micro-colonies of various sizes scattered across the epithelial cell monolayer was confirmed by phase contrast microscopy (**Figure 1B**). The amount of bioluminescence light recorded after washing the 24 hr-old biofilm culture with MEM and removing planktonic cells was only 70-80% of the light recorded before washing (**Figure 3**). This confirmed that light was indeed emitted from *Pseudomonas* biofilm clusters adhered to the epithelial monolayer (**Figure 2**). In order to be able to know the cell density in each well, the biofilm cfu count was estimated after washing the biofilm. The cfu estimated for cells attached to the CF epithelial monolayer as biofilms was 2.6-3.8 x 10⁷ cfu/well and 4.2-5.4 x10⁷cfu/well for NH57388A and MR299 strains respectively. The cfu estimated for biofilms of the mucoid NH57388A strain was less than that estimated for the non-mucoid strain MR299. This might indicate that complete release of cells from biofilms was not achieved for mucoid NH57388A strain during vortexing.

Calcofluor binds to β(1-3) and β(1-4) polysaccharide linkage found in the biofilm matrices produced by exopolysacchride producing organisms. Staining of the biofilm structures with calcofluor (fluorescent dye) revealed the presence of a pack of *Pseudomonas* cells contained within a polysaccharide matrix (**Figure 4A** and *4B*). The biofilm formed by the two *Pseudomonas* strains NH57388A and MR299 measured on average 20-30 x 30-40 µm in diameter, and were not different from each other (**Figure 4A** and **4B**). The presence of abundant numbers of intact and healthy looking *Pseudomonas* cells packed in the biofilm matrices confirmed that indeed the microcolony structures formed on the monolayer were *Pseudomonas* biofilms.

### Clearing of biofilms by phage

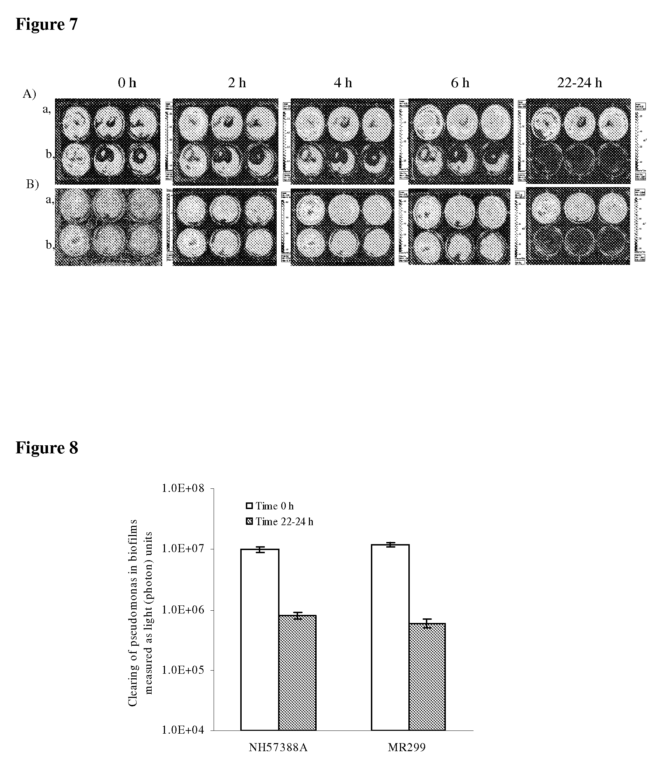

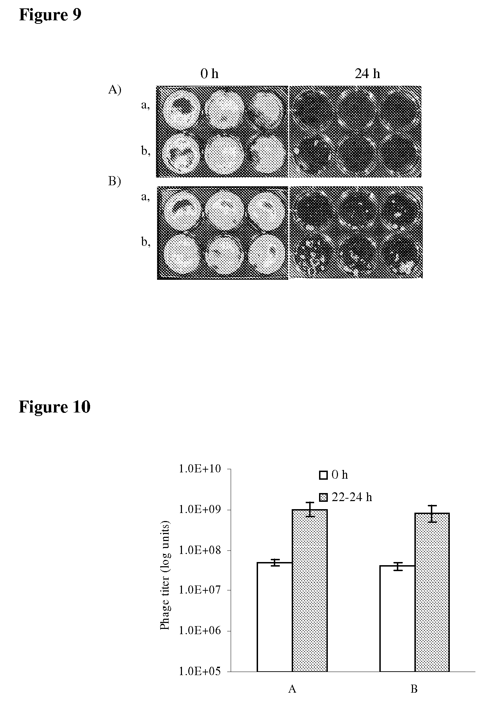

The ability of undefined cocktails of *Pseudomonas* phages in clearing biofilms of *Pseudomonas* NH57388A or MR299 was examined. In the presence of phage, the amount of luminescence light recorded for both *Pseudomonas* biofilms decreased dramatically by 10 to 20 fold over the 24 hr period. Whereas, in the control biofilms (with no phage added), the light level remained high and unchanged over the same period (**Figures 7A** & **7B**; 8A & 8B). A similar result was obtained when a defined phage mix (purified from the undefined phage mix) containing φNH-4 and φMR299-2 was applied to biofilms of NH57388A or MR299 strains (**Figure 9**). This shows that phages φNH-4 and φMR299-2 were the main virulent phages present in the undefined cocktail. The results confirm that the significant reduction in the amount of light was a direct result of lysis of the *Pseudomonas* cells by phages. Since the cfu estimated for biofilms was 2.6-3.8 x10⁷cfu/well and 4.2-5.4 x10⁷cfu/ well for NH57388A and MR299 strains respectively, and the amount of phage added was 0.5-1.0 x10⁸/well (MOI = 2-5) suggests that the extended time period required for clearing of the biofilms by the phage cocktail was directly related to the biofilm structure and not to the phage: cell ratio. Furthermore, the increase in phage titer by 10- to 20-fold during the 24 hr incubation period confirmed that phage multiplication took place in *Pseudomonas* biofilms (**Figure 10**).

### Clearing of Pseudomonas from murine lungs

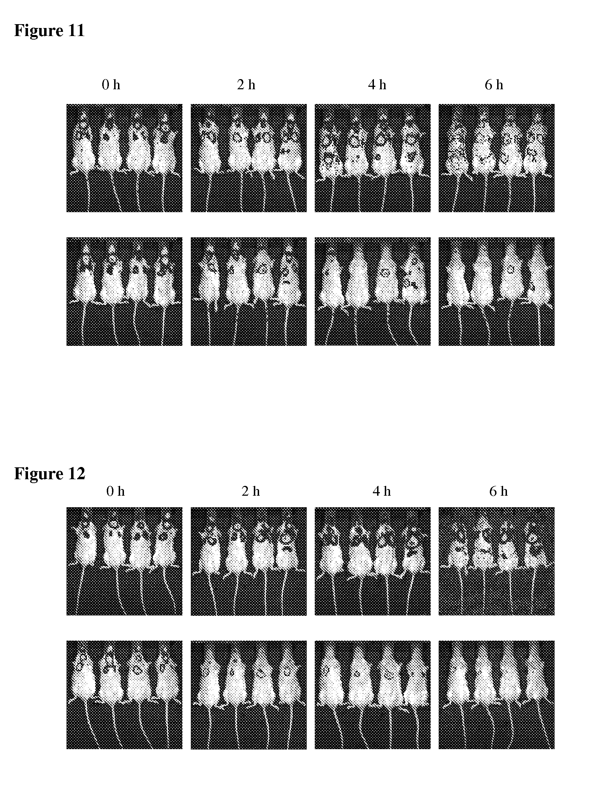

Clearing of *Pseudomonas* from lungs of infected 8-week-old female BALB/c mice with phage cocktail containing φNH-4 and φMR299-2 phages was examined and results are shown in (**Figures 11** & **12**). Presence of *Pseudomonas* was evident in lungs of both test and control mice 2hr after infection. The amount of light recorded in the control mice (without phages) increased 2- to 3-fold and reached maximum level at time 6 hr. Whereas, the amount of light recorded in mice treated with the phage cocktail decreased and reached lowest, and undetectable levels during the same period (**Figures 11** & **12**). The data clearly shows that the reduction in the amount of light over the 6 hr period after administration of phages was a direct result of the disintegration of the *Pseudomonas* cells by phages. The results confirmed the effectiveness of phages in clearing *Pseudomonas* from lungs of experimentally infected mice.

The invention is not limited to the embodiments herein before described but may be varied in both construction and detail.

**Table1. Strains and plasmids used**

| Plasmid/Strain | Description | Source |
|---|---|---|
| Plasmid p16S*lux* | *lux*-tagged plasmid vector | 20 |
| *P. aeraginosa* NH57388A | Stable mucoid CF mouse sputum isolate | 19 |
| *P. aerugiuosa* MR299 | Human CF sputum isolate | UCC hospital, Cork |
| *P. aerugiuosa* NH57388A::p16S*lux* | *lux*-tagged Stable mucoid CF mouse sputum isolate | This study |
| *P. aeruginosa* MR299::p16S*lux* | *lux*-tagged human CF sputum isolate | This study |

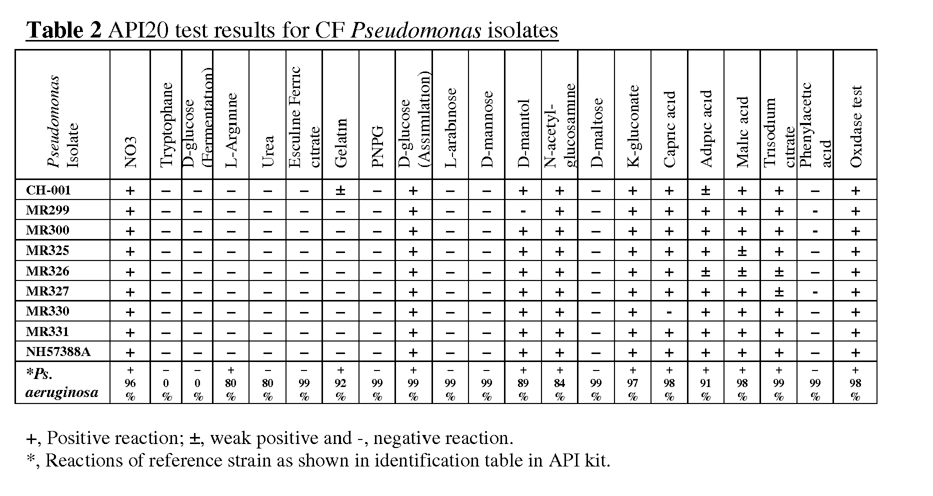

**Table 3. PB1-like Pseudomonas phage genome sequences producing significant alignments against phage NH-4 complete genome**

| Accession | Description | Source | Location | Yr | Genome size, bp (%GC) | ORF's | Unique ORF 's | DNA identity to NH-4 |
|---|---|---|---|---|---|---|---|---|
| FM897211.1 | *Pseudomonas* phage 14-1, complete genome | Sewage | Regensburg, Germany | 2004 | 66238 (55.6) | 90 | - | 95% |
| FMRR7021.1 | *Pseudomonas* phage SN, complete genome | SN Lake | Russia | 2004 | 66,390 (55.6) | 92 | 2 | 89% |
| FM201282.1 | Pseudomonas phage LMA2 complete genome | River | Maastricht, Holland | 2007 | 66 530 (55.6) | 95 | 2 | 97% |
| FM201281.1 | *Pseudomonas* phage LBL3 complete genome | Pond | Blanes, Spain | 2006 | 64427 (55.5) | 88 | 2 | 94% |
| EU716414.1 | *Pseudomonas* phage PB1, complete sequence | Sewage | Edinburgh, Scotland | 1960 | 65,764 (55.5) | 93 | - | 92% |
| DQ163917.1 | Bacteriophage F8, complete genome | unknown | Unknown | 1972 | 66 015 (55.6) | 93 | 1 | 93% |
| N/A | Bacteriophage NH-4, complete genome | Sewage | Cork, Ireland | 2008 | 66 049 (55.5) | 114 | 8* | 100% |

| | | | | | | | | |
|---|---|---|---|---|---|---|---|---|
| ***Unique ORF's of phage NH-4 (ORF; 2, 8, 13, 17, 49, 84, 100, and 107).** | | | | | | | | |

**Table 4. PaP3-like Pseudomonas phage genome sequences producing significant alignments against phage MR299-2 complete genome**

| Accession | Description | Source | Location | Yr | Genome size, bp (%GC) | ORF 's | Unique ORF's | DNA identity to MR299-2 |
|---|---|---|---|---|---|---|---|---|
| AY078382.2 | *P. aeruginosa* phage PaP3, complete genome | Sewage | Chongqing, China | 2003 | 45,503(52.2) | 71 | - | 97% |
| AM910650.1 | *Pseudomonas* phage LUZ24, complete genome | Sewage | Leuven, Belgium | 2007 | 45,625(52.3) | 69 | - | 70% |
| N/A | *P. aeruginosa phage* MR299-2 complete genome | Sewage | Cork, Ireland | 2008 | 45,441(51.9) | 73 | 3* | 100% |

| | | | | | | | | |
|---|---|---|---|---|---|---|---|---|
| **Unique ORF's of phage MR299-2 (ORF; 43, 50 and 61).** | | | | | | | | |

**Table 5. Comparison of Pseudomonas phage PAK-P1 against newly isolated phages NH-4 and MR299-2**

| Phage | PAK-P1 | NH-4 | MR299-2 |
|---|---|---|---|
| Family | Myoviridae | Myoviridae | Podoviridae |
| Genome size (kb) | 93,398 | 66,049 | 45,441 |
| GC content | 49.5% | 55.5% | 51.9% |

**Table 6. Sensitivity of CF isolate Pseudomonas strains to phages NH-4 and MR299-2. Sensitivity to phage was determined using spot plaque assay by applying 10u1 phage suspension to a lawn of test organism.**

| | *Pseudomonas* strain | | | | | | | | | |
|---|---|---|---|---|---|---|---|---|---|---|
| Phage | CH001 | MR299 | MR300 | MR325 | MR326 | MR327 | MR330 | MR331 | *NH | POA1 |
| φNH-4 | + | + | + | - | + | + | + | + | + | + |
| φMR299-2 | + | + | - | + | + | + | + | + | + | + |

| | | | | | | | | | | |
|---|---|---|---|---|---|---|---|---|---|---|
| +, lyses and -, no lyses. * NH57388A | | | | | | | | | | |

### References

Alemayehu, D., Ross, R.P., O'Sullivan, O., Coffey, A., Stanton, C., Fitzgerald, G.F., McAuliffe, O. (2009). Genome of a virulent bacteriophage Lb338-1 that lyses the probiotic Lactobacillus paracasei cheese strain. Gene 448(1):29-39.
Anderson, G.G., Moreau-Marquis, S., Stanton B.A., and O'Toole, G.A. (2008). In vitro analysis of tobramycin-treated P. aeruginosa biofilms on cystic fibrosis-derived airway epithelial cells. Infec. Immun. 76(4): 1423-33.
Bruscia E, Sangiuolo F, Sinibaldi P, Goncz KK, Novelli G, Gruenert DC. (2002). Isolation of CF cell lines corrected at DeltaF508-CFTR locus by SFHR-mediated targeting. Gene Ther. 9(11):683-5.
Capra, M. L., Del, L. Quiberoni A., Ackermann, H. W., Moineau, S. and Reinheimer, J. A. 2006. Characterization of a new virulent phage (MLC-A) of Lactobacillus paracasei. J Dairy Sci. 89(7): 2414-23.
Cozens, A.L., Yezzi, M.J., Kunzelmann, K., Ohrui, T, Chin, L., Eng, K., Finkbeiner, W.E., Widdicombe, J.H., Gruenert, D.C. (1994). CFTR expression and chloride secretion in polarized immortal human bronchial epithelial cells. Am J. Respir. Cell Mol Biol. 10(1):38-47.
Moineau S, Pandian S., and Klaenhammer T.R. 1994. Evolution of a Lytic Bacteriophage via DNA Acquisition from the Lactococcus lactis Chromosome. Appl Environ Microbiol. 60(6):1832.
Murphy, F. A., Fauquet, C.M., Bishop, D. H. L., Ghabrial, S.A., Jarvis, A. W., Martelli, G.P., Mayo, M.A. and Summers, M.D. Virus Taxonomy: Sixth Report of The International Committee on Taxonomy of Viruses. 1995. Springer-Verlag ed. Wies, N.Y.
O' Sullivan, D., Ross, R.P., Twomey, D.P., Fitzgerald, G.F., Hill, C., Coffey, A. (2001). Naturally occurring lactococcal plasmid pAH90 links bacteriophage resistance and mobility functions to a food-grade selectable marker. Appl Environ Microbiol. 2001:67(2):929-37.
Riedel, C.U., Casey, P.G., Mulcahy, H., O'Gara, F., Cormac G. M. Gahan, C.G.M and Hill, C. (2007). Construction of p16Slux, a novel vector for improved bioluminescent labeling of Gram-negative bacteria. Appl. Environ. Microbiol. 73(21): 7092-7095.
Shen, H., Han,F., Yuzi Lin, Y. and Yu. W. (2006). A high efficient electroporation of Pseudomonas sp. QDA pretreated with alginate lyase. Enzy. Microbial. Technol. 39(4):677-682.
Spilker, T., Coenye, T., Vandamme, P., LiPuma, J.J. (2004). PCR-based assay for differentiation of P. aeruginosa from other Pseudomonas species recovered from cystic fibrosis patients. J. Clin. Microbiol. 42(5):2074-2079.
Tenover, F.C., Arbeit, R.D., Goering, R.V., Mickelsen, P.A., Murray, B.E., Persing, D.H., Swaminathan, B. (1995) Interpreting Chromosomal DNA Restriction Patterns Produced By Pulsed-Field Gel Electrophoresis: Criteria for Bacterial Strain Typing J. Clin. Microbiol. 33: 2233-2230.
Van Regenmortel, M.H.V., Fuaquet, C.M., Bishop, D.H.L., Carsten, E., Estes, M.K., Lemon, S., et al. (2000). Virus Taxonomy. Classification and Nomenclature of Viruses. Seventh Report of the International Committee on Taxonomy of Viruses. San Diego, CA: Academic press.
Wirtanen, G., Salo, S. Helander, I.M. Mattila-Sandholm T. (2001). Microbiological methods for testing disinfectant efficiency on Pseudomonas biofilm. Coll. Surfaces Biointerfaces 20: 37-50.

## Claims

1. An isolated phage MR299-2 or NH-4 deposited under NCIMB Deposit Accession Nos. 41729 and 41730, respectively, said phage having lytic activity against *P. aeruginosa* strains, and variants thereof, wherein said variants retain the phenotypic characteristics of said phage and wherein said phage and variants thereof have lytic activity against *P. aeruginosa* strains.

2. An isolated phage as claimed in Claim 1 in which the variants have the same lytic activity against *P. aeruginosa* strains as the bacteriophage.

3. An isolated phage MR299-2 variant of Claim 1 having lytic activity against *P. aeruginosa* strains CH001, MR299, MR325, MR326, MR327, MR330, MR331, POA1 MR299 and NH57388A.

4. An isolated phage NH-4 variant of Claim 1 having lytic activity against *P. aeruginosa* strains CH001, MR299, MR300, MR326, MR327, MR330, MR331, POA1 MR299 and NH57388A.

5. An isolated phage MR299-2 variant as claimed in any of Claims 1 to 3 having at least 96% sequence homology with SEQUENCE ID NO: 5.

6. An isolated phage MR299-2 variant as claimed in any of Claim 5 having at least 99% sequence homology with SEQUENCE ID NO: 5.

7. An isolated phage NH-4 variant as claimed in any of Claims 1, 2 or 4 having at least 96% sequence homology with SEQUENCE ID NO: 6.

8. An isolated phage NH-4 variant as claimed in Claim 7 having at least 99% sequence homology with SEQUENCE ID NO: 6.

9. Isolated progeny of the phage of Claim 1, wherein said progeny have the same phenotypic characteristics of said phage and have the same lytic activity against *P. aeruginosa* strains.

10. Isolated progeny as claimed in Claim 9 having a RFLP profile that is substantially equivalent to a RFLP profile of the bacteriophage.

11. A phage cocktail comprising (a) an isolated phage MR299-2 of any of claims 1 to 8, or an isolated variant or progeny thereof, and (b) an isolated phage NH-4 of any of Claims 1 to 8, or an isolated variant or progeny thereof, wherein the phage cocktail has lytic activity against *P. aeruginosa* strains.

12. A pharmaceutical preparation comprising an isolated phage of any of Claims 1 to 8, or an isolated variant or progeny thereof, and a pharmaceutically acceptable carrier.

13. A pharmaceutical preparation comprising a phage cocktail of Claim 11 and a pharmaceutically acceptable carrier.

14. An isolated phage as claimed in any of Claims 1 to 6, or an isolated variant or progeny thereof, or a phage cocktail of Claim 10, for use in the treatment or prevention of *P. aeuriginosa-mediated* infection in a mammal.

15. Use as claimed in Claim 14 for the treatment or prevention of *P. aeuriginosa-*mediated lung infection in a mammal with cystic fibrosis.
